# EUROPEAN PATENT SPECIFICATION

(11) **EP 3 331 984 B1**
(45) Date of publication and mention of the grant of the patent: **22.09.2021**
(21) Application number: 16835767.1
(22) Date of filing: 08.08.2016
(51) Int. Cl.: A61L 27/36, C12M 1/02

(54) **RAPID ALLOGRAFT TREATMENT SYSTEMS AND METHODS**
SYSTEME UND VERFAHREN ZUR SCHNELLEN BEHANDLUNG VON ALLOTRANSPLANTATEN
SYSTÈMES ET PROCÉDÉS DE TRAITEMENT D'ALLOGREFFE RAPIDES

(30) Priority: 07.08.2015 US 201562202661 P; 14.09.2015 US 201562218289 P
(43) Date of publication of application: 13.06.2018
(73) Proprietor: AlloSource, Centennial, CO 80111 (US)
(72) Inventor: SOUTHARD, Matthew James, Denver, Colorado 80247 (US); SAMANIEGO, Adrian C., HIghlands Ranch, Colorado 80129 (US); BLOOD, Kenneth, Littleton, Colorado 80123 (US); BULL, Marina Katelyn, Highlands Ranch, Colorado 80126-7123 (US); STILWELL, Reginald, Parker, Colorado 80134 (US); BARRETT RORICK, Carolyn, Denver, Colorado 80209 (US); PETERSON, Matthew, Thornton, Colorado 80229 (US); ZAJDOWICZ, Jan, Aurora, Colorado 80016 (US); JOSLYN, Arthur, Centennial, Colorado 80015 (US); HANZLICEK, Kathryn, Aurora, Colorado 80015 (US)
(74) Representative: Griffin, Philippa Jane
(86) International application number: PCT/US2016/046070
(87) International publication number: WO 2017/027481

(56) References cited:
- WO-A2-00/15097
- US-A1- 2011 004 091
- US-A1- 2013 316 012
- US-A1- 2014 079 741
- US-A1- 2014 206 077
- US-A1- 2014 302 112
- US-B2- 7 866 878
- ELLIOTT GRUSKIN ET AL: "Demineralized bone matrix in bone repair: History and use", ADVANCED DRUG DELIVERY REVIEWS, vol. 64, no. 12, 1 September 2012 (2012-09-01), pages 1063-1077, XP055559703, AMSTERDAM, NL ISSN: 0169-409X, DOI: 10.1016/j.addr.2012.06.008
- PEGG D E ET AL: "Cryopreservation of articular cartilage. Part 1: Conventional cryopreservation methods", CRYOBIOLOGY, ACADEMIC PRESS INC, US, vol. 52, no. 3, 1 June 2006 (2006-06-01), pages 335-346, XP024943414, ISSN: 0011-2240, DOI: 10.1016/J.CRYOBIOL.2006.01.005 [retrieved on 2006-06-01]

## Description

This application claims benefit of priority of U.S. Provisional Application No. 62/202,661, filed August 7, 2015, and U.S. Provisional Application No. 62/218,289, filed September 14, 2015.

### BACKGROUND

Embodiments of the present disclosure are directed in general to the field of medical grafts, and in particular to methods for processing tissue compositions.

Tissue compositions, such as those derived from bone, cartilage, tendon, and skin, have been used for many years in various surgical procedures, including treatments for certain medical conditions, including tissue defects and wounds and in reconstructive surgical procedures. Medical grafting procedures often involve the implantation of autogenous, allograft, or synthetic grafts into a patient to treat a particular condition or disease. The use of musculoskeletal allograft tissue in reconstructive orthopedic procedures and other medical procedures has markedly increased in recent years, and millions of musculoskeletal allografts have been safely transplanted. Tissue grafts are often implemented in various industries related to orthopedics, reconstructive surgery, podiatry, and cartilage replacement. Musculoskeletal tissue, tendons, cartilage, skin, heart valves and corneas are common types of tissue allografts.

Allograft and autogenous tissue for human transplant are both derived from humans; with autogenous tissue being tissue recovered from a patient for future use for that patient, while allograft tissue is recovered from an individual (donor) other than the patient receiving the graft. Allograft tissue is often taken from deceased donors that have donated their tissue so that it can be used for living people who are in need of it, for example, patients whose bones have degenerated from cancer. Such tissues represent a gift from the donor or the donor family to enhance the quality of life for other people.

In some instances, tissue obtained from a donor must be processed or manipulated in some way to manufacture a useful tissue graft. Although presently used tissue graft compositions and methods of use and manufacture provide real benefits to patients in need thereof, still further improvements are desirable. Embodiments of the present disclosure provide solutions to at least some of these outstanding needs. WO 00/15097 describes methods for using resonant acoustic energy to detect or effect structures. Gruskin et al. Advanced Drug Delivery Reviews (2012) Vol. 64 pp 1063-1077 describes the history and use of demineralized bone matrix in bone repair. US7886878 describes methods for resonant-vibratory mixing. Pegg et al. Cryobiology (2006) Vol 52 pp 335-346 describes conventional cryopreservation methods for cryopreservation of articular cartilage.

### BRIEF SUMMARY

The present invention is directed to methods as defined in the claims. In one apect, provided are methods of processing a tissue, the methods including loading a processing vessel with a tissue and a processing solution, thereby providing a combination comprising the tissue and the processing solution disposed in the processing vessel; and applying resonant acoustic energy having a frequency between 15 Hertz and 60 Hertz to the processing vessel, thereby vibrating the processing vessel and the combination disposed therein to form a processed tissue.

In another aspect, provided are methods of demineralizing a bone tissue, the methods including loading a processing vessel with a bone tissue and an acid processing solution, thereby providing a combination comprising the bone tissue and the acid processing solution disposed in the processing vessel; and applying resonant acoustic energy having a frequency between 15 Hertz and 60 Hertz to the processing vessel, thereby vibrating the processing vessel and the combination disposed therein to form a demineralized bone tissue.

In another aspect, provided are methods of cryopreserving a tissue, the methods including loading a processing vessel with a tissue and a processing solution comprising a cryoprotectant, thereby providing a combination comprising the tissue and the cryopreservation processing solution disposed in the processing vessel; applying resonant acoustic energy having a frequency between 15 Hertz and 60 Hertz to the processing vessel, thereby vibrating the processing vessel and the combination disposed therein to form a processed tissue comprising the tissue mixed with the cryoprotectant; and freezing the processed tissue to form a cryopreserved tissue.

In another aspect, provided are methods of decellularizing a tissue, the methods including loading a processing vessel with a tissue and a decellularization processing solution, thereby providing a combination comprising the tissue and the decellularization processing solution disposed in the processing vessel; and applying resonant acoustic energy having a frequency between 15 Hertz and 60 Hertz to the processing vessel, thereby vibrating the processing vessel and the combination disposed therein to form a decellularized tissue.

In another aspect, provided are methods of processing a tissue to produce stromal vascular fraction, the methods including loading a processing vessel with adipose tissue and a processing solution comprising a cell culture medium, thereby providing a combination comprising the adipose tissue and the processing solution disposed in the processing vessel; and applying resonant acoustic energy having a frequency between 15 Hertz and 60 Hertz to the processing vessel, thereby vibrating the processing vessel and the combination disposed therein to form a processed tissue.

Methods of washing a tissue are described and not claimed, the methods including loading a processing vessel with a tissue and a washing solution, thereby providing a combination comprising the tissue and the washing solution disposed in the processing vessel; and applying resonant acoustic energy to the processing vessel, thereby vibrating the processing vessel and the combination disposed therein to wash the tissue, the washing comprising removing biological fluids, particulates, or both, from the tissue.

In another aspect, provided are methods of reducing microbial contamination of a tissue, the methods including loading a processing vessel with a tissue and a processing solution, thereby providing a combination comprising the tissue and the processing solution disposed in the processing vessel; and applying resonant acoustic energy having a frequency between 15 Hertz and 60 Hertz to the processing vessel, thereby vibrating the processing vessel and the combination disposed therein to remove at least a portion of microbial load from the tissue.

In another aspect, provided are methods of assessing the microbial contamination of a tissue, the methods including loading a processing vessel with a tissue and a processing solution, thereby providing a combination comprising the tissue and the processing solution disposed in the processing vessel; applying resonant acoustic energy having a frequency between 15 Hertz and 60 Hertz to the processing vessel, thereby vibrating the processing vessel and the combination disposed therein to release microbes from the tissue into the processing fluid; and assessing the processing fluid to determine the microbial load of the tissue.

In another aspect, provided are methods of homogenizing a tissue, the methods including loading a processing vessel with a tissue; and applying resonant acoustic energy having a frequency between 15 Hertz and 60 Hertz to the processing vessel, thereby vibrating the processing vessel and the tissue disposed therein to form a homogenized tissue.

In another aspect, provided are methods of producing a homogenized tissue product, the methods including loading a processing vessel with a tissue and at least one of a biological component or a chemical agent thereby providing a combination comprising the tissue and at least one of a biological component or a chemical agent disposed in the processing vessel; and applying resonant acoustic energy having a frequency between 15 Hertz and 60 Hertz to the processing vessel, thereby vibrating the processing vessel and the tissue disposed therein to form a homogenized tissue product.

Methods of improving viability of cells in a tissue are described, the methods including loading a processing vessel with a tissue and a processing solution, thereby providing a combination comprising the tissue and the processing solution disposed in the processing vessel; and applying resonant acoustic energy to the processing vessel, thereby vibrating the processing vessel and the combination disposed therein to form a tissue comprising cells with enhanced viability.

In another aspect, provided are processed tissue and tissue products made according to any of the above methods.

Systems for processing a tissue according to any of the above methods are described, the systems including a processing vessel; and a high intensity mixing device that applies acoustic resonance energy to the processing vessel disposed therein.

The above described and many other features and attendant advantages of embodiments of the present disclosure will become apparent and further understood by reference to the following detailed description when considered in conjunction with the accompanying drawings.

### BRIEF DESCRIPTION OF THE DRAWINGS

These figures are intended to be illustrative, not limiting. Although the aspects of the disclosure are generally described in the context of these figures, it should be understood that it is not intended to limit the scope of the disclosure to these particular aspects.
**FIG. 1** shows steps in a method of processing tissue according to some aspects of the present disclosure.
**FIG. 2** shows steps in a method of demineralizing bone tissue according to some aspects of the present disclosure.
**FIG. 3** shows an exemplary system for processing tissue according to some aspects of the disclosure.
**FIG. 4** shows a schematic of an exemplary system for processing tissue according to some aspects of the disclosure.
**FIG. 5** shows a schematic of an exemplary system for demineralizing bone tissue according to some aspects of the disclosure.
**FIG. 6** shows the results of compressibility and residual calcium content assessment for bone tissue demineralized using an exemplary method according to some aspects of this disclosure.
**FIGS. 7A-7B****,** respectively, show full thickness skin tissue prior to and after decellularization using an exemplary method according to certain aspects of this disclosure.
**FIGS. 8A-8C****,** respectively, show split-thickness skin tissue prior to delamination, after delamination, and after decellularization using an exemplary method according to certain aspects of this disclosure.

### DETAILED DESCRIPTION

This disclosure provides methods, systems, and compositions in the field of medical grafts, and particularly, relates to tissue processing. The disclosure relates to methods of processing tissue including processing methods for the manufacturing of implantable tissue grafts or cells. The processed tissues made by the systems and methods described herein may be useful in various industries including, amongst others, orthopedics, reconstructive surgery, dental surgery, and cartilage replacement.

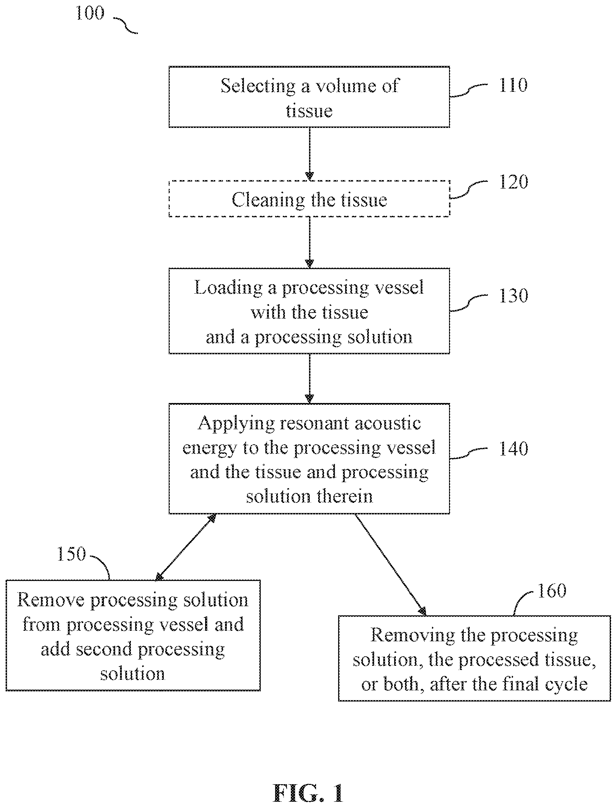

**FIG. 1** shows exemplary method 100 for tissue processing according to one aspect of the present disclosure. The method 100 may include step 110 of selecting a volume of tissue for processing. Optionally, the method may include step 120 of cleaning the tissue to remove blood and other biological fluids or particulates. The method includes step 130 of loading a processing vessel with the tissue and a processing solution. In step 140, a resonant acoustic field (acoustic resonance) is applied to the processing vessel and the combination of tissue and processing solution therein for a duration of time 140. Step 140 may be repeated a plurality of times. Each application of resonant acoustic energy to the tissue may be considered one cycle. In some instances, when step 140 is repeated (such as when method 100 comprises multiple cycles), step 150 of removing the processing solution in the processing vessel and replacing it with a second processing solution may be performed. In some instances, the second processing solution is the same as the processing solution placed in the processing vessel in step 130. In some instances, the second processing solution may be a processing solution having one or more different properties or components as compared to the processing solution placed in the processing vessel in step 130. The volume of the second processing solution may be equivalent to, greater than, or less than the volume of the processing solution placed in the processing vessel in step 130. The method 100 further includes step 160 of removing one or both of the processing solution (or the second processing solution; not shown) or the processed tissue after the final application of resonant acoustic energy (cycle). The processed tissue may then be dried, further processed by some other means, further processed using a method according to an embodiment described herein, submerged in a storage solution, or some combination thereof.

It has been discovered that vibration caused by resonant acoustic energy provides a useful, effective, and surprisingly efficient alternative to traditional mechanical impeller agitation or ultrasonic mixing. Resonant acoustic energy may be used to apply low acoustic frequencies and high energy to a mechanical system, which in turn is acoustically transferred to a processing vessel placed within the system. The system operates at resonance and therefore there is a near-complete exchange of energy from the mechanical system to the contents of the processing vessel, and only the contents of the processing vessel absorb energy. The acoustic energy can create a uniform shear field throughout the processing vessel, resulting in rapid dispersion of material. The acoustic energy can introduce multiple small scale intertwining eddies throughout the contents of the processing vessel. As compared with traditionally-used mechanical impeller agitation, resonant acoustic processing mixes by creating microscale turbulence, rather than mixing through bulk fluid flow. Similarly, as compared with traditionally used ultrasonic agitation (such as sonication), resonant acoustic processing uses magnitudes lower frequency of acoustic energy, and enables a larger scale of mixing. An exemplary resonant acoustic vibration device is a Resodyn LabRAM ResonantAcoustic® Mixer (Resodyn Acoustic Mixers, Inc., Butte, Montana). In some instances, the resonant acoustic vibration device may be devices such as those described in U.S. Patent No. 7,866,878 and U.S. Patent Application No. 2015/0146496.

The resonant acoustic energy may increase the rate or efficiency of processing, or both, and the methods may produce products having improved characteristics over tissue products made using conventional methods. Within the processing vessel, resonant acoustic energy applied through resonant acoustic vibration can facilitate the movement of a liquid into and/or throughout tissue. The vibration of resonant acoustic energy may enhance the rate of interaction between tissue and processing solution. The application of resonant acoustic energy may also be effective in increasing the reaction kinetics or mass transfer kinetics of certain tissue processing techniques such as, for example, demineralization or decellularization. As a result, the rate of tissue processing may be increased as compared to typical tissue processing methods that do not use resonant acoustic energy. The application of resonant acoustic energy to a combination of tissue and processing solution may increase the yield in the production process. In some instances, the methods may provide at least one of more uniform, customized, or predictable processed tissues. For instance, the methods disclosed herein may be used to process tissue regardless of its size and shape to produce a processed tissue and, ultimately, a medical graft, that is more uniform in size and composition, among other qualities. In some instances, use of resonant acoustic energy may permit tissue to be processed without the use of harsh conditions that may impact viability of native cells (cells in the tissue) in the long term, such as in a final graft product. In some instances, use of resonant acoustic energy may permit tissue processing to be performed using less harsh conditions or using reduced amounts of reagents, such as expensive reagents or reagents that could impair cell viability long term.

### I. METHODS OF PROCESSING TISSUE

In one aspect, provided is a method of processing a tissue, the method comprising loading a processing vessel with a tissue and applying resonant acoustic energy to the processing vessel, thereby vibrating the processing vessel and the tissue disposed therein to form a processed tissue. There are various applications for this method, depending on the type of tissue. There are multiple factors impacting tissue processing including, but not limited to, the type of tissue, the neutralization method(s) (if any), the amount of time that resonant acoustic energy is applied to the tissue (including any of the total amount of time, the amount of time for any given application, and the intervals of time for a series of applications), the intensity of the resonant acoustic eneregy for any given application, the frequency of the resonant acoustic energy for any given application, the temperature of the system or at which the tissue is maintained during processing, and the machine used to apply the resonant acoustic energy. These factors influence each other and may be selected to influence the properties of the resulting processed tissue including but not limited to the yield of processed tissue, cell or tissue viability, and tissue structural integrity, and the overall processing rate. In some instances, the method of processing tissue further comprises adding at least one of a processing solution or a biological component to the processing vessel with the tissue. In some instances, the tissue placed in the processing vessel is one or more intact portions of tissue. In certain instances, the tissue placed in the processing vessel may be homogenized tissue and the method produces a homogenized tissue product.

In one aspect, provided is a method of processing a tissue, the method comprising loading a processing vessel with a tissue and a processing solution, thereby providing a combination comprising the tissue and the processing solution disposed in the processing vessel; and applying resonant acoustic energy to the processing vessel, thereby vibrating the processing vessel and the combination disposed therein to form a processed tissue. There are various applications for this method, depending on the type of tissue and the type of processing solution. There are multiple factors impacting tissue processing including, but not limited to, the type of tissue, the composition of the processing solution(s), the neutralization method(s) (if any), the amount of time that resonant acoustic energy is applied to the tissue (including any of the total amount of time, the amount of time for any given application, and the intervals of time for a series of applications), the intensity of the resonant acoustic energy for any given application, the frequency of the resonant acoustic eneregy for any given application, the temperature of the system or at which the tissue and processing solution are maintained during processing, and the machine used to apply the resonant acoustic energy. These factors influence each other and may be selected to influence the properties of the resulting processed tissue including but not limited to the yield of processed tissue, cell or tissue viability, and tissue structural integrity, and the overall processing rate. In some instances, the method of processing tissue further comprises adding a biological component to the processing vessel with the tissue and the processing solution. In some instances, the tissue placed in the processing vessel is one or more intact portions of tissue.

The methods provided in this disclosure may be used to process tissue to achieve different results depending on the type of tissue, the processing solution, and aspects of the resonant acoustic energy applied to the tissue. In some instances, the methods provided herein are methods for demineralizing bone. In some instances, the methods provided herein are methods of decellularizing tissue. In some instances, the methods provided herein are methods of washing (cleaning) tissue. In some instances, the methods provided herein are methods for cryopreserving tissue. In some instances, the methods provided herein are methods for reducing the microbial load of tissue, such as by decontaminating the tissue. In some instances, the methods provided herein are methods for preparing stromal vascular fraction from adipose tissue. In some instances, the methods provided herein are methods of homogenizing tissue to form a tissue paste or putty. As a result of these methods, the processed tissue may be demineralized bone tissue, decellularized tissue, tissue mixed with a cryoprotectant, tissue having reduced unwanted biological fluid or particulates (washed tissue), tissue having reduced microbial contamination, stromal vascular fraction, homogenized tissue, or a homogenized tissue product.

The methods of this disclosure may be applied to a variety of types of tissue including, but not limited to, bone, tendon, skin, cartilage, fascia, muscle, nerves, vascular tissue, birth tissue, and adipose tissue. In some instances, the tissue used for processing is obtained from a deceased donor. In some instances, the tissue used for processing is obtained from a living donor. These tissues are further discussed below.

Provided are methods of processing tissue using resonant acoustic energy, the methods encompassing various processing modalities and various tissues. **FIG. 1** shows the steps in a method 100 of processing a tissue according to methods of the present disclosure. The method 100 may include the step 110 of selecting a volume of tissue for processing.

The method may include step 130 of loading a processing vessel with the cleaned tissue and a processing solution. The processing vessel is generally sealed to maintain the combination of the processing solution and tissue therein. In some examples, the volume of processing solution may be between between 360 mL and 2,400 mL.

In some instances, the volume of tissue selected for processing is limited by the capacity of the processing vessel. As the size of the processing vessel increases, the volume of tissue and processing fluid that it can hold increases. In some instances, the volume of the processing solution may be determined by the weight or volume of tissue to be processed. In other instances, the weight or volume of tissue to be processed may be determined by the volume of the processing solution. In some instances, the ratio of the tissue to processing solution may be influenced by the nature of the processing performed on the tissue during the method (i.e. some processing methods preferably having more or less solution relative to tissue).

In some instances, where the method is for demineralizing bone tissue, the ratio of the volume of processing solution (acid solution) to bone tissue weight may be between 100 mL: 5g to 100 mL: 14g. In some instances, the ratio is at least 100 mL: 14g (*i.e.* more solution may be used).

In some instances, where the method is for decellularizing tissue, the ratio of the volume of processing solution to tissue weight may be between 100 mL: 18g to 100 mL:27g. In some instances, the ratio is at least 100 mL:27g (*i.e.* more solution may be used).

In some instances, where the method is for tissue homogenization, the ratio of tissue volume to processing solution may be from 10:1 to 1:1. In some instances, no processing solution is used in the method.

In some instances, where the method is for processing tissue to enhance cell viability, the ratio of the volume of processing solution to tissue weight may be between 100 mL:2g to 100 mL:6g. In some instances, the ratio is at least 100 mL:6g. Such methods include methods of cryopreserving tissue and for combining tissue with nutrients or nutritvie components, such as a cell culture medium, serum, a buffered solution, a saline solution, water, an antibiotic, a cryoprotectant, or a combination thereof.

In some instances, where the method is for cleaning or washing tissue, the ratio of the volume of processing solution to tissue weight may be between 100 mL: 5g to 100 mL:50g. In some instances, the ratio is at least 100 mL:50g (*i.e.* more solution may be used).

In some instances, where the method is for decontaminating tissue or determining microbial load, the ratio of the volume of processing solution to tissue weight may be between 100 mL:5g to 100 mL:50g. In some instances, the ratio is at least 100 mL:50g (*i.e.* more solution may be used).

In some instances, where the tissue is adipose, the ratio of tissue volume to processing solution may be from 1:10 to 2:1. In some embodiments, the adipose tissue may have a volume of 1 cm³ (cc) to 1000 cm³ (cc) and the processing solution may have a volume of 10 mL to 500 mL. The volume of tissue may be increased if the volume of processing solution is increased proportionally.

In some instances, where the tissue is cartilage, the ratio of tissue volume to processing solution may be from 1:10 to 1:1. In some embodiments, the cartilage tissue may have a volume of 1 cm³ (cc) to 500 cm³ (cc) and the processing solution may have a volume of 10 mL to 500 mL. The volume of tissue may be increased if the volume of processing solution is increased proportionally.

In some instances, where the tissue is muscle, the ratio of tissue volume to processing solution may be from 10:1 to 1:1. In some embodiments, the muscle tissue may have a volume of about 1000 cm³ (cc) and the processing solution may have a volume of 100 mL to 1000 mL. The volume of tissue may be increased if the volume of processing solution is increased proportionally.

In some instances, where the tissue is fascia, birth tissue, or tendons, the ratio of tissue volume to processing solution may be from 1:10 to 1:1000. In some embodiments, these tissues may have a volume of about 1 cc, and the processing solution may have a volume of about 10 mL to 1000 mL. The volume of tissue may be increased if the volume of processing solution is increased proportionally.

In some instances, where the tissue is nerves or vasular tissue, the ratio of tissue volume to processing solution may be 1:10 - 1:1000. In some embodiments, these tissues may have a volume of 1 cc, and the processing solution may have a volume of about 10 mL to 1000 mL. The volume of tissue may be increased if the volume of processing solution is increased proportionally.

In some embodiments, a ratio of 10% tissue volume to 70% processing solution volume may be used. Exemplary volumes (sizes) of various tissues that may be processed using the provided methods are set forth in **Tables 1-2.** In some instances, smaller tissue sizes may result in faster processing.

| **Table 1** | |
|---|---|
| Tissue volume for decellularization, cleaning, or removing microbial contamination | |
| **Tendons** | 0.025 cc-5 cc |
| **Skin- partial thickness** | >1 cc |
| **Skin- full thickness** | >1 cc |
| **Cartilage** | 0.025 cc-3 cc |
| **Fascia** | >1 cc |
| **Muscle** | >1 cc |
| **Nerves** | >1 cc |
| **Vascular** | >1 cc |
| **Birth tissue** | >1 cc |

| **Table 2** | |
|---|---|
| Tissue volume for cryopreservation | |
| **Tendons** | 0.025 cc-5 cc |
| **Cartilage** | 0.025 cc-3 cc |
| **Muscle** | >1 cc |
| **Vascular** | >1 cc |
| **Birth tissue** | >1 cc |

The method may then include step 140 of applying an acoustic energy to the processing vessel and the combination of tissue and solution for a duration of time 140. Exemplary equipment for performing step 140 of applying a resonant acoustic energy includes a Resodyn LabRAM™ Resonant Acoustic Mixer (Resodyn Acoustic Mixers, Inc., Butte, Montana). In some instances, the equipment used to apply the resonant acoustic energy may include systems and devices such as described in U.S. Patent No. 7,866,878 and U.S. Patent Application No. 2015/0146496.

In one aspect, the resonant acoustic energy has an intensity (acceleration) and a frequency and is applied for at least one period of time. In some embodiments, the intensity of the resonant acoustic field and the duration of time it is applied may be selected based on the data set forth in **Table 9.**

The frequency is between 15 Hertz and 60 Hertz. In some instances, the frequency may be 15 Hz, 20 Hz, 25 Hz, 30 Hz, 35 Hz, 40 Hz, 45 Hz, 50 Hz, 55 Hz, or 60 Hertz. In some instances, the frequency is 60 Hertz. In some instances, the intensity (acceleration) may be between 10 and 100 times the energy of G-Force (10 G to 100 G). In some instances, the resonant acoustic energy may exert up to 100 times the energy of G-Force on the processing vessel and combination. For example, the intensity may be between 10 and 60 times the energy of G-Force (10 G to 60 G). In another example, the intensity may be between 10 and 70 times the energy of G-Force (10 Gto 70 G). In another example, the intensity may be between 40 and 70 times the energy of G-Force (40 G to 70 G). In another example, the intensity may be between 40 and 60 times the energy of G-Force (40 G to 60 G). In another example, the intensity may be between 60 and 100 times the energy of G-Force (60 G to 100 G) if the temperature of the processing vessel and the combination of the processing solution and tissue therein is maintained at no greater than about 37°C. For example, the temperature may be maintained between 4°C and 37°C. In some instances, if the temperature of the processing vessel and combination therein is maintained at no greater than about 37°C, the intensity may be between 60 and 80 times the energy of G-Force (60 G to 80 G). In some instances, the intensity of the resonant acoustic energy may be modulated during the period of time it is applied to the processing vessel and combination therein such that the resonant acoustic energy has a sequence of a plurality of intensities during the period of application. In some instances, where maintaining cell viability or tissue integrity is not a criteria for the processed tissue, the intensity may be between 60 and 100 times the energy of G-Force (60 G to 100 G) even if the temperature of the processing vessel and the combination of the processing solution and tissue therein rises above 37°C. In some instances, the temperature of the processing vessel and combination therein is maintained below 50°C. In general, temperatures of 50°C and above may result in significant cell death as proteins typically begin to denature at this temperature. In view of this, methods in which the temperature of the processing vessel and combination therein reach temperatures at or above 50°C are provided but the processing time (length of time that the resonant acoustic energy is applied) may be limited to shorter time periods, such as, for example, no more than 10 minutes.

In some instances, the intensity of the resonant acoustic energy applied to homogenize tissue may be 40 G to 70 G and applied for up to about 10 min at a time. In some instances, the intensity of the resonant acoustic energy may be 10 G to 50 G and applied for up to about 45 min at a time.

In some instances, the intensity of the resonant acoustic energy applied to produce stromal vascular fraction tissue may be 40 G to 70 G and applied for up to about 10 min at a time. In some instances, the intensity of the resonant acoustic energy may be 10 G to 50 G and applied for up to about 45 min at a time. In some instances, the intensity of the resonant acoustic energy may be 10 G to 50 G and applied for up to about 45 min at a time.

In some instances, the intensity of the resonant acoustic energy applied for cellular enhancement of tissue may be 40 G to 70 G and applied for up to about 10 min at a time. In some instances, the intensity of the resonant acoustic energy may be 10 G to 50 G and applied for up to about 45 min at a time.

In some instances, where the tissue is cartilage, the intensity of the resonant acoustic energy may be 10 G to 70 G and applied for up to about 10 min at a time. In certain instances, where the tissue is cartilage, the intensity of the resonant acoustic energy may be 10 G to 50 G and applied for up to about 45 min at a time.

In some instances, where the tissue is adipose, the intensity of the resonant acoustic energy may be 40 G to 70 G and applied for up to about 10 min at a time. In certain instances, where the tissue is adipose, the intensity of the resonant acoustic energy may be 10 G to 50 G and applied for up to about 45 min at a time. In some cases, where the tissue is adipose being processed into SVF, the intensity of the resonant acoustic energy may be 10 G to 70 G and applied for up to about 10 min at a time. In certain cases, where the tissue is adipose being processed into SVF, the intensity of the resonant acoustic energy may be 10 G to 50 G and applied for up to about 60 min at a time.

In some instances, where the tissue is skin, the intensity of the resonant acoustic energy may be 10 G to 100 G an applied for up to 60 min. In some cases, where the tissue is skin being deceullarized, a plurality of intensities ranging from 10 G to 60 G may be applied in a series, each intensity applied for up to 10-30 seconds. In one example, the plurality of intensities may comprise 1 sec at 20 G, 10 sec at 60 G, 3 sec at 15 G, 10 sec at 60 G, 3 sec at 15 G, 10 sec at 60 G, 3 sec at 15 G, 10 sec at 60 G, 3 sec at 15 G, and 10 sec at 60 G. Without being bound to any particular theory, the oscilating series of intensities at varying times may facilitate cell lysis by creating a hostile environment in the processing vessel.

In some instances, where the tissue is muscle, the intensity of the resonant acoustic energy may be 40 G for up to about 3 min at a time to 70 G for up to about 10 min at a time.

In some instances, where the tissue is fascia, birth tissue, or tendons, the intensity of the resonant acoustic energy may be 10 G to 70 G and applied for up to about 10 min at a time. In some instances, where the tissue is fascia, birth tissue, or tendons, the intensity of the resonant acoustic energy may be 10 G to 50 G and applied for up to about 60 min at a time.

In some instances, where the tissue is nerves, the intensity of the resonant acoustic may be up to 30 G applied for up to about 30 min.

In some instances, where the tissue is vasular tissue, the intensity of the resonant acoustic may be up to 40 G applied for up to about 30 min.

The resonant acoustic energy is applied to the processing vessel and the combination therein for at least one period of time. In some instances, period of time may be 1 second, 2 seconds, 5 seconds, 10 seconds, 20 seconds, 30 seconds, 1 minute, 5 minutes, 10 minutes, 15 minutes, 20 minutes, 25 minutes, 30 minutes, 35 minutes, 40 minutes, 45 minutes, or a period of time within 5 % of any of these time periods. In some instances, the period of time is between 1 minute and 4.5 hours. In some instances, the resonant acoustic energy is applied only one time to the processing vessel and combination therein. In other instances, the resonant acoustic energy is applied a plurality of times (such as in plurality of cycles). In some instances, where the resonant acoustic energy is applied a plurality of times, the total amount of time that the resonant acoustic field may be between 1 minute and 4.5 hours. In some instances, the resonant acoustic energy may be applied to the processing vessel and the combination therein at least one time, at least twice, at least three times, at least four times, or at least five times. In some instances, the the resonant acoustic energy is applied no more than twice, three times, four times, or five times.

Additional features of the intensity and duration of the resonant acoustic energy are discussed below in **Section A.**

In some instances, method 100 may include step 150 of removing the processing solution from the processing vessel after application of the resonant acoustic energy (cycle) and adding a new processing solution to the processing vessel. Where the method comprises applying the resonant acoustic energy to the processing vessel and combination therein multiple times, step 150 may be performed between each cycle.

Method 100 may further include step 160 of removing the processing solution, the processed tissue, or both from the processing vessel after the final application of resonant acoustic energy and combining the processed tissue with a storage solution.

In some instances, the tissue may be cleaned prior to or after using the provided methods to process the tissue. In some instances, the tissue may be cleaned using systems and methods as described in U.S. Patent Nos. 7,658,888, 7,776,291, 7,794,653, 7,919,043, 8,303,898, and 8,486,344. In some embodiments, the cleaning is performed using conventional cleaning techniques, such as the standard cleaning protocol of the American Association of Tissue Banks (AATB). Other conventional methods of cleaning tissue or tissue graft products may also be used. In some instances, the method 100 may include step 120 of cleaning the selected volume of tissue. According to some embodiments, it may be desirable to clean the tissue before processing to remove blood and other liquids.

In the context of this disclosure, a processing vessel includes any container or vessel that can be sealed to maintain the processing solution and tissue inside of the processing vessel and sustain acoustic resonance energy of up to 100 G while maintaining the integrity of the vessel and the seal. Examples include vessels made of non-reactive plastic or resin, metal, or glass. In some embodiments, the processing vessel is disposable. In some embodiments, the processing vessel is jacketed to accommodate cooling or heating. In some embodiments, the processing vessel is sealed with vacuum processing. In the context of this disclosure, loading means placing a tissue and a processing solution into a processing vessel. That processing vessel may be sealed (e.g., aseptically, or air tight) so as to contain contents therein when resonant acoustic energy is applied. An exemplary processing vessel may be a lidded vessel capable of holding a volume of up to 3,000 mL.

In some instances, after application of the resonant acoustic energy, the processing solution may be removed from the processing vessel and a second processing fluid may be added to the processing vessel, thereby forming a second combination of the processed tissue and the second processing fluid. Resonant acoustic energy may then be applied to the processing vessel and the fresh combination disposed therein. These steps of removing the processing solution and adding a second processing fluid may be repeated more than one time for a given tissue.

In some embodiments, after application of the resonant acoustic energy, the processing solution may be removed from the processing vessel and a volume of fresh processing solution may be added to the processing vessel containing the tissue, thereby forming a fresh combination of tissue and fresh processing solution. Resonant acoustic energy may then be applied to the processing vessel and the fresh combination disposed therein. This process of removal of the processing solution and addition of a fresh volume may be repeated more than one time for a given tissue sample.

In some embodiments, the methods provided herein may further include the step of combining the processed tissue with a storage solution. Storage solutions may include, but are not limited to, a cell culture medium, a buffer solution, a saline solution, sterile water, serum, an antimicrobial solution, an antibiotic solution, a cryopreservation solution containing a cryoprotectant, or combinations thereof.

In some embodiments, the methods may further comprise combining the processed tissue with an aerosolized component. In one example, the aerosolized component may be a cross-linking agent. In another example, the aerosolized component may be an antibiotic.

### A. Overview of Processing Methods

### 1. Bone Demineralization

In one aspect, the methods of tissue processing as provided herein include methods for demineralizing bone tissue. As discussed further below, bone tissue comprises various amounts of minerals. Bone demineralization procedures involve removing mineral components from bone to produce demineralized bone. Demineralized bone matrix (DBM) refers to allograft bone that has had inorganic mineral removed, leaving behind the organic collagen matrix. The American Association of Tissue Banks (AATB) defines demineralized bone matrix as containing no more than 8% residual calcium as determined by standard methods. Using this criteria, fully demineralized bone tissue should have no more than 8% residual calcium. Traditional methods of bone demineralization include mechanical agitation of the bone while in an acidic solution. The acidic solution solubilizes and extracts the minerals (primarily calcium) that is present in the bone tissue and precipitates it out of solution (*e.g.*, where NaCl is used, the precipitate is calcium chloride). Such traditional methods can be lengthy, taking up to 150 minutes, and may have a low and variable yield. As discussed further below, the bone tissue that may be demineralized according to the methods provided herein may be cortical bone, cancellous bone, or a combination thereof. In instances where the provided methods are methods of bone demineralization, the processing solution comprises an acid solution. Such acid solutions are discussed further below in Section B. The processing solution may also comprise a component that facilitates the demineralization process. For example, the processing solution may include a chelating agent to facilitate extraction of calcium ions from the bone tissue. An exemplary chelating agent is EDTA. In another example, the processing solution may comprise water, a saline solution, or a buffer solution to adjust acid concentration, pH, osmolarity, or a combination of any of these characteristics. For example, the processing solution may comprise water or phosphate buffered saline (PBS). In some instances, following processing with the acid solution, the bone tissue may be neutralized using water or a buffer solution to dilute out the acid and prevent over demineralization. An exemplary neutralization solution is PBS.

It has been discovered that resonant acoustic vibration facilitates the movement of the acid solution into the bone tissue so as to facilitate solubilization and extraction of the minerals (primarily calcium) that is present in the bone tissue. Resonant acoustic vibration increases the rate of demineralization and improves yields as compared with traditional demineralization methods. In some instances, the methods provided herein may demineralize bone tissue, including bone pieces that are 1 cm³, 14 cm³, 20 mm x 15 mm x 10 mm, 14 mm x 10 mm x 10 mm, 50 mm x 20 mm x 5 mm, in 5 minutes using a 1 N HCl processing solution, the demineralized bone being at least 50% compressible as to its original shape and size and having a residual calcium content of no more that 8%. In some instances, the demineralization methods provided herein provide an average increase in demineralization efficiency as compared to standard demineralization methods of at least 50% after one exposure to acid solution (either by standard protocols or by applying resonant acoustic energy according to the methods provided herein). In some instances, the average increase in demineralization efficiency is at least 60% after one exposure to acid solution. In some embodiments, the increase in demineralization efficiency is as set forth in **Table 8.** In some instances, the processing time for demineralizatoin is shorter when the size of the bone tissue is smaller. For example, ground or pulverized bone may demineralize faster than larger portions of bone. In some instances, resonant acoustic vibration, utilizes a closed system, which may minimize the risk of contamination to the tissue.

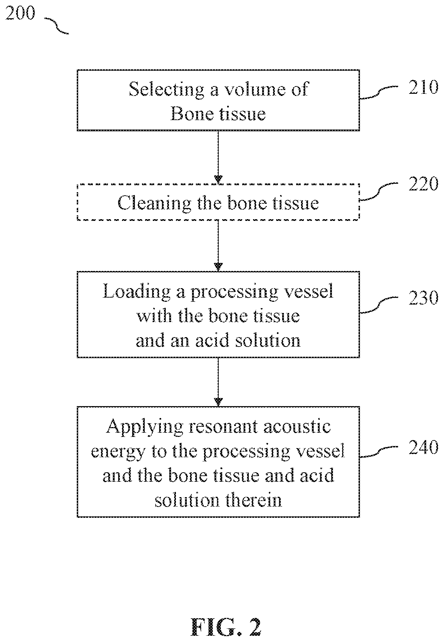

**FIG. 2** shows exemplary method 200 of demineralizing bone according to aspects of the present disclosure. The method 200 may include step 210 of selecting a volume of bone tissue. Examples of bone tissue sizes for use in a demineralization method may include bone tissue having dimension of 8-14 mm by 8-14 mm by 8-14 mm, 9 mm by 8 mm by 112 mm to 25 mm by 16mm by 10 mm, or 20-50 mm by 10-20 mm by 3-5 mm. Smaller portions of bone tissue may result in faster demineralization. The bone tissue may be cortical bone, cancellous bone, or a mixture thereof. In some instances, cortical bone and cancellous bone in bone tissue obtained from a donor are separated from each other and then demineralized. In some instances, the cortical bone and cancellous bone may be demineralized in separate batches. The bone tissue may be of relatively uniform density, free of soft tissue, with no large voids in the cancellous matrix.

The method 200 may optionally include step 220 of cleaning the selected volume of bone tissue. According to some embodiments, it may be desirable to clean the bone tissue before demineralization to remove blood and liquids. In one embodiment, the cleaning may be performed using conventional cleaning techniques, such as the standard bone cleaning protocol of the AATB. In some instances, bone tissue may be cleaned using methods described herein before demineralization using methods described herein.

Method 200 may include step 230 of loading a processing vessel with the cleaned bone tissue and a processing solution. The processing solution generally comprises an acid solution. In some instances, the acid solution is a mineral acid solution. For example, the acid solution may be hydrochloric acid. In some instances, the concentration of the acid solution is between 0.05 N and 5 N. For example, the acid solution concentration may be between 0.05 N and 5 N, between 2.5 N and 3 N, between 0.05 N and 0.1 N, between 0.05 N and 0.5 N, or between 0.1 N and 0.5 N. Depending on the nature of the bone tissue being processed, or the desired final processed bone tissue, different acid solution concentrations may be chosen. In one example, an acid solution of 2.5 N to 3.5 N may be used to demineralize large pieces of bone, particularly cortical bone (which is dense). In another example, an acid solution of 0.1 N to 2 N may be used to demineralize cancellous bone. In another example, an acid solution of 0.5 N to 1 N may be used to demineralize cancellous bone. In another example, an acid solution of 0.05 N to 0.1 N may be used to produce partially demineralized bone. In some instances, the ratio of bone tissue to acid solution is at least 14 g of bone tissue to 100 mL of acid solution. In some instances, the ratio of bone tissue weight to acid solution volume may be less than 1:5. In some instances, the ratio of bone tissue weight to acid solution volume may be 1:10 or greater. In one example, the acid solution (vol) to the bone tissue (g) ratio may be between 50 mL: 1 g and 5 mL: 1 g. In general, increasing the volume of acid solution in relation to the amount of bone tissue does not negatively impact the demineralization process.

Method 200 may then include step 240 of applying resonant acoustic energy to the processing vessel and the combination of bone tissue and acid solution therein for a duration of time. In some instances, the equipment used to apply an acoustic field may be a Resodyn LabRAM™ Resonant Acoustic® Mixer (Resodyn Acoustic Mixers, Inc., Butte, MT). According to some embodiments, the equipment used to apply the resonant acoustic energy may include devices such as those described in U.S. Patent No. 7,866,878 and U.S. Patent Application No. 2015/0146496. In the methods of the invention, the frequency of the the resonant acoustic energy is between 15 Hertz and 60 Hertz. In one example, the frequency may be 60 Hertz. An exemplary acceleration of the acoustic resonance energy is between 10 and 100 times the energy of G-Force. In some instances, the intensity (acceleration) of the the resonant acoustic energy may be between 40 and 60 times the energy of G-Force (40 G to 60 G). The resonant acoustic energy may be applied for 5 minutes, 10 minutes, 20 minutes, 25 minutes, 30 minutes, 35 minutes, 40 minutes, 45 minutes, 50 minutes, 55 minutes, or 60 minutes. In some instances, the resonant acoustic energy may be applied to the processing vessel and combination therein a plurality of times (cycles). For example, resonant acoustic energy may be applied at least once, at least twice, at least three times, at least four times, or at least five times. In some instances, the resonant acoustic energy is applied no more than once, twice, three times, four times, or five times. The acid solution may be removed after each cycle and a new equivalent volume of acid solution may be added to the processing vessel containing the bone tissue before the next cycle. In an exemplary embodiment, the acid solution is removed after the final cycle and the demineralized bone tissue is submerged in sterile water. In some instances, the temperature of processing vessel and the combination of bone tissue and acid solution therein during the application of the resonant acoustic energy is maintained between 15°C and 40°C.

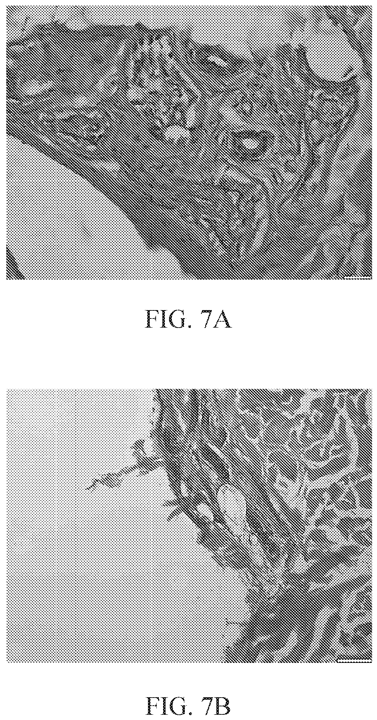

As discussed further below, the demineralization methods of this disclosure produce demineralized bone having a reduced mineral (calcium) content. In some embodiments, the demineralized bone has a calcium content of not more than 8%. In some instances, demineralized bone may be sponge-like. For example, in some cases, the bone may be reshaped (compressed) from an original shape to a subsequent shape, wherein the subsequent shape is between 5% and 99% of the volume of the original shape. Upon release of the compression, the bone can return to its original shape. According to some embodiments, as demonstrated herein in **FIG.** 7, residual calcium content and compressibility of the bone may be correlated such that a compressibility of at least 50% of the original dimensions of bone reflects a residual calcium content of not more that 8%. The evaluation of these characteristics of demineralized bone tissue may be through manual manipulation. For example, by deforming or holding the material in the hand, it is possible to observe how much or how little the bone tissue can be reshaped, for example by noting the extent to which the material can be distorted. The evaluation of these characteristics of demineralized bone tissue may be through machine manipulation.

According to some embodiments, application of the acoustic field to the processing vessel containing the combination is effective to at least partially demineralize the bone tissue in less than 5 minutes, 10 minutes, 15 minutes, 20 minutes, 25 minutes, or 30 minutes.

In some embodiments, where the maximum volume of the processing vessel is 900 mL, the bone tissue may have a volume between 10 mm³ and 500 cm³. This may be the total (bulk) volume of bone tissue in the processing vessel or the volume of any individual piece of bone. In some instances, the bone tissue volume may be 1 cm³, 1.5 cm³, 2 cm³, 2.5 cm³, 3 cm³, 4 cm³, 5 cm³, 7 cm³, 10 cm³, 15 cm³, 20 cm³, 25 cm³, 30 cm³, 35 cm³, 40 cm³, 50 cm³, 60 cm³, 70 cm³, 80 cm³, 90 cm³, or 500 cm³. In some embodiments, an individual portion of bone tissue may have a surface area between 350 mm² and 2700 mm². In some instances, the bone tissue processed according to the provided methods may be between 1 g and 1 kg. In some instances, the amount of bone may be between 1 and 20 g, between 1 g and 50 g. In other instances, the amount of bone may be between 1g and 100 g. For example, the tissue may be 1 g, 2 g, 3 g, 4 g, 5 g, 6 g, 7 g, 8 g, 9 g, 10 g, 12 g, 15 g, 18 g, 20 g, 25 g, 30 g, 35 g, 40 g, 45 g, 50 g, 55 g, 60 g, 65 g, 70 g, 75 g, 80 g, 90 g, 95 g, 100 g, 125 g, 150 g, 175 g, 200 g, 225 g, 250 g, 275 g, 300 g, 325 g, 400 g, 425 g, 450 g, 475 g, 500 g, 525 g, 550 g, 575 g, 600 g, 625 g, 650 g, 675 g, 700 g, 725 g, 750 g, 775 g, 800 g, 825 g, 850 g, 875 g, 900 g, 925 g, 950 g, 975 g, or 1 kg. In some instances, the lowest ratio of bone tissue to processing vessel volume is 500 cm³ of bone tissue to 900 mL processing vessel volume. Thus, where the size of the processing vessel increases, the amount of bone tissue demineralized may increase proportionally.

According to some embodiments, the bone tissue may be cleaned 220 prior to the loading step 230, and the cleaning step may include at least two cycles of dry cleaning and at least two cycles of wet cleaning, when a dry cleaning cycle centrifuges the tissue at 1,500 G for 3 minutes and a wet cleaning cycle centrifuges the tissue and 3% hydrogen peroxide at 1,500 G for 5 minutes. This may be followed by a rinse of the tissue, this rinse may be with sterile water.

### 2. Decellularization

In another aspect, the methods of tissue processing as provided herein include methods for decellularizing tissue. Decellularization is the process of isolating the extracellular matrix of a tissue from its inhabiting cells, leaving an extracellular scaffold which maintains the structural and chemical integrity of the original tissue. Current methods and concepts relating to tissue decellularization are described in Gilbert, T.W. et al. Biomaterials 27:3675-3683 (2006) and Crapo, P.M. et al., Biomaterials 32(12):3233-3243 (2011). Tissue that may be decellularized using the methods described herein include, but are not limited to tendons, skin (partial-thickness and full-thickness), cartilage, fascia, muscle, nerves, vascular tissue, birth tissue, and adipose tissue. These tissues are further discussed below. In some instances, the method of decellularization includes using one or more processing solutions that are described in Gilbert 2006 or Crapo 2011. In some instances, the processing solution may comprise one or more components that facilitate decellularization that are described in Gilbert 2006 or Crapo 2011. In some instances, the rate of decellularization using the methods described herein is faster, taking less time to decellularize tissue in comparison to known standard methods. In some instances, the concentration of reagents/components in the processing solution that cause or facilitate decellularize tissue may be decreased as compared to known standard methods. In some instances, the processed tissue loses at least 40%, 45%, 50%, 55%, 60%, 65%, 70%, 75%, 80%, 85%, 90%, 95%, or 100% cell viability after processing.

In some instances, the methods described herein may decellularize tissue using a processing solution (decellularization solution) that does not contain harsh chemicals or agents that may be detrimental to living tissue (such as tissue that may come into contact with the processed tissue that is made into a graft product and implanted in a patient). For example, in some instances, the processing solution (decellularization solution) may be water alone. In some instances, the decellularization solution does not contain sodium hydroxide (NaOH) or contains a relatively low concentration of sodium hydroxide as compared to standard tissue decellularization protocols (0.05 - 1.0 N). However, in some instances, the processing solution may contain 0.05 - 1.0 N NaOH. For example, the processing solution may contain 0.1 N NaOH. In some instances, the decellularization method comprising a step of exposing the tissue to a saline solution prior to applying the resonant acoustic energy. For example, the tissue may be soaked in a saline solution (5%). In some instances, the tissue may exposed to saline at a temperature of 2-10°C. In some instances, the processing solution for the decellularization process is maintained at a temperature of 30-40°C. In one example, the tissue may be soaked in a 5% saline solution (NaCl) at a temperature of 2-10°C and the resonant acoustic energy may be applied at a temperature of 30-40°C such that the change in salinity and temperature combined with the resonant acoustic energy facilitates cell lysis.

### 3. Cryopreservation

In another aspect, the methods of tissue processing as provided herein include methods for cryopreserving tissue. Cryopreservation is a process wherein biological material such as cells, tissues, extracellular matrix, organs, or any other biological constructs susceptible to damage caused by unregulated chemical kinetics are preserved by cooling to very low temperatures (typically -80°C or -196°C). At low enough temperatures, any enzymatic or chemical activity that might cause damage to the biological material in question is effectively stopped. Cryopreservation methods seek to reach low temperatures without causing additional damage caused by the formation of ice during freezing by freezing the biological material in the presence of cryoprotectant molecules. Traditional cryopreservation methods typically rely on coating the material to be frozen with a the cryoprotectant molecules. The cryoprotectants (also known as cryoprotective agents or cryopreservatives) protect the biological material from the damaging effects of freezing (such as ice crystal formation and increased solute concentration as the water molecules in the biological material freeze). In some instances, the methods of cryopreservation described herein permit more thorough exposure of the tissue to the cryoprotectant during processing, permitting deeper penetration of the cryoprotectant into tissue, and thereby resulting in increased cell viability of the tissue following cryopreservation and thawing. In some instances, the methods provided herein produce processed tissue that retains at least two fold greater cell viability after freezing and thawing. In some instances, the processed tissue retains at least 40%, 45%, 50%, 55%, 60%, 65%, 70%, 75%, 80%, 85%, 90% or 95% cell viability after freezing and thawing as determined by the cell count in the tissue before processing and cell count in the tissue after freezing and thawing. In one example, the processed tissue retains at least 50% cell viability as compared to the tissue before processing. The tissues which may be preserved or cryopreserved include but are not limited to cartilage, muscle, vascular tissue, birth tissue, and adipose tissue. These tissues are further discussed below.

### 4. Production of Stromal Vascular Fraction

In another aspect, the methods of tissue processing as provided herein include methods for producing Stromal Vascular Fraction (SVF) from adipose tissue. In some instances, the adipose tissue may be the lipoaspirate obtained from liposuction of excess adipose tissue from a donor subject. SVF generally refers to a cellular component of adipose tissue that includes various cell types including adipose derived stem cells, a type of mesenchymal stem cell (MSC). SVF is a useful source for stem cells, which can be directly administered to a patient for a variety of purposes or can be combined with a tissue graft or a carrier for administration to a patient. Standard methods of producing SVF involve grinding and digesting the adipose tissue for an extended period of time (such as 45-60 minutes) and separating the SVF from the other adipose tissue components through centrifugation and sieving, whereby the SVF is located in a cell pellet. In some instances, the rate of processing the adipose tissue to produce SVF using the methods described herein is faster, taking less time to break up the adipose tissue in comparison to known standard methods and still retaining comparable cell viability for the SVF produced. For example, in some instances, the processing methods of this disclosure may sufficiently break up adipose tissue in 15 min. In some instances, the processing methods of this disclosure may sufficiently break up adipose tissue at least 3 times faster than standard protocols using grinding and enzymatic digestion.

In some instances, the intensity of the resonant acoustic energy may be 40 G to 70 G and applied for up to about 10 min at a time. In some instances, the intensity of the resonant acoustic energy may be 10 G to 50 G and applied for up to about 45 min at a time. In the methods of the invention, the frequency of the resonant acoustic energy is 15 Hertz and 60 Hertz. In certain embodiments, the frequency is 60 Hertz.

In some instances, the provided methods produce SVF comprising at least 10% CD90+ cells. In some instances, the methods produce SVF comprising at least 10%, 12%, 15%, 17%, or 20% CD90+ cells. In some instances, the SVF produced comprises about 10%-20% CD90+ cells. Relative to standard SVF protocols using grinding and enzymatic digestion, the provided methods may produce SVF having at least about two fold greater CD90+ cell content. In some instances, CD90+ expressing cells are generally representative of mesenchymal stem cells. In some instances, the processing solution comprises a cell culture medium, which is discussed further below. In some instances, the processing solution does not contain collagenase or contains relatively low concentrations of collagenase. For example, in some instances, the processing solution may be water alone. In some instances, the processing solution may comprise up to a ratio of about 150,000 - 175,000 Units collagenase to 1000 cm³ (cc) tissue. For example, the processing solution may comprise no more than 150,000 - 175,000 Units of collagenase for 1000 cm³ (cc) of tissue. However, in some instances, the processing solution may comprise a ratio of about 310,000-350,000 Units collagenase to 1000 cm³ (cc) tissue. In some cases, the processing solution may comprise 325,000 Units collagenase for up to about 1000 cm³ (cc) tissue. For example, for up to 1000 cm³ (cc) of tissue, the processing solution may include 15,000 U; 30,000 U; 35,000 U; 45,000 U; 50,000 U; 55,000 U; 60,000 U, 65,000 U; 70,000 U; 75,000 U; 80,000 U; 85,000 U; 90,000 U; 95,000 U; 100,000 U; 110,000 U; 125,000 U; 130,000 U; 145,000 U; 150,000 U; 160,000 U; 175,000 U; 180,000 U; 190,000 U; 200,00 U; 210,000 U; 225,000 U; 240,000 U; 250,000 U; 260,000 U; 275,000 U, 290,000 U; 307,000 U, or another amount within 10% of any of these amounts. If the amount of tissue is increased, the amount of collagenase may be increased proportionally. Lack of or reduced amounts of collagenase during processing may be desirable in instances where residual collagenase in the SVF product may be considered detrimental to living tissue (such as tissue that may come into contact with the SVF when administered or implanted in a patient).

### 5. Cleaning/Washing Tissue

In another aspect, the methods of tissue processing as provided herein include methods for washing tissue. For example, the methods may increase the passage of cleansing solutions or agents through membranes of the tissue, increasing the washing efficiency or increasing the rate of washing. Cleansing solutions and agents are discussed further below. Cleaning or washing of tissue may include the removal of unwanted materials from the tissue such a biological fluids (such as blood) or particulate matter. Tissues that may be washed using the provided methods include but are not limited to cortical bone, cancellous bone, tendons, skin (full-thickness and partial-thickness), cartilage, muscle, nerves, vascular tissue, birth tissue, and adipose tissue. These tissues are further discussed below.

### 6. Microbial Load - Assessing, Reducing

In another aspect, the methods of tissue processing as provided herein include methods for removing or reducing microbial contamination from tissue. Removing or reducing microbial contamination refers to removing, killing, or deactivating microbes present on the tissue (including but not limited to bacteria, viruses, prions, fungi, spore forms, and unicellular eukaryotic organisms such as Plasmodium). Such methods may also be referred to as tissue decontaminating methods. In some instances, the methods provided may increase the passage of an antimicrobial solution (processing solution) through membranes of the tissue, increasing the efficiency or rate of decontamination. In some instances, the methods provided may effectively disrupt existing or forming biofilms. Antimicrobial solutions are discussed further below. In some instances, the antimicrobial solution may contain an antibiotic. In some instances, the antimicrobial solution may kill microbes on contact by causing cell lysis. Tissues that may be decontaminated using the provided methods include but are not limited to cortical bone, cancellous bone, tendons, partial-thickness skin, full-thickness skin, cartilage, muscle, nerves, vascular tissue, birth tissue, and adipose tissue. These tissues are further discussed below.

In another aspect, the methods of tissue processing as provided herein include methods of measuring or determining the microbial contamination (load) of tissue. Tissue intended for the preparation of tissue grafts is generally evaluated for microbial contamination prior to use. Swabs are widely used in the pharmaceutical and medical device industry for evaluating microbial contaminants on small, hard, non-porous manufacturing equipment, in addition to detecting microbial contaminants in environmental monitoring programs. Swabs are also used on porous, freeze-dried, and other frozen tissue, however, there are concerns that swabbing is not sufficiently sensitive or reproducible on such surfaces. The ability of the swab to recover contaminant microorganisms is dependent on two events; the first is its ability to "pick-up" viable contaminants from the surface of the article being swabbed and the second event, is the "release" of any microbial contaminants from the swab into an appropriate growth environment (*e.g.*, solid agar medium or broth). In addition, for some tissue, a swab is not capable of contacting the entire surface area of the tissue (inaccessibility), thus not allowing for complete analysis of the allograft for microbial contaminants.

In the methods of measuring or determining the microbial contamination (load) of tissue provided herein, determining the microbial contamination includes processing the tissue in an extraction fluid, which can subsequently be analyzed for microbial contamination. Extraction solutions may include, but are not limited to a saline solution, a buffer solution, and water. Exemplary extraction solutions are PBS and water. In some instances, during the application of the resonant acoustic energy, microbial contaminants present on the tissue may be transferred to the extraction fluid and maintain their viability so as to permit later testing. In this manner, the extraction fluid can be analyzed for microbial contamination, thus providing a determination of whether or not the tissue itself is contaminated. After applying the resonant acoustic energy to the combination of the processing solution (extraction fluid), the extraction fluid may be assessed to determine at least one of the amount or type of microbial contaminants that were present on the tissue. The analysis of extraction fluid may include, but is not limited to, any of filtering and culturing the filtrate to identify microbial growth, measuring protein levels (particularly microbial proteins), and measuring nucleic-acid levels (particularly microbial nucleic acids). Additional details regarding systems and methods for analyzing extraction fluid for microbial contamination can be found in U.S. Patent No. 8,158,379. In some instances, the methods provided herein may be comparable to or improved over the methods in which tissue is extracted in an extraction fluid and physically agitated (such as by sonication) and are improved over swab methods. In some instances, the methods provided herein may be comparable to or improved over the methods described in U.S. Patent No. 8,158,379. In some instances, aspects and embodiments of the methods described in U.S. Patent No. 8,158,379 can be incorporated into the methods described herein. In some instances, the methods described U.S. Patent No. 8,158,379 may be modified to incorporate the methods described herein.

### 7. Tissue Homogenization

In another aspect, the methods of tissue processing as provided herein include methods for homogenizing tissue. Homogenization refers to the process whereby a tissue, or a tissue and another component, are brought to a state such that all fractions (tissue components, other components) are equally distributed in the homogenized composition. Two types of tissue homogenization methods are provided. In one aspect, the method of tissue homogenization produces a homogenized tissue. In another aspect, the homogenization method produces a homogenized tissue product. The tissues to which homogenization may be applied include adipose tissue and skin tissue. These tissues are further discussed below. In a preferred embodiment, the tissue is adipose tissue. In some instances, the homogenized tissue produced by the provided methods comprises a uniform, soft, viscous (in some instances, creamy), moist substance that is referred to herein as a paste or putty. In some instances, the homogenized tissue produced by the provided methods comprises a tissue powder. As used herein, a homogenized tissue product refers to a homogenized tissue that is homogeneously combined with another component such as, for example, a biological component or a chemical agent. Exemplay biological components include bone particles (such as demineralized bone particles), minced cartilage, cells (such as stem cells), or a combination of any thereof. The homogenized tissue product may be in the form of a paste or putty or may be dehydrated into the form of a powder. In some instances, plastic or metal balls may be included in the processing vessel with the tissue to facilitate homogenization, which can be removed from the final product.

In some instances, the homogenization methods provided herein comprise loading a processing vessel with adipose tissue or skin tissue (and, optionally, a processing solution, thereby providing a combination comprising the tissue and the processing solution disposed in the processing vessel); and applying resonant acoustic energy to the processing vessel, thereby vibrating the processing vessel and the combination disposed therein to form a homogenized tissue, the homogenized tissue being in the form of a putty or paste. In some instances, the tissue placed in the processing vessel is dehydrated (lyophilized), and the homogenized tissue produced by the method is in the form of a powder.

In some instances, the homogenization methods provided herein comprise loading a processing vessel with adipose tissue or skin tissue and a particulate or chemical agent (or both), thereby providing a combination comprising the tissue and the particulate or chemical agent (or both) disposed in the processing vessel. Optionally, a processing solution may also be added to the processing vessel and be a component of the combination therein. Resonant acoustic energy is then applied to the processing vessel, thereby vibrating the processing vessel and the combination disposed therein to form a homogenized tissue product. In some instances, the homogenized tissue product is in the form of a putty or paste with the particulate or chemical agent (or both) uniformly distributed throughout. In some instances, the tissue placed in the processing vessel is dehydrated (lyophilized), and the homogenized product tissue produced by the method is in the form of a powder with the particulate or chemical agent (or both) uniformly distributed throughout. The particulate may be particulate tissue. For example, the particulate may be bone particles, such as ground demineralized bone matrix, minced cartilage, or cells (such as, but not limited to, mesenchymal stem cells or platelet rich plasma). In some instances, chemical agent may be pharmaceutical drug or a thickening agent such as a medical polymer or a polysaccharide).

In some instances, the homogenization methods provided herein comprise loading a processing vessel with homogenized tissue and a particulate or chemical agent (or both), thereby providing a combination comprising the tissue and the particulate or chemical agent (or both) disposed in the processing vessel. Optionally, a processing solution may also be added to the processing vessel and be a component of the combination therein. Resonant acoustic energy is then applied to the processing vessel, thereby vibrating the processing vessel and the combination disposed therein to form a homogenized tissue product, the homogenized tissue product being in the form of a putty or paste with the particulate or chemical agent (or both) uniformly distributed throughout. In some instances, the tissue placed in the processing vessel is dehydrated (lyophilized), and the homogenized product tissue produced by the method is in the form of a powder with the particulate or chemical agent (or both) uniformly distributed throughout. The particulate may be particulate tissue. For example, the particulate may be bone particles, such as ground demineralized bone matrix, minced cartilage, or cells (such as, but not limited to, mesenchymal stem cells or platelet rich plasma). In some instances, chemical agent may be pharmaceutical drug or a thickening agent such as a medical polymer or a polysaccharide).

In some instances, the processing solution helps lubricate and/or liquify the tissue or product. The consistency of the homogenized tissue or homogenized tissue product may be manipulated by the adjusting the volume of processing solution added to the processing vessel, with increased fluidity of the product as increased volumes of the processing solution volume are used and decreased fluidity of the product as less or no processing solution is used. As discussed above, in some instances, the tissue placed in the processing vessel may be dehydrated (lyophilized). In other instances, the tissue placed in the processing vessel may be hydrated and the homogenized tissue or homogenized tissue product comprises a paste or putty. Where the the homogenized tissue or homogenized tissue product comprises a paste or putty, a further step of dehyration (lyophilization) may be performed to produce a lyophilized homogenized tissue or a lyophilized homogenized tissue product.

### 8. Cellular Enhancement

Methods of tissue processing as described herein include methods for enhancing cell viability. Such methods include methods of cryopreserving tissue as described above. In some instances, the methods comprise combining tissue with a processing solution that contains nutrients or nutritive components. Such methods may be used to improve the condition and cell viability of cells in tissue that may be a in a weakened state such as, for example, tissue that has been thawed following cryopreservation or after a period of transportation. In such methods, the processing tissue may comprise cell culture medium, serum, antibiotics, or a combination of any thereof. In other instances, the processing methods may remove harmful environmental factors from a tissue. For example, the method may include the removal of a fixative (cross-linking agent) or a cryoprotectant by processing the tissue with a processing solution that contains no fixative or cryoprotectant. For such instances, the processing solution may comprise a buffer solution, a saline solution, water, or a combination of any thereof. In another example, the method may comprise processing the tissue to adjust the pH or osmolarity of the tissue. For example, the tissue may have been in an environment in which the pH was lower or higher than optimal for eurkaryotic cells, or may have been in an environment in which the osmolarity was greater or less than optimal for eurkaryotic cells, or both. In such intances, the tissue may be processed using a processing solution to adjust the pH or osmolarity of the tissue. For example, the processing solution may be comprise a buffer solution, a saline solution, water, or a combination of any thereof.

### B. Processing Solutions

The methods of tissue processing of this disclosure may use one or more solutions. Different solutions may be used at different stages of the process. The same solution may be used at different stages of the process.

In one aspect, the methods provided involve combining the tissue with a processing solution. In some instances, the processing solution may comprise a demineralization solution or agent, a decellularization solution or agent, a cryopreservation solution or agent, a cleansing solution or agent, an extraction solution, a saline solution, a buffer solution, a cell culture medium, a cell culture component, or water, each of which is described further below. In some instances, the processing solution may comprise a solvent, a detergent, a cross-linking agent, an oxidizing agent, a chelating agent, an antimicrobial solution or agent, a polymer, a cryoprotectant, a disinfectant, or water, each of which is described further below. In some instances, the processing solution may comprise water such as sterile water and super oxidized water. In some instances, the processing solution enhancing cell viability by providing nutrients, by providing protective agents, or by removing harmful environmental components. In some instances, the processing solutions facilitates tissue degradation, useful in method for tissue homogenization and production of stromal vascular fraction.

In some instances, the demineralization solution may be an acid solution such as, for example, a mineral acid. Acid solutions are further described below. In some instances, the demineralization solution may comprise a chelating agent (such as EDTA), a buffer solution (such as PBS), a saline solution, water, or a combination thereof, each of which is described further below.

In some examples, the decellularization solution or agent may be a basic solution (such as a solution containing NaOH), an acid solution, a detergent, a chelating agent, a saline solution, a buffer solution, an tissue digestive enzyme, water, or a combination thereof, each of which is described further below. For example the decellularization solution or agent may comprise sodium hydroxide (NaOH), hydrochloric acid (HCl), hydrogen peroxide (H₂O₂), sodium dodecyl sulfate (SDS), Triton X-100 (C₁₄H₂₂O(C₂H₄O)ₙ(n = 9-10)), EDTA, a saline solution (hypertonic or hypotonic), PBS, or water. In some instances, the decellularization solution does not contain harsh chemicals or agents that may be detrimental to living tissue (such as tissue that may come into contact with the processed tissue that is made into a graft product and implanted in a patient). For example, in some instances the decellularization solution is water alone. In some instances, the decellularization solution may comprise one or more components that facilitate decellularization that are described in Gilbert 2006 or Crapo 2011. For example, various decellularization solutions and agents have been identified as useful for decellularization of different types of tissues. Such known solutions and agents may be incorporated into the decellularization methods provided herein and their action facilitated by the use of resonant acoustic energy. For example, in some instances, the decellularization solution may include a tissue digestive enzyme such as collagenase. In some instances, the decellularization solution does not contain sodium hydroxide or contains a relatively low concentration of sodium hydroxide as compared to standard tissue decellularization protocols, for example at or around 0.1 N. For example, the sodium hydroxide may be 0.05 N to 0.5 N, or 0.08 N to 0.3 N, or 0.09 N to 0.2 N, or 0.1 N to 0.2 N.

In some instances, the processing solution may be cryopreservation solution and comprise a cryoprotectant. Exemplary cryoprotectants include, for example, dimethyl sulfoxide (DMSO), methanol, butanediol, propanediol, polyvinylpyrrolidone, glycerol, hydroxyethyl starch, alginate, and glycols, such as, for example, ethylene glycol, polyethylene glycol, propylene glycol, and butylene glycol. In some instances, combinations of more than one cryoprotectant may be used. In one example, the processing solution may include 6 mol ethyene glycol 1-1 and 1.8 mol glycerol 1-1. In some instances, the processing solution may contain 5% to 30% of a cryoprotectant, or combination of cryoprotectants, in a buffer solution such as cell culture medium. In some instances, the processing solution may comprise serum or platelet rich plasma, or both, and one or more cryoprotectants. In one example, the processing solution comprises cell culture medium containing 10-20% DMSO. In some instances, the cryoprotectant may be a compound that aids in dehydration (*e.g.,* sugars) or formation of a solid state (*e.g.,* polymers, complex carbohydrates).

In some instances, the processing solution may be an antimicrobial solution or agent, such as a a disinfectant or an antibiotic. The antimicrobial solution or agent may include an alcohol (such as isopropyl alcohol), sodium hypochlorite (bleach), a cross-linking agent (such as glutaraldehyde), a detergent (such as sodium dodecyl sulfate (SDS)), an oxidizing agent (such as hydrogen peroxide), an acid solution (such as peracetic acid), an organic solvent (such as acetone), chlorohexidine or salts thereof (e.g., chlorhexidine gluconate), water (including electrolyzed water such as Microcyn™), antibiotics (such as Polymyxin B), or combinations thereof.

In some instances, processing solution may be a cleansing solution or agent (a washing solution). The cleansing or washing solution or agent may be an alcohol, a cross-linking agent, a detergent, an oxidizing agent, an organic solvent, water, or a combination thereof. For example, such solutions and agents include, but are not limited to, isopropyl alcohol (C₃H₈O), hydrogen peroxide (H₂O₂), glutaraldehyde (C₅H₈O₂), acetone (C₃H₆O), sodium dodecyl sulfate (SDS), and water.

In some instances, the processing solution may be an extraction solution for assessing microbial load of a tissue. Extraction solutions may include, but are not limited to a saline solution, a buffer solution, and water. Exemplary extraction solutions are PBS and water.

In some instances, the processing solution used in the methods provided herein facilitates enhancement of cell viability. In some cases, the processing solution may comprise a cryoprotectant as discussed above. In some instances, the processing tissue may comprise cell culture medium, serum, an antibiotic, or a combination of any thereof. In some instances, the processing solution may comprise a buffer solution, a saline solution, water, an antibiotic, or a combination of any thereof.

In some instances, the processing solution used in the methods provided herein facilitates tissue homogenization. Processing solutions for tissue homogenization may include any of a saline solution, a buffer solution, an organic acid solution (such as acetic acid and citric acid), a mineral acid solution (discussed below), an alkaline metal salt solution (such as NaOH and KOH), and water. In some instances, the acid and base solutions may have a concentration of 0.1 N - 0.5 N. Exemplary processing solutions are PBS and water. In some instances, the processing solution may include a tissue digestive enzyme.

In some instances, the processing solution may be an acid solution. Acid solutions may include hydrochloric acid (HCl), acetic acid (CH₃COOH), citric acid (C₆H₈O₇), formic acid (CH₂O₂), ethylenediaminetetraacetic acid (EDTA), nitric acid (HNO₃), propionic acid (C₃H₆O₂), phosphoric acid (H₃PO₄), gluconic acid (C₆H₁₂O₇), malic acid (C₄H₆O₅), tartaric acid (CaH₆O₆), and fumaric acid (C₄H₄O₄). In some instances, the acid solution is a mineral acid. Mineral acids include, but are not limited to, hydrochloric acid (HCl), nitric acid (HNO₃), phosphoric acid (H₃PO₄), sulfuric acid (H₂SO₄), boric acid (H₃BO₃), hydrofluoric acid (HF), hydrobromic acid (HBr), perchloric acid (HClO₄), and hydroiodic acid (HI). In some instances, the acid solution may be ethylenediaminetetraacetic acid (EDTA).

In some instances the processing solution may be a detergent. Detergents used in biomedical laboratories are mild surfactants (surface acting agents), used for the disruption of cell membranes (cell lysis) and the release of intracellular materials in a soluble form. Detergents break protein-protein, protein-lipid and lipid-lipid associations and denature proteins and other macromolecules. Detergents may be ionic, nonionic, zwitterionic, or chaotropic. In some instances, the detergent may be an ionic detergent such as, for example, sodium dodecyl sulfate (SDS), deoxycholate, cholate, or sarkosyl. In some instances, the detergent may be a nonionic detergent such as, for example, Triton X-100, n-dodecyl-β-D-maltoside (DDM), digitonin, Tween-20, orTween-80. In some instances, the detergent may be a zwitterionic detergent such as, for example, CHAPS. In some instances, the detergent may be urea.

In some instances, the processing solution may be a buffer solution. A buffer solution (also referred to as a pH buffer or hydrogen ion buffer) is an aqueous solution consisting of a mixture of a weak acid and its conjugate base, or vice versa. In one example, the buffer solution may be phosphate buffered saline (PBS). In some instances, the buffer solution may be a cell culture medium.

In some instances, the processing solution may comprise a cell culture medium, serum, or a combination thereof. An exemplary media are minimal essential medium (MEM), Dulbecco's Modified Eagle Medium (DMEM), and chondrocyte growth medium. An exemplary serum is fetal bovine serum (FBS). In some instances, the processing solution may comprise one or more antibiotics.

In some instances, the processing solution may be a saline solution. The saline solution may be hypertonic or hypotonic. In some instances, the saline solution is a solution of sodium chloride (NaCl) in water.

In some instances, the processing solution for tissue homogenization, the processing solution for production of SVF, or both, may including a tissue digestive enzyme such as collagenase. Collagenases are enzymes that break the peptide bonds in collagen. They assist in destroying extracellular structures and breaking down tissue structures. The type of collagenase may be selected for use in the processing solution based on the type of tissue to be processed. In some instances, the processing solution may comprise a ratio of about 325,000 Units collagenase to 1000 cm³ (cc) tissue. In some instances, the processing solution may comprise a ratio of about 310,000-350,000 Units collagenase to 1000 cm³ (cc) tissue. In some cases, the processing solution may comprise 325,000 Units collagenase for up to about 1000 cm³ (cc) tissue. In some cases, the processing solution may comprise 310,000-350,000 Units collagenase for up to about 1000 cm³ (cc) tissue. For example, for up to 1000 cm³ (cc) of tissue, the processing solution may include 15,000 U; 30,000 U; 35,000 U; 45,000 U; 50,000 U; 55,000 U; 60,000 U, 65,000 U; 70,000 U; 75,000 U; 80,000 U; 85,000 U; 90,000 U; 95,000 U; 100,000 U; 110,000 U; 125,000 U; 130,000 U; 145,000 U; 150,000 U; 160,000 U; 175,000 U; 180,000 U; 190,000 U; 200,00 U; 210,000 U; 225,000 U; 240,000 U; 250,000 U; 260,000 U; 275,000 U, 290,000 U; 307,000 U, or another amount within 10% of any of these amounts. If the amount of tissue is increased, the amount of collagenase may be increased proportionally.

In some instances, the processing solution may comprises a chelating agent such as, for example, EDTA. In some instances, the processing solution may comprise a cross-linking or fixative agent such as, for example, glutaraldehyde.

### C. Evaluation of Processed Tissue

In some embodiments, the tissue, the processing fluid, or both, are evaluated after application of the resonant acoustic energy to assess at least one characteristic. Such assessment may be made to determine the extent of tissue processing that has occurred. For instance, the assessment may be performed to determine the criteria established as characterizing the final properties of the processed tissue has been achieved. In some instances, the assessment may be performed to determine if at least one more application of resonant acoustic energy is needed to achieve the criteria established as characterizing the final properties of the processed tissue. For example, in some instances, where the processed tissue is assessed and the criteria are not met, resonant acoustic energy may be applied again to the processed tissue (another cycle of resonant acoustic energy application) as described above.

In some instances, where the tissue processing method is a method of demineralizing bone tissue, the characteristics assessed may include include, but are not limited to, one or more of assessment of the calcium content of the tissue, the BMP-2 content of the tissue, the compressibility of the tissue, the presence of hard nodules in the processed tissue, shape of tissue, and/or dimensions of tissue. A compressibility criteria as used herein refers to the ability to deform the processed tissue by compression from its original shape to a compressed shape, the compressed shape being between 5% and 99% of the volume of the original shape, and the springing back of the processed tissue to the original shape following release of the compression. In some instances, the compressibility criteria may be the ability to compress the processed bone to up to at least 50% of its original volume, after which the processed bone regains its original shape. In some instances, this degree of compressibility correlates with a residual calcium content of no more than 8%. As such, compressibility of bone tissue may be assessed to determine if sufficient demineralization has occurred or if further demineralization using additional application of resonant acoustic energy is needed.

In some instances, where the tissue processing method is a method of decellularizing tissue, the characteristics assessed may include, but are not limited to, assessing the tissue for viable cells histologically or metabolically (using reagents such as Presto Blue® reagent or 3-(4,5-Dimethylthiazol-2-yl)-2,5-diphenyltetrazolium bromide (MTT)). Assessing the content of viable cells in the processed tissue will indicate if the tissue has been sufficiently decellularized.

In some instances, where the tissue processing method is a method of cryopreserving tissue, the characteristics assessed may include, but are not limited to, assessing the tissue for viable cells. For example, the viability of cells in the tissue may be assessed metabolically using reagents such as Presto Blue® reagent or MTT. In some instances, the processed tissue is frozen for a period of time (such as at least one week), then thawed, and then assessed for cell viability.

In some instances, where the tissue processing method is a method of reducing the microbial load of tissue, or measuring the extent of microbial load, the characteristics assessed may include, but are not limited to, assessing the tissue for the presence of microbes. For example, in some instances, the tissue may be assessed using standard microbiology culturing techniques. In some instances, the tissue may be assessed for the presence of microbial nucleic acids. For example, the presence of microbe may be assessed by standard filtering followed by plating and *in vitro* culturing, by Most Probable Number (MPN) by serial dilutions (culturing in liquid medium as described in the FDA's Bacteriological Analytical Manual (October 2010), Appendix 1 (author: R. Blodgett) (available at http://www.fda.gov/Food/FoodScienceResearch/LaboratoryMethods/ucml09656.htm), by quantitative PCR, by AT bioluminescence, and by an enzyme-linked immunosorbent assay (ELISA).

### D. Tissues

There are many varieties of tissues that may be processed according to the system and methods of this disclosure. Exemplary tissues that may be processed include bone, tendons, skin, cartilage, fascia, muscle, nerves, vascular tissue, birth tissue, and adipose tissue.

The tissue may be obtained from a donor subject. The donor subject may be a human donor or a non-human animal. Non-human animals include, for example, non-human primates, rodents, canines, felines, equines, ovines, bovines, porcines, and the like. In some instances, the tissue may be obtained from a human donor, or may be derived from tissue obtained from a human donor. In some instances, the tissue may be obtained from a patient intended to receive the tissue graft such that the tissue is autologous to the patient. In some instances, the tissue may be obtained from a subject other than the patient intended to receive the tissue graft, wherein the subject is the same species as the patient, such that the tissue is allogenic to the patient. In some instances, the tissue may be obtained from a non-human animal such that the bone substrate is xenogenic to a human patient.

The tissue may be machined, cut, or processed into a desired final shape before or after processing using the methods described herein. Such shapes include any of those discussed in this disclosure. In some instances, the tissue may be machined, cut, or processed into shapes such as, but not limited to, a cube, a strip, a sphere, a wedge, or a disk.

In some instances, the tissue processed using the method may be bone. Bone is composed of organic and inorganic elements. By weight, bone is approximately 20% water. The weight of dry bone is made up of inorganic minerals such as calcium phosphate (e.g., about 65-70% of the weight) and an organic matrix of fibrous protein and collagen (e.g., about 30-35% of the weight). Both mineralized and demineralized bone can be used for grafting purposes.

The bone tissue may be cancellous bone or cortical bone. In some instances, the bone tissue is cancellous (trabecular) bone. Cancellous bone, also known as spongy bone, can be found at the end of long bones. Cancellous bone is typically less dense, softer, weaker, and less stiff than cortical bone. Cancellous bone may include bone growth factors. Cancellous bone has a trabecullar-like structure formed from an interconnected network of bone projections of variable thickness and length. The projections define voids in the bone. Cortical bone, also known as compact bone, can be found in the outer shell portion of various bones. Cortical bone is typically, dense, hard, strong, and stiff. Cortical bone may include bone growth factors. In some instances, the bone tissue may be cortical bone that has been processed to contain divets, holes, or both. The methods of this disclosure may be used to demineralize bone, wash bone, decontaminate bone, assess microbial load of bone tissue, or more than one of these processes.

Cortical bone and cancellous bone may be obtained from a donor individual using standard techniques. Bone contains several inorganic mineral components, such as calcium phosphate, calcium carbonate, magnesium, fluoride, sodium, and the like. The mineral or calcium content of bone tissue obtained from a donor may vary. In some cases, cortical bone obtained from a donor may be about 95% mineralized, while cancellous bone may be about 35-45% mineralized. In some cases, cortical bone obtained from a donor may be about 73.2 wt% mineral content, while cancellous bone may be about 71.5 wt% mineral content. In some cases, the mineral content of bone tissue obtained from a donor is about 25% prior to demineralization. Additional information regarding the mineral content of bone and issues relating to demineralization can be found in U.S. Patent No. 9,289,452.

In some instances, the tissue processed using the method may be tendon. Tendons are defined as flexible but inelastic cords of strong fibrous collagen tissue that attach muscle to bone. Tendons can be structured as single stranded, double stranded, double bundled, or in other preshaped configurations. Tendons can be processed and used as therapeutic compositions to treat a variety of medical conditions, including the treatment of, among others, the semitendinosus, gracilis, tibialis, peroneus longus, patella ligament, and achilles.

In some instances, the tissue processed using the method may be skin. Skin tissue is the thin outer layer of tissue on the human body. Skin has three layers: the epidermis, the dermis, and the hypodermis. The epidermis is the outermost waterproof layer; the dermis contains tough connective tissue, hair follicles, and sweat glands; and the hypodermis is the deeper subcutaneous tissue made of fat and connective tissue. Skin can be processed as either full-thickness skin or partial-thickness skin, depending on whether it includes the fat component of the hypodermis or just the outermost skin components. Partial-thickness skin contains the epidermal layer and a thin layer of dermis. It may be recovered from a donor with a dermatome, the recovery of which sets the overall thickness of the recovered partial-thickness skin. In some instances, full-thickness skin may have a thickness of about 1 mm to 5 mm. In some instances, partial-thickness skin may have a thickness of about 0.2 mm to 2 mm. In some instances, skin tissue as described in U.S. Patent Publication No. 2014/0271790 may be processed according to the provided methods.

In some instances, the tissue processed using the method may be cartilage. Cartilage can be defined as flexible but inelastic cords of strong fibrous collagen tissue that cushions bones at joints or makes up other parts of the body. Cartilage tissue can be found throughout the human anatomy. The cells within cartilage tissue are called chondrocytes. These cells generate proteins, such as collagen, proteoglycan, and elastin, that are involved in the formation and maintenance of the cartilage. Hyaline cartilage is present on certain bone surfaces, where it is commonly referred to as articular cartilage. In some instances, the tissue may be articular cartilage. Articular cartilage contains significant amounts of collagen (about two-thirds of the dry weight of articular cartilage), and cross-linking of the collagen imparts a high material strength and firmness to the tissue. These mechanical properties are important to the proper performance of the articular cartilage within the body. In some instances, cartilage tissue as described in U.S. Patent Nos. 9,186,380; 9,186,253; and 9,168,140 may be processed according to the provided methods. In some instances, viability of native chondrocytes in the processed cartilage tissue may be important for utility of cartilage grafts made therefrom. Articular cartilage is not vascularized and, when damaged (such as by trauma or degenerative causes), has little or no capacity for in vivo self-repair. Processed cartilage comprising viable native chondrocytes may facilitate healing of such damage upon implantation by providing both structural support and a source of chondrocytes that may facilitate chondrogeneis *in situ,* filling in defects and integrate with existing native cartilage and/or subchondral bone at the treatment site.

In some instances, the tissue processed using the method may be fascia. Fascia can be defined as the layers of fibrous material within the body that surround muscles and other anatomical features. For example, an abundance of fascia connective tissue can be found at the quadriceps and inner or frontal thigh areas. Typically, fascia is flexible and contains collagen fibers which have been formed by fibroblasts. Embodiments of the present disclosure encompass techniques for developing fibers or filaments from fascia, processing the fibers or filaments into surgical products, and administering such products to recipient patients. In some instances, fascia as described in U.S. Patent Publication No. 2014/0271790 may be processed according to the provided methods.

In some instances, the tissue processed using the method may be muscle. Muscle can be defined as a band or bundle of fibrous tissue that has the ability to contract. Muscle can be processed and used as therapeutic compositions to treat a variety of medical conditions.

In some instances, the tissue processed using the method may be nerves. Nerves can be defined as a bundle of fibers that use electrical and chemical signals to transmit sensory and motor information from one body part to another. Nerves can be processed and used as therapeutic compositions to treat a variety of medical conditions.

In some instances, the tissue processed using the method may be vascular tissue. Vascular tissue can be defined as the tissue that transports nutrients, including veins and arteries. Vascular tissue can be processed and used as therapeutic compositions to treat a variety of medical conditions.

In some instances, the tissue processed using the method may be birth tissue. Birth tissue may include the amniotic sac (which includes two tissue layers, the amnion and chorion), the placenta, the umbilical cord, and the cells or fluid contained in each. In some instances, birth tissue as described in U.S. Patent Publication Nos. 2012/0083900 and 2013/0204393 may be processed according to the provided methods. Amnion is the innermost layer of the placental membranes. It is a thin semi-transparent membrane normally 20 µm to 500 µm in thickness. The amnion comprises a single layer of ectodermally derived columnar epithelial cells adhered to a membrane comprised of collagen I, collagen III, collagen IV, laminin, and fibronectin which in turn is attached to an underlying layer of connective tissue. The connective tissue includes an acellular compact layer of reticular fibers, a fibroblast layer and a spongy layer consisting of a network of fine fibrils surrounded by mucus. The thicker chorion tissue contains all of the vascular vessels and capillaries, nerves and majority of the cells, although a single layer of specialized epithelial cells line the inner-most surface of the amnion tissue (the side closest to the baby). Amniotic membrane has been used for many years in various surgical procedures where anti-scar formation is desired such as, for example, treatment of skin, ocular surface, spine, knee, child birth-related injuries, shoulder surgery, spinal surgeries, trauma related cases, cardiovascular procedures, brain/neurological procedures, burn and wound care, etc. The material provides good wound protection, can reduce pain, reduce wound dehydration, and provide anti-inflammatory and antimicrobial effects. In some instances, amnion tissue may also be used as a surgical dressing.

In some instances, the tissue to be processed may be adipose tissue. Adipose tissue can be defined as loose connective tissue composed of adipocytes which is located throughout the body, including under the skin and in deposits between the muscles and around organs. In some instances, adipose tissue as described in U.S. Patent Publication No. US 2014/0056865 may be processed according to the provided methods.

In some instances, an adipose matrix material is provided as an allogeneic delivery vehicle. Embodiments of the present disclosure encompass pure, clean matrix materials, which are derived from a tissue that is common to the vast majority of the human body. Hence, the matrix materials are usefully applicable to a wide range of injured/surgical sites. Adipose derived matrix systems and methods can be used to deliver various types of materials to a treatment site within the human body. For example, an ostebiologic composition containing cells, proteins, and/or large molecules, combined with an adipose derived matrix, can be administered to a patient.

### II. COMPOSITIONS

In one aspect, provided in this disclosure are processed tissues or cell populations (also called processed tissue or cell population composition, graft, composition, composite graft, or tissue graft) made using the methods described herein. Such processed tissues and cell populations are useful for implantation into a subject such as at a tissue defect site. The processed tissues and cell populations provided herein have improved characteristics over comparable processed tissues and cell populations made using conventional, known methods. In some instances, the processed tissues and cell populations may have increased cell viability. In other instances, the processed tissues and cell populations may have increased cellular components of interest. In some cases, the processed tissues and cell populations do not include, or contain a relatively reduced amount of, chemical processing agents that may be detrimental to living cells (such as upon implantation at a defect site in a subject).

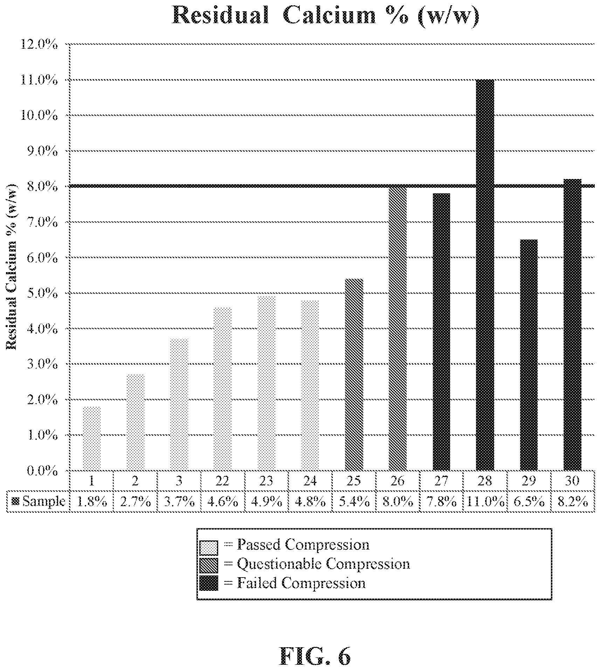

In some instances, the processed tissue comprises demineralized bone. The demineralized bone may be demineralized cancellous bone, demineralized cortical bone, or a combination thereof. The demineralized bone may be in larger intact pieces or may be ground bone. In some instances, the bone is fully demineralized bone. In other instances, the bone is partially demineralized bone. In one aspect, fully demineralized bone comprises no more than 8% residual calcium content. In some instances, fully demineralized bone may be compressible up to 50 % of its original size. In some instances, the demineralized bone may also be free of hard nodules. In some instances, the bone morphogenic protein (BMP) content of the demineralized bone is greater than that of demineralized bone prepared using conventional demineralization procedures. Without being bound to any particular theory, the increased rate at which demineralization may be performed using the provided methods reduces the length of time that the bone tissue is exposed to the harsh acid solution and may, as a result, reduce the damage to native proteins in the tissue. In some instances, the BMP content is at least 10%, 15%, 20%, 25%, 30%, 35%, 40%, 45%, 50%, 55%, or 60% greater than tissue demineralized using conventional demineralization processes (such as those including exposure to HCl solutions for over 1 hour). In some embodiments, the processed tissue comprises demineralized cancellous bone comprising no more than 8% calcium. In some embodiments, the demineralized cancellous bone has a relatively uniform density. In some embodiments, the demineralized cancellous bone contains no large voids defined in the cancellous matrix. In some embodiments, the demineralized bone is compressible to 5% to 99% of the original volume of the tissue and can spring back to the original shape following compression. In some embodiments, the demineralized bone contains no soft tissue. Demineralized bone compositions according to certain embodiments are described in **Tables 3-8** and **FIG. 6****,** a significant improvement in demineralization efficiency of bone tissue can be observed after a single resonant acoustic processing cycle, as compared with standard methodologies which require lengthy processing.

In some instances, the processed tissue comprises decellularized tissue. The decellularized tissue may be cartilage, adipose, skin, muscle, birth tissue, tendon, fascia, nerves, or vascular tissue. In some instances, the tissue is processed without using any harsh chemical agents, the decellularized tissue does not contain any residual amounts of chemical agents. For example, sodium hydroxide is a common decellularization reagent and residual amounts may be present in decellularized tissue prepared using it. In some cases, where the tissue is decellularized in water, saline, or a buffer solution, the processed tissue does not contain any residual harsh chemical agents. In some instances, the processed tissue may contain minimal amounts of chemical agents, such minimal amounts being less than the amounts of such chemical agents found in tissue decellularized using conventional methods. In some instances, the use of resonant acoustic energy in the decellularization method may permit minimal amounts of chemical agents to be used. In some embodiments, as set forth in FIG. 12, using the methods disclosed herein, skin can be decellularized in a shorter period of time using sterile water, as compared with a traditional longer processing method requiring extended exposure to NaOH.

In some instances, the processed tissue comprises cryopreserved tissue. The cryopreserved tissue may be cartilage, adipose, skin, muscle, birth tissue, tendon, fascia, nerves, or vascular tissue. In some instances, the cryoporeserved tissue comprises an increased proportion of viable native cells as compared to tissue preserved using standard cryopreservation methods. Without being bound to any particular theory, the methods of cryopreservation described herein may permit more thorough exposure of the tissue to the cryoprotectant during processing by permitting deeper penetration of the cryoprotectant into tissue, thereby resulting in increased cell viability of the tissue following cryopreservation and thawing. In some instances, the cryopreserved tissue retains at least two fold greater cell viability after freezing and thawing. In some instances, the processed tissue retains at least 40%, 45%, 50%, 55%, 60%, 65%, 70%, 75%, 80%, 85%, 90% or 95% cell viability after freezing and thawing as determined by the cell count in the tissue before processing and cell count in the tissue after freezing and thawing. In one example, the cryopreserved tissue retains at least 50% cell viability as compared to the tissue before processing. In some embodiments, as set forth in **Table 10,** processing tissue according to the methods disclosed herein significantly increases the viability of cryopreserved tissue as compared to controls prepared using a conventional cryopreservation method.

In some instances, the SVF is produced without using digestive enzymes (such as collagenase), and the SVF does not contain any residual amounts of digestive enzymes. In some cases, where the tissue is decellularized in water, saline, a buffer solution, or a cell culture medium, the SVF does not contain any residual digestive enzymes. In some instances, the SVF may contain minimal amounts of chemical agents, such minimal amounts being less than the amounts of such chemical agents found in SVF made using conventional methods. In some instances, the use of resonant acoustic energy in the producing SVF may permit minimal amounts of digestive enzymes to be used. For example, in some instances, the SVF is produced using at least two fold less digestive enzymes than SVF made by conventional protocols, and the SVF contains relatively less residual amounts of digestive enzymes. In some instances, the SVF comprises at least 10% CD90+ cells. In some instances, the methods produce SVF comprising at least 10%, 12%, 15%, 17%, or 20% CD90+ cells. In some instances, the SVF produced comprises about 10%-20% CD90+ cells. Relative to SVF made using standard protocols (grinding and enzymatic digestion), the provided methods may produce SVF having at least about two fold greater CD90+ cell content. In some instances, the processing solution comprises a cell culture medium, which is discussed further below. In some instances, the processing solution does not contain collagenase or contains relatively low concentrations of collagenase. For example, in some instances, the processing solution may be water alone. In some instances, the processing solution may comprise up to a ratio of about 150,000 - 175,000 Units collagenase to 1000 cm³ (cc) tissue. For example, the processing solution may comprise no more than 150,000 - 175,000 Units of collagenase for 1000 cm³ (cc) of tissue. For example, the processing solution may comprise 0 - 150,000 - 175,000 Units of collagenase for 1000 cm³ (cc) of tissue. Lack of or reduced amounts of collagenase during processing may be desirable in instances where residual collagenase in the SVF product may be considered detrimental to living tissue (such as tissue that may come into contact with the SVF when administered or implanted in a patient). In some instances, the SVF produced by the methods provided herein may be a relatively cleaner product comprising primarily cells and with minimal fibrous tissue components. Without being held to any particular theory, in some instances, the reduced processing time and reduced digestive enzyme used in the provided methods may result in less degradation of the extracellular matrix of the tissue during processing. In some embodiments, as set forth in **Table 11,** SVF may be processed from adipose tissue using to the methods disclosed herein and such processing may be performed in significantly less processing time, while using less enzyme, and have no negative effect on cell viability.

The demineralized bone and decellularized tissue of this disclosure may be used to prepare a variety of allograft compositions. In some embodiments, at least a portion of the processed tissue or cell population composition is combined with cells such as stem cells. For example, in some embodiments, the processed tissue or cell population composition is seeded with stem cells, such as mesenchymal stem cells.

In some embodiments, mesenchymal stem cells may be seeded onto and adhered to demineralized bone or decellularized tissue. In some instances, the mesenchymal stem cells are not cultured *ex vivo* (*e.g.,* on a plastic dish) prior to seeding the cell suspension on the demineralized bone and are not cultured (grown) on the demineralized bone or decellularized tissue. In some instances, the demineralized bone and decellularized tissue may be used to manufacture allografts such as those described in U.S. Patent Publication Nos. 2010/0124776, 2014/0024115, and 2014/0286911.

In some instances, the homogenized tissue provided herein may be a useful biological carrier for particles, cells, and other materials. The homogenized tissue may be adipose or skin. In a preferred embodiment, the homogenized tissue is adipose tissue. In some instances, the homogenized tissue comprises uniform, soft, viscous (in some instances, creamy), moist substance that is referred to herein as a paste or putty. In some instances, the homogenized tissue may be a powder, either formed from dehydrated tissue or dehydrated after homogenization. Homogenized tissue can operate as a putty, carrier, or glue, optionally for rendering granular particles into a moldable packable homogenized tissue product. Similarly, the homogenized tissue may be a useful carrier for pharmaceutical drugs (such as antibiotics), permitting such drugs to readily be administered at a defect site. For example, homogenized tissue can be combined with bone particles, minced cartilage, or cells, to form a homogenized tissue product in the form of a putty, a paste, or powder having the particulate components uniformly distributed throughout the homogenized tissue. In some instances, homogenized tissue may be combined with any tissue or material so as to improve or enhance the moldability of that tissue or material, for use as a scaffold for implantation at a treatment site within a patient, or as a fixative in non-weight bearing applications. In some instances, the homogenized tissue may be combined with a medical grade polymer or polysaccharide to thicken the consistency of the homogenized tissue product.

In some instances, the homogenized tissue product comprises biological components distributed uniformly throughout. The homogenized tissue product may be adipose tissue or skin tissue combined with a biological particulate or a chemical agent. In some instances, the biological particulate may be bone particles, such as ground demineralized bone matrix, minced cartilage, or cells (such as, but not limited to, mesenchymal stem cells or platelet rich plasma. In some instances, chemical agent may be pharmaceutical drug or a thickening agent such as a medical polymer or a polysaccharide). In some instances, the homogenized tissue product comprises uniform, soft, viscous (in some instances, creamy), moist substance that is referred to herein as a paste or putty. In some instances, the homogenized tissue product is a powder. Homogenized tissue product may be in the form of a moldable packable product. Similarly, the homogenized tissue product may be a useful carrier for pharmaceutical drugs (such as antibiotics), permitting such drugs to readily be administered at a defect site. For example, homogenized tissue product can be combined with bone particles, to form a putty or a paste having the bone particles uniformly distributed throughout the homogenized tissue product. In some instances, homogenized tissue product may be combined with any tissue or material so as to improve or enhance the moldability of that tissue or material, for use as a scaffold for implantation at a treatment site within a patient, or as a fixative in non-weight bearing applications. In some instances, the homogenized tissue product may be combined with a medical grade polymer or polysaccharide to thicken the consistency of the homogenized tissue product.

In some embodiments, the processed tissue or cell population composition may be combined with one or more other biological components. For example, in some embodiments, at least a portion of the processed tissue or cell population may be coated or combined with a biological adhesive. Suitable biological adhesives include, but are not limited to, fibrin, fibrinogen, thrombin, fibrin glue (e.g., TISSEEL), polysaccharide gel, cyanoacrylate glue, gelatin-resorcin-formalin adhesive, collagen gel, synthetic acrylate-based adhesive, cellulose-based adhesive, basement membrane matrix (e.g., MATRIGEL®, BD Biosciences, San Jose, CA), laminin, elastin, proteoglycans, autologous glue, and combinations thereof.

In some embodiments, at least a portion of the processed tissue or cell population composition may be combined with one or more growth factors. Suitable growth factors include, but are not limited to, transforming growth factor-beta (TGFβ), fibroblast growth factor (FGF) (*e.g.,* FGF2, FGF5), bone morphogenetic protein (BMP) (*e.g.,* BMP2, BMP4, BMP6, BMP7), platelet derived growth factor (PDGF), and insulin-related growth factor (IGF) (*e.g.,* IGF1, IGF2). In some embodiments, the processed tissue or cell population composition may be at least partially coated with, or combined with, a biological adhesive prior to adding the one or more growth factors and/or seeding the cells (*e.g.*, mesenchymal stem cells) on the processed tissue or cell population composition.

### III. SYSTEMS

Systems for performing the methods of processing tissue are described herein.

In one aspect, systems useful for manufacturing tissue grafts of the disclosure are described. The systems include various components. As used herein, the term "component" is broadly defined and includes any suitable apparatus or collections of apparatuses suitable for carrying out the manufacturing methods described herein. The components need not be integrally connected or situated with respect to each other in any particular way. Embodiments include any suitable arrangements of the components with respect to each other. For example, the components need not be in the same room. However, in some instances, the components are connected to each other in an integral unit. In some instances, the same components may perform multiple functions.

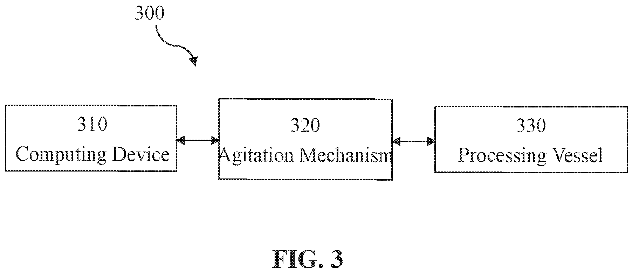

Turning to the drawings, **FIG. 3** depicts a schematic of representative system 300 for manufacturing the processed tissue described herein. In some embodiments one or more components shown in **FIG. 3** may be omitted. Similarly, in some embodiments, components not shown in **FIG. 3** may also be included.

The system 300 may include a processing vessel 330 that is configured to receive the tissue. The processing vessel 330 is of sufficient size to contain a desired volume of tissue and a desired volume of processing solution. Generally, the processing vessel 330 may be made of a non-reactive plastic or resin, metal, or glass. In some instances, the processing vessel 330 may be a beaker, flask, test tube, conical tube, bottle, vial, dish, or other vessel suitable for containing the tissue and the processing solution in a sealed environment.

In another aspect, the system 300 includes an agitation mechanism 320. In some instances, the agitation mechanism 320 is a resonant acoustic vibration device that applies resonance acoustic energy to the processing vessel and its contents. Low frequency, high-intensity acoustic energy may be used to create a uniform shear field throughout the entire processing vessel, which results in rapid fluidization (like a fluidized bed) and dispersion of material. The resonant acoustic vibration device introduces acoustic energy into the processing solution contained by the processing vessel 330 and the tissue components therein. In some instances, the resonant acoustic vibration device includes an oscillating mechanical driver that create motion in a mechanical system comprised of engineered plates, eccentric weights and springs. The energy generated by the device is then acoustically transferred to the material to be mixed. The underlying technology principle of the the resonant acoustic vibration device is that it operates at resonance. An exemplary resonant acoustic vibration device is a Resodyn LabRAM ResonantAcoustic® Mixer (Resodyn Acoustic Mixers, Inc., Butte, Montana). In some instances, the resonant acoustic vibration device may be devices such as those described in U.S. Patent No. 7,866,878 and U.S. Patent Application No. 2015/0146496.

The system 300 may comprise one or more computing devices such as, for example, computing device 310. Typical examples of computing device 310 include a general-purpose computer, a programmed microprocessor, a microcontroller, a peripheral integrated circuit element, and other devices or arrangements of devices that are capable of implementing the steps that constitute the provided manufacturing processes. The computing device 310 may comprise a memory and a processor. In some instances, the memory may comprise software instructions configured to cause the processor to execute one or more functions. The computing devices can also include network components. The network components allow the computing devices to connect to one or more networks and/or other databases through an I/O interface.

For computing device 310, the software instructions may be configured to cause the processor to coordinate the components of the agitation mechanism 320 to agitate the processing vessel 330 and its contents. For example, the software instruction may cause timed and/or sequential physical, mechanical, or electrochemical adjustment to the components of the agitation mechanism 320 to agitate the processing vessel 330 for one or more periods of time, at one or more agitation speeds, or a combination thereof. In one example, where the agitation mechanism 320 is a resonant acoustic vibration device, the software instructions may include a timed and/or sequential application of resonant acoustic energy of a selected intensity and a selected frequency for a selected period of time. The software instructions may have a range of parameter settings for selection depending on the nature of the tissue, the processing solution, or a combination thereof. In some instances, computing device 310 may be configured as part of the agitation mechanism 320. In another instance, computing device 310 may be separate from but in communication with the agitation mechanism 320.

In some instances, systems of the disclosure include all of the components of system 300. For example, system 300 in its entirety is useful for processing tissue. In other instances, systems of the disclosure may include only some of the components of the system 300. It is contemplated that the systems of the disclosure may also include other components that facilitate the mixing of the tissue with the processing solution to form the processed tissue.

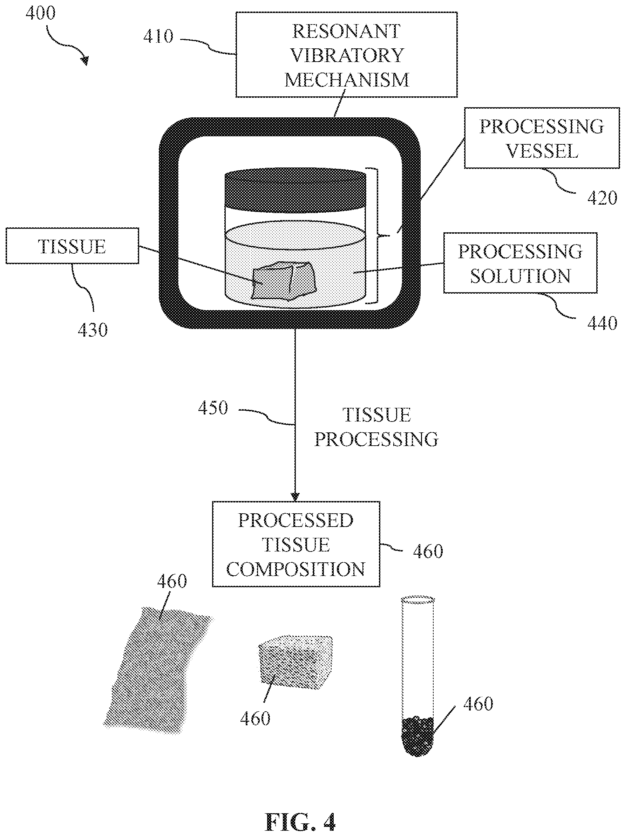

**FIG. 4** shows exemplary system 400 for processing tissue according to aspects of of the present disclosure. The resonant vibratory mechanism 410 may house the processing vessel 420 containing a combination comprising tissue 430 and processing solution 440. The tissue processing 450 occurs within the processing vessel 420 within the resonant vibratory mechanism 410. Application of the resonant acoustic energy to process the tissue 450 produces a processed tissue or processed tissue product 460. In some cases, the tissue 430 can be intact in cubes, strips, blocks, or some other shape. In some cases, the tissue 430 can be ground tissue or minced tissue. In some cases, the tissue 430 can be stromal vascular fraction. In some cases, the tissue 430 may be a tissue paste or putty. In some instances, the processed tissue or processed tissue product 460 retains a similar shape and similar dimensions to the tissue 430. In other instances, particular where the method is for producing homogenized tissue, the tissue 430 may be intact in cubes, strips, blocks, or some other shape, and the homogenized tissue 460 may be a tissue paste or putty. **FIG. 4** is only representative of certain features of the claimed methods and does not show each embodiment or aspect of the claimed methods fully or at all.

Processing vessel 420 is a container or vessel on to which a seal may be applied to maintain the processing solution and tissue inside of the processing vessel and sustain acoustic resonance energy of up to 100 G while maintaining the integrity of the vessel and the seal. Examples include vessels made of non-reactive plastic or resin, metal, or glass. In some embodiments, the processing vessel is disposable. In some embodiments, the processing vessel may be jacketed to facilitate cooling or retention of heat of the processing vessel and the combination therein. In some embodiments, the processing vessel may be vacuum sealed. In the context of this disclosure, loading means placing a tissue and a processing solution into a processing vessel. That processing vessel may be sealed (e.g., aseptically, or air tight) so as to contain contents therein when resoanat acoustic energy is applied. An exemplary processing vessel may be a lidded vessel capable of holding a volume of up to 3,000 mL. In some instances, the processing vessel 420 may hold a volume of up to 500 ml, 1 L, 2 L, or 3 L.

In some instances, the processing vessel 420 and the combination of the processing solution 440 and tissue 420 therein may be maintained at a temperature between 0 °C and 50 °C. In some instances, the resonant vibratory mechanism 410 may comprise a cooling system to facilitate maintaining the temperature of its interior into which the processing vessel 420 is placed.

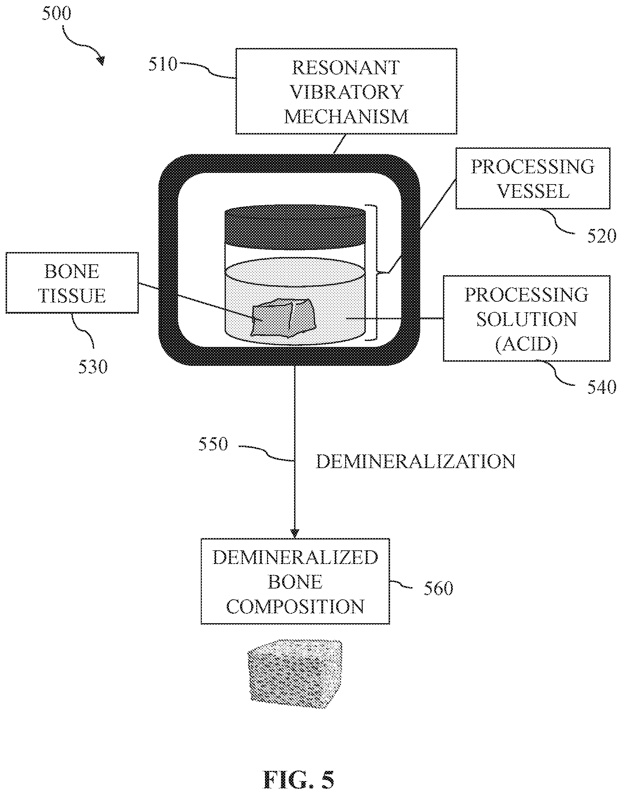

**FIG. 5** shows shows aspects of a bone demineralization system and method according to embodiments of the present disclosure. The resonant vibratory mechanism 510 may house the processing vessel 520 containing a combination comprising bone tissue 530 and processing solution (acid) 540. Processing vessel 520 may be a sealed vessel as discussed above with respect to processing vessel 420 of system 400. The demineralization process 550 can occur within the processing vessel 520 within the resonant vibratory mechanism 510. In some instances, the processed tissue comprises a demineralized bone composition 560. In some cases, the bone tissue 530 can be intact. In some cases, the bone tissue 530 can be ground.

An exemplary acid solution 540 can be a strong acid such as hydrochloric acid, citric acid, acetic acid, propionic acid, phosphoric acid, gluconic acid, malic acid, tartaric acid, fumaric acid, formic acid, ethylene diamine tetra-aceteic acid, or nitric acid. The acid solution 540 may be used at a normality between 0.1N and 12.0 N. The acid solution 540 may have a temperature between 15 °C and 40 °C. The volume of acid solution 540 may be between between 360 mL and 2,400mL.

### IV. EXEMPLARY EMBODIMENTS

Provided below are exemplary, non-limiting embodiments of the methods, systems, and products described in this disclosure.

In one aspect, provided are methods of processing a tissue, the methods including loading a processing vessel with a tissue and a processing solution, thereby providing a combination comprising the tissue and the processing solution disposed in the processing vessel; and applying resonant acoustic energy to the processing vessel, thereby vibrating the processing vessel and the combination disposed therein to form a processed tissue. In the methods of the invention, the resonant acoustic energy has a frequency between 15 Hertz and 60 Hertz. In some instances, the resonant acoustic energy exerts up to 100 times the energy of G-force on the processing vessel and combination. In some instances, the resonant acoustic energy is applied a plurality of times for up to a total time of 2 minutes to 4.5 hours. In some instances, the tissue, the processing solution, or both, are evaluated after application of the resonant acoustic energy to assess at least one characteristic. In some instances, the method further includes removing the processing solution from the processing vessel after application of the resonant acoustic energy; adding a second processing solution to the processing vessel, thereby forming a second combination of the processed tissue and the second processing solution; and applying resonant acoustic energy to the processing vessel, thereby vibrating the processing vessel and the second combination disposed therein. In some instances, the processed tissue is demineralized bone tissue, decellularized tissue, tissue mixed with a cryoprotectant, tissue cleaned of microbial contamination, or homogenized tissue. In some instances, the tissue comprises cortical bone, cancellous bone, cortical and cancellous bone, tendon, skin, cartilage, fascia, muscle, nerves, vascular tissue, birth tissue, or adipose tissue. In some instances, the processing solution comprises a demineralization solution or agent, a decellularization solution or agent, a cryopreservation solution or agent, a cleansing solution or agent, an extraction solution, a saline solution, a buffer solution, a cell culture medium, a cell culture component, or water. In some instances, the processing solution comprises at least one of an acid solution, a basic solution, a buffer solution, a saline solution, an alcohol, an organic solvent, a detergent, a cross-linking agent, an oxidizing agent, a chelating agent, an antimicrobial solution or agent, a polymer, a cryoprotectant, a disinfectant, or water. In some instances, the tissue is bone tissue, the processing solution comprises an acid solution, and the processed tissue is demineralized bone. In some instances, the tissue is adipose, the processing solution comprises a cell culture medium, and the processed tissue is stromal vascular fraction. In some instances, the tissue is adipose or skin, the processing solution comprises cell culture medium, a buffer solution, an acid solution, an alkaline metal salt solution, a solution comprising a digestive enzyme, and the processed tissue is a homogenized tissue. In some instances, the tissue is dried after application of the acoustic field. In some instances, the tissue comprises cleaned tissue. In another aspect, provided are compositions comprising a processed tissue prepared according to the methods provided above.

In another aspect, provided are methods of demineralizing a bone tissue, the methods including loading a processing vessel with a bone tissue and an acid processing solution, thereby providing a combination comprising the bone tissue and the acid processing solution disposed in the processing vessel; and applying resonant acoustic energy to the processing vessel, thereby vibrating the processing vessel and the combination disposed therein to form a demineralized bone tissue. In some instances, the bone tissue comprises cortical bone, cancellous bone, or cortical and cancellous bone. In some instances, the processing acid solution comprises a mineral acid.

In another aspect, provided are methods of cryopreserving a tissue, the methods including loading a processing vessel with a tissue and a processing solution comprising a cryoprotectant, thereby providing a combination comprising the tissue and the cryopreservation processing solution disposed in the processing vessel; applying resonant acoustic energy to the processing vessel, thereby vibrating the processing vessel and the combination disposed therein to form a processed tissue comprising the tissue mixed with the cryoprotectant; and freezing the processed tissue to form a cryopreserved tissue. In some instances, the tissue comprises tendon, skin, cartilage, fascia, muscle, nerves, vascular tissue, birth tissue, or adipose tissue.

In another aspect, provided are methods of decellularizing a tissue, the methods including loading a processing vessel with a tissue and a decellularization processing solution, thereby providing a combination comprising the tissue and the decellularization processing solution disposed in the processing vessel; and applying resonant acoustic energy to the processing vessel, thereby vibrating the processing vessel and the combination disposed therein to form a decellularized tissue. In some instances, the tissue comprises tendon, skin, cartilage, fascia, muscle, nerves, vascular tissue, birth tissue, or adipose tissue. In some instances, the decellularization processing solution comprises a basic solution, an acid solution, a detergent, a chelating agent, a saline solution, a buffer solution, a tissue digestive enzyme, water, or a combination of any thereof. In some instances, the tissue has been soaked in a saline solution prior to loading into the processing vessel.

In another aspect, provided are methods of processing a tissue to produce stromal vascular fraction, the methods including loading a processing vessel with adipose tissue and a processing solution, thereby providing a combination comprising the adipose tissue and the processing solution disposed in the processing vessel; and applying resonant acoustic energy to the processing vessel, thereby vibrating the processing vessel and the combination disposed therein to form a processed tissue. In some instances, the processing solution comprises a saline solution, a buffer solution, a cell culture medium, a cell culture component, or water.

In another aspect, provided are methods of washing a tissue, the methods including loading a processing vessel with a tissue and a washing solution, thereby providing a combination comprising the tissue and the washing solution disposed in the processing vessel; and applying resonant acoustic energy to the processing vessel, thereby vibrating the processing vessel and the combination disposed therein to wash the tissue, the washing comprising removing biological fluids, particulates, or both, from the tissue. In some instances, the tissue comprises cortical bone, cancellous bone, cortical and cancellous bone, tendon, skin, cartilage, fascia, muscle, nerves, vascular tissue, birth tissue, or adipose tissue. In some instances, the washing solution comprises a cleansing solution or agent, a saline solution, a buffer solution, a cell culture medium, a cell culture component, or water.

In another aspect, provided are methods of reducing microbial contamination of a tissue, the methods including loading a processing vessel with a tissue and a processing solution, thereby providing a combination comprising the tissue and the processing solution disposed in the processing vessel; and applying resonant acoustic energy to the processing vessel, thereby vibrating the processing vessel and the combination disposed therein to remove at least a portion of microbial load from the tissue. In some instances, the tissue comprises cortical bone, cancellous bone, cortical and cancellous bone, tendon, skin, cartilage, fascia, muscle, nerves, vascular tissue, birth tissue, or adipose tissue. In some instances, the processing solution comprises an alcohol, an acid solution, an organic solvent, an oxidizing agent, a cross-linking agent, sodium hypochlorite, water, an antibiotic, or combinations thereof.

In another aspect, provided are methods of assessing the microbial contamination of a tissue, the methods including loading a processing vessel with a tissue and a processing solution, thereby providing a combination comprising the tissue and the processing solution disposed in the processing vessel; applying resonant acoustic energy to the processing vessel, thereby vibrating the processing vessel and the combination disposed therein to release microbes from the tissue into the processing fluid; and assessing the processing fluid to determine the microbial load of the tissue. In some instances, the tissue comprises cortical bone, cancellous bone, cortical and cancellous bone, tendon, skin, cartilage, fascia, muscle, nerves, vascular tissue, birth tissue, or adipose tissue. In some instances, the processing solution comprises an alcohol, an acid solution, an organic solvent, an oxidizing agent, a cross-linking agent, sodium hypochlorite, water, or combinations thereof.

In another aspect, provided are methods of homogenizing a tissue, the methods including loading a processing vessel with a tissue; and applying resonant acoustic energy to the processing vessel, thereby vibrating the processing vessel and the tissue disposed therein to form a homogenized tissue. In some instances, the tissue comprises skin tissue or adipose tissue. In some instances, the method further including loading a processing solution into the processing vessel with the tissue, wherein applying resonant acoustic energy to the processing vessel forms a homogenized tissue.

In another aspect, provided are methods of producing a homogenized tissue product, the methods including loading a processing vessel with a tissue and at least one of a biological component or a chemical agent thereby providing a combination comprising the tissue and at least one of a biological component or a chemical agent disposed in the processing vessel; and applying resonant acoustic energy to the processing vessel, thereby vibrating the processing vessel and the tissue disposed therein to form a homogenized tissue product. In some instances, the tissue comprises skin tissue or adipose tissue. In some instances, the tissue comprises homogenized tissue. In some instances, the particulate biological matter comprises ground bone, minced cartilage, or cells. In some instances, the method further including loading a processing solution into the processing vessel with the tissue and the at least one of a biological component or a chemical agent, wherein the processing solution comprises an acid solution, an alkaline metal salt solution, a saline solution, a buffer solution, a solution comprising collagenase, or water.

In another aspect, provided are methods of improving viability of cells in a tissue, the methods including loading a processing vessel with a tissue and a processing solution, thereby providing a combination comprising the tissue and the processing solution disposed in the processing vessel; and applying resonant acoustic energy to the processing vessel, thereby vibrating the processing vessel and the combination disposed therein to form a tissue comprising cells with enhanced viability. In some instances, the tissue comprises cortical bone, cancellous bone, cortical and cancellous bone, tendon, skin, cartilage, fascia, muscle, nerves, vascular tissue, birth tissue, or adipose tissue. In some instances, the processing solution comprises at least one of a media solution, serum, a buffer solution, a solutoin comprising an antibiotic, a solutoin comprising a cryoprotectant, a saline solution, water, or a combination of any thereof.

In another aspect, provided are processed tissue and tissue products made according to any of the above methods.

In another aspect, provided are systems for processing a tissue accoridng to any of the above methods, the systems including a processing vessel; and a high intensity mixing device that applies acoustic resonance energy to the processing vessel disposed therein.

In one embodiment, provided is a method of demineralizing bone tissue, which may include cleaning a volume of bone tissue and subsequently combining the bone tissue with an acid solution in a processing vessel. The method may further include using equipment to apply resonant acoustic energy having a predetermined frequency and a predetermined resonance intensity to the processing vessel and combination of bone and acid therein for a period of time and at a predetermined temperature or within a predetermined temperature range. In some embodiments, the demineralizing solution may be removed at the end of the period of time and replaced with a new volume of demineralizing solution, and the resonant acoustic energy may be applied at least one more additional time. The resonant acoustic energy may be applied a plurality of times to the bone tissue until the bone is demineralized to a target calcium content or handling characteristic, at which point the demineralized bone may be submerged in sterile water. The demineralized bone may subsequently be stored a storage solution or processed further as described above.

In one embodiment, pieces of cancellous bone tissue (10 x 10 x 10 mm, 20 mm x 15 mm x 10 mm, 14 mm x 10 mm x 10 mm, 50 mm x 20 mm x 5 mm) may be cleaned according to standard cleaning protocols and then rehydrated. The tissue may be weighed and grouped based on weight to maintain uniformity between each sample group and processing method. Demineralization may be performed using 1 N HCl as the processing solution and 5 cycles of resonant acoustic energy (60G, 60 Hz), the cycle lengths comprising either 10 min each or 30 min each. The ratio of HCl volume to weight of tissue loaded into the processing vessel may be 1500 ml to 100 g. For example, approximately 26 grams of tissue may be placed into processing vessels for each test group. The processing vessels may be filled to 80% full, which in some instances, comprises approximately 360 ml of HCl. In some instances, pieces of cancellous bone tissue may also be deminieralized in parallel using a standard demineralization method in which the bone tissue is stirred at room temperature in IN HCl for 0.5-1 hrs. The same ratio of HCl volume to weight of tissue (specifically, 26 g tissue and 360 mL of HCl) may be used for the control sample. At the completion of five cyles, the processing solution may be removed and the demineralized bone may be placed in a neutralizing solution. In some embodiments, the difference in the compression passing yield rates and the processing time between the methods provided herein and the standard stir plate processing method may be as set forth in **Table 3.** In some embodiments, the provided method may yield demineralized bone with a compression passing rate above 80% in less than 30 minutes. In some instances, there may be little difference between using a 10 min and 30 min cycle time with respect to compression passing yield rates. In one embodiment, five 10 min cycles as described may demineralize bone cubes 15 times faster than the standard protocol (10 min vs 150 min; 1 cycle vs 3 cycles). In one embodiment, five 10 min cycles as described may demineralize large and small blocks of bone 7.5 times faster than the standard protocol (20 min vs. 150 min; 2 cycles vs 3 cycles). In addition, in some embodiments, there may be no "over-demineralization" of tissue.

In another embodiment, pieces of cancellous bone tissue (10 mm³, 14 mm³, 50 mm x 20 mm x 5 mm; 225 total pieces) may be cleaned according to standard cleaning protocols and then rehydrated. The tissue may be weighed and divided into processing sample weights of 26 grams. Each processing sample may be loaded into a processing vessel containing approximately 360 mL of 1 N HCl). Acoustic resonance energy (60 G, 60 Hz) may be applied to the processing vessel containing the combination in five ten-minute cycles. In some embodiments, the described method may result in average percent pass rate based on compressibility, overall structural integrity, or both, may be as set forth in **Table 4** and **Table 5.** In one embodiment, the described method may demineralize 224 out of 225 grafts as measured by compressibility after 5 cycles. In some instances, after 3 cycles, the described method may demineralize 113 out of 120 medium cubes (94.2%) as measured by compressibility after 5 cycles. In some instances, after 2 cycles, the described method may demineralize 26 g each of 14 mm³ bone cubes and 50 mm x 20 mm x 5 mm bone strips as measured by compressibility after 5 cycles. In some instances, compared to the processing time using a standard demineralization protocol, the provided methods may increase yield by approximately 28% and is reduce processing time overall.

In one embodiment, cubes of cancellous bone tissue (10 mm³) may be cleaned according to standard cleaning protocols and then rehydrated. The tissue may be weighed and divided into processing sample weights of 26 grams. Each processing sample may be loaded into a processing vessel containing approximately 360 mL of processing solution (either 1 M or 0.5 M HCl). Acoustic resonance energy (60 G, 60 Hz) may be applied to the processing vessel containing the combination in five ten-minute cycles. After each acoustic resonance energy cycle, the processing solution may be discarded and the tissue assessed for compression. The tissue may then be re-loaded in the processing vessel with a fresh volume of processing solution for each subsequent cycle. At the completion of five cyles, the processing solution may be removed and the demineralized bone tissue may be placed in a neutralizing solution. In one embodiment, the percent of samples meeting the compression criteria after such a processing method may be as set forth in **Table 6** and **Table 7.**

In another embodiment, 30 cubes of cancellous bone tissue (10 mm³) may be cleaned according to standard cleaning protocols and then rehydrated. The cubes may be demineralized according to the methods provided in this disclosure in 750 mL of 1 N HCl in a 32 ounce jar (-80% full) for 1 cycle of 5 min at 50 G intensity and 60 Hz frequency. The tissue may then be assessed for compressibility and residual calcium content. In some embodiments, bone tissue processed by this method may be demineralized as shown in **FIG. 6****.** For example, 80% (24/30) of the tissue samples may be sufficiently demineralized to meet compression criteria. In some instances, 93% (28/20) of the samples may be sufficiently demineralized as assessed by measuring the residual calcium criteria. In some instances, 93% (28/30) samples may be sufficiently demineralized to meet both the residual calcium content and compressibility criteria. In some embodiments, an acid (HCl) exposure time of only 5 minutes using the described method may be sufficient to demineralize bone tissue grafts to meet desired criteria for demineralized bone products. In some instances, a compression criteria of 50% compressibility is comparable to a residual calcium content of not more than 8%.

In some instances, the demineralization methods provided herein provide an average percent increase in demineralization efficiency as set forth in **Table 8.**

In some embodiments, as set forth in **Tabe 9,** resonance acoustic energy applied to cartilage containing viable, native cells according to the provided methods demontrates a wide range of intensities and exposure times at which cell viability is not negatively impacted. For example, cartilage tissue prepared as described in U.S. Patent No. 9,186,253 (8 mm x 1 mm thick disks, laser etched with square pattern) may be combined with chondrocyte cell culture medium and then exposed to resonant acoustic energy having an intensity from 10 G to 100 G for from 10 min to 45 min. In some instances, cartilage processed in this manner with resonant acoustic energy at 10 G to 40 G from 10 min to 45 min retains its original percent viable cells. In some instances, cartilage processed in this manner with resonant acoustic energy at 50 G from 10 min to 40 min retains its original percent viable cells. In some instances, cartilage processed in this manner with resonant acoustic energy at 60 G from 10 min to 20 min retains its original percent viable cells. In some instances, cartilage processed in this manner with resonant acoustic energy at 60 G from 25 min to 445 min, 70 G at 10 - 20 min, and 80 G for 10 - 15 min and retains at least 50% of the original percent viable cells. In some instances, cartilage processed in this manner with resonant acoustic energy at 70 G for 25-45 min, 80 G for 20-45 min, 90 G for 10-45 min, and 100 G for 10-45 min maintained less than 50% of the original viabile percent. In some instances, such conditions where cell viability was reduced as compared to the original percent cell viability, may be usable for processing tissue where the temperature of the processing vessel and its contents are maintained at about 37°C.

In some embodiments, the provided methods using resonant acoustic energy may facilitate cryopreservation of tissue containing living cells. For example, cartilage tissue prepared as described in U.S. Patent No. 9,186,253 (8 mm x 1 mm thick disks, laser etched with square pattern) may be combined with 20% DMSO + 80% cell culture medium and processed at 30 G intensity and 60 Hz frequency for 30 - 45 min. In some instances, after processing, the tissue samples may be cryopreserved in 10% DMSO + 90% FBS for at least 3 months. The cell viability of the starting cartilage tissue and the cryopreserved tissue may be assessed, such as by using a metabolic activity assay. In some embodiments, processing tissue using the described method may significantly increase the viability of the cryopreserved tissue as compared to the control tissue as set forth in **Table 10.** In some instances, there may be at least about a two-fold increase in cell viability for the described methods using resonant acoustic energy as compared to controls cryopreserved using convention cryopreservation methods. Without being held to any particular theory, in some instances, the increase in cell viability of tissue samples cryopreserved using the described methods may be due to the ability of resonant acoustic energy to drive the cryoprotectant into the matrix of the tissue thereby protecting cells that would otherwise be more succeptible to the negative impact of freezing and be destroyed or severely weakened. In embodiments, tissue cryopreserved as described may comprise at least 40% of the original viable cells upon thawing and culturing.

In some embodiments, the provided methods may be useful for processing adipose tissue into stromal vascular fraction (SVF). For example, adipose tissue may be mixed with culture medium containing 0%, 50%, or 100% collagenase and resonant acoustic energy applied thereto, the processing vessels containing the tissue and culture medium also containing at least one ball configured to whisk the contents of the processing vessels when the vessel is agitated or vibrated. The resonant acoustic energy may be applied for 15 min at either 40 G or 50 G and 60 Hertz. Processed tissue may then be sieved (9.5 mm, 4 mm) and passed through mesh (300-500 µm). After phase separation, the supernatant may be centrifuged to generate a pellet comprising the SVF. The cell viability of the SVF and the percent of mesenchymal stem cells may be assessed, such as by asesssing cells for CD90+ expression. In some embodiments, as shown in **Table 11,** the described methods of producing SVF do not negatively impact cell viability. In some instances, adipose tissue processing time may be reduced. In some embodiments, SVF may be produced from adipose using the described methods without the use of collagenase or with only 50% collagenase, as compared to a conventional processing method that uses 100% collagenase. In some embodiments, SVF produced with reduced the amount of collagenase may comprise healthier cells in the long term. In some embodiments, the described methods may also produce SVF comprising a higher content of mesenchymal stem cells (reflected by CD90+ cells). In some embodiments, the described methods may also produce SVF comprising a higher content of CD90+ cells. In some embodiments, the CD90+ cell content of the SVF produced by the described methods may be at least two fold greater than the CD90+ cell content of SVF produced by a conventional processing method.

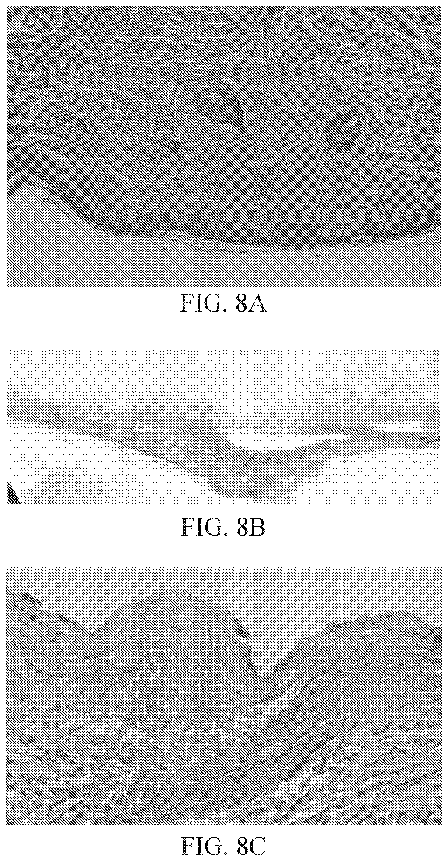

In some embodiments, the provided methods may be used to decellularize skin tissue. The method may be used for either full thickness skin or partial thickness skin. For example, the tissue (250g) may be combined with a saline solution (5%) and agitated for 48 hrs at 2-10°C. The tissue may then be delaminated, mixed with warm water, and vibrated using resonant acoustic energy for three cyles of an oscilating series of intensities as set forth in **Table 12.** Tissue sample may be evaluated histologically to assess extent of decellularization. In some embodiments, as shown in **FIGS. 7A-7B** and **FIGS. 8A-8C****,** full thickness and split thickness skin, respectively, may be decellularized using the described methods. In some embodiments, tissue such as skin tissue may be decellularized without using harsh chemical agents.

In one embodiment, the method may be used to demineralize a human deceased donor bone tissue, including bone tissue that was recovered from a donor. In this embodiment, the method includes loading a processing vessel with a human deceased donor bone tissue and a hydrochloric acid solution, thus providing a combination of the human deceased donor bone tissue and the hydrochloric acid solution in the processing vessel. The method further includes using equipment to apply an acoustic field to the processing vessel and the combination of the human deceased donor bone tissue and the hydrochloric acid solution for a duration of time, the acoustic field having a frequency and a resonance energy, and application of the acoustic field to the processing vessel and the combination of the human deceased donor bone tissue and the hydrochloric acid solution is effective to at least partially demineralize the human deceased donor bone tissue. Up to 5,000 mm³ of human deceased donor bone tissue may be demineralized in between 30 seconds and 30 minutes to result in a human deceased donor bone product that contains less than 8% residual calcium. The human deceased donor bone tissue may be cortical bone, cancellous bone, or cortical and cancellous bone. The hydrochloric acid solution may have a normality between 0.1 N and 2.0 N. The hydrochloric acid solution may have a temperature between 15 °C and 40 °C. In this embodiment, the volume to weight ratio for the hydrochloric acid solution to human deceased donor bone tissue may be between 100 mL:5 g and 100 ml: 17 g. The volume of hydrochloric acid solution may be between 60 mL and 750 mL, and the processing vessel may be a container capable of holding a volume of up to 3,000 mL. The acoustic frequency may be between 15 Hertz and 60 Hertz and the acceleration of the acoustic resonance energy is up to 100 times the energy of G-Force. The human deceased donor bone tissue may have a volume between 500 mm³ and 5,000 mm³ and a surface area between 350 mm² and 2700 mm². The application of the acoustic field may be for a period of time between 5 minutes and 30 minutes. The human deceased donor bone tissue may be cleaned prior to the loading step, wherein the cleaning step may include at least two cycles of dry cleaning and at least two cycles of wet cleaning. A dry cleaning cycle may comprise centrifuging the tissue at 1,500 G for 3 minutes. A wet cleaning cycle may comprise centrifuging the tissue and 3% hydrogen peroxide at 1,500 G for 5 minutes, followed by a rinse of the tissue with sterile water. In this embodiment, the application of the acoustic field for a period of time may be repeated at least once. The hydrochloric acid solution may be removed after the application of the acoustic field and a new volume of hydrochloric acid solution may be added to the processing vessel containing the human deceased donor bone tissue. In this embodiment, the hydrochloric acid solution may be removed after the application of the acoustic field and the human deceased donor bone tissue may be submerged in sterile water. The method may result in a demineralized bone composition. The composition may be of relatively uniform density, free of soft tissue, with no large voids in the cancellous matrix, can be reshaped from an original shape to a subsequent shape that is between 5% and 99% of the volume of the original shape, and can spring back to the original shape following the reshaping protocol.

In another embodiment, the method may be used to demineralize a human deceased donor bone tissue, such as that recovered from a donor. In this embodiment, the method includes loading a processing vessel with a human deceased donor bone tissue and an acid solution, thus providing a combination of the human deceased donor bone tissue and the acid solution in the processing vessel and using equipment to apply an acoustic field to the processing vessel and the combination of human deceased donor bone tissue and the acid solution for a duration of time, the acoustic field having a frequency and a resonance energy. In this embodiment, application of the acoustic field to the processing vessel containing the combination of the human deceased donor bone tissue and the acid solution is effective to at least partially demineralize the human deceased donor bone tissue. Up to 5,000 mm³ of human deceased donor bone tissue may be demineralized in between 5 minutes and 30 minutes to result in an end product that contains less than 8% residual calcium. The acid solution may be citric acid, formic acid, ethylene diamine tetra-acetic acid, or nitric acid, has a molarity between 0.1 M and 12 M, and may have a temperature between 15 °C and 40 °C. The volume to weight ratio for the acid solution to human deceased donor bone tissue may be between 100 mL:5 g and 100 ml:17 g. The volume of acid solution may be between 360 mL and 750 mL and the processing vessel is a container capable of holding a volume of up to 3,000 mL. The acoustic frequency may be between 15 Hertz and 60 Hertz and the acceleration of the acoustic resonance energy may be up to 100 times the energy of G-Force. The human deceased donor bone tissue may have a volume between 500 mm³ and 5,000 mm³ and a surface area between 350 mm² and 2,700 mm². The application of the acoustic field may be for a period of time between 5 minutes and 10 minutes. The human deceased donor bone tissue may be cleaned prior to combining it with the acid solution. The application of the acoustic field for a period of time may be repeated at least once. The acid solution may be removed after the application of the acoustic field and a new volume of acid solution is added to the processing vessel containing the human deceased donor bone tissue. The method may yield a demineralized bone composition. This demineralized bone composition may be of relatively uniform density, free of soft tissue, with no large voids in the cancellous matrix, can be reshaped from an original shape to a subsequent shape that is between 5% and 99% of the volume of the original shape, and can spring back to the original shape following the reshaping protocol.

In another embodiment, the method may be used to demineralize a human deceased donor bone tissue, such as that recovered from a donor. In this embodiment, the method includes loading a processing vessel with a human deceased donor bone tissue and an acid solution, thus providing a combination of the human deceased donor bone tissue and acid solution in the processing vessel, and using equipment to apply an acoustic field to the processing vessel and the combination of the human deceased donor bone tissue and acid solution for a duration of time, the acoustic field having a frequency and a resonance energy, wherein application of the acoustic field to the processing vessel containing the combination of the human deceased donor bone tissue and the acid solution is effective to at least partially demineralize the human deceased donor bone tissue in less than 30 minutes. The human deceased donor bone tissue may be cortical or cancellous bone. The acid solution may be selected from the group containing citric acid, formic acid, ethylene diamine tetra-acetic acid, and nitric acid. The acid solution may have a molarity between 0.1 M and 12 M. The acid solutoin may have a temperature between 15 °C and 40 °C. The volume to weight ratio for the acid solution to the human deceased donor bone tissue may be between 100 mL:5 g and 100 mL:17 g. The volume of acid solution may be between 360 mL and 750 mL. The processing vessel may be a container capable of holding a volume of up to 3,000 mL. The acoustic frequency may be between 15 Hertz and 60 Hertz and the acceleration of the acoustic resonance energy may be up to 100 times the energy of G-Force. The human deceased donor bone tissue may have a volume between 500 mm³ and 5,000 mm³ and a surface area between 350 mm² and 2700 mm². The application of the acoustic field may be for a period of time is between 5 minutes and 10 minutes. The human deceased donor bone tissue may be cleaned prior to combining it with the acid solution, and the application of the acoustic field for a period of time may be repeated at least once. Further, the acid solution may be removed after the application of the acoustic field and a new volume of acid solution is added to the processing vessel containing the human deceased donor bone tissue. The acid solution may be removed after the application of the acoustic field and the human deceased donor bone tissue is submerged in sterile water. The method may yield a demineralized bone composition. The demineralized bone composition may be of relatively uniform density, free of soft tissue, with no large voids in the cancellous matrix, can be reshaped from an original shape to a subsequent shape that is between 5% and 99% of the volume of the original shape, and can spring back to the original shape following the reshaping protocol.

In one embodiment, the method may be used to demineralize a human deceased donor bone tissue, such as that recovered from a donor. In this embodiment, the method includes loading a processing vessel with 26 g of human deceased donor bone tissue and 390 mL of IN hydrochloric acid solution, thus providing a combination comprising the human deceased donor bone tissue and the hydrochloric acid solution in the processing vessel. The method further includes using equipment to apply an acoustic field to the processing vessel and combination of the human deceased donor bone tissue and hydrochloric acid solution for between 10 minutes, the acoustic field having a frequency and a resonance energy, wherein application of the acoustic field to the processing vessel containing the combination of the human deceased donor tissue and acid solution is effective to at least partially demineralize the human deceased donor tissue in less than 30 minutes, resulting in a product that contains less than 8% residual calcium. The human deceased donor bone tissue may be cortical or cancellous bone. The hydrochloric acid solution may have a temperature between 15 °C and 40 °C and the processing vessel may be a container capable of holding a volume of up to 3,000 mL. The acoustic frequency may be between 15 Hertz and 60 Hertz and the acceleration of the acoustic resonance energy may be up to 100 times the energy of G-Force. The human deceased donor bone tissue may be cleaned prior to the combination with the hydrochloric acid solution. The application of the acoustic field for 10 minutes may be repeated at least once and the hydrochloric acid solution may be removed after the application of the acoustic field and a new volume of hydrochloric acid solution may be added to the processing vessel containing the human deceased donor bone tissue. The hydrochloric acid solution may be removed after the application of the acoustic field and the human deceased donor bone tissue is submerged in sterile water. The method may yield a demineralized bone composition. This demineralized bone composition may be of relatively uniform density, free of soft tissue, with no large voids in the cancellous matrix, can be reshaped from an original shape to a subsequent shape that is between 5% and 99% of the volume of the original shape, and can spring back to the original shape following the reshaping protocol.

In another embodiment, the method may be a method of rapid treatment of a human donor tissue, such as that recovered from a donor. In this embodiment, the method may include loading a processing vessel with a human donor tissue and a solution, thus providing a combination of the human donor tissue and solution disposed in the processing vessel, and using equipment to apply an acoustic field to the processing vessel and the combination of the human donor tissue and the solution for a duration of time, the acoustic field having a frequency and a resonance energy. The human donor tissue may be cortical bone, cancellous bone, cortical and cancellous bone, tendons, partial-thickness skin, full-thickness skin, cartilage, fascia, muscle, nerves, vascular tissue, birth tissue, adipose tissue, or stromal vascular fraction. The human donor tissue may be obtained from a deceased donor or it may be obtained from a living donor.

In some instances of this embodiment, the human donor tissue may selected from the group containing tendons, partial-thickness skin, full-thickness skin, cartilage, fascia, muscle, nerves, vascular tissue, birth tissue, adipose tissue, and stromal vascular fraction, and application of the acoustic field to the processing vessel and the combination comprising the human donor tissue and the solution is effective to increase the decellularization reaction speed of the human donor tissue. In some instances, the human donor tissue may be selected from the group containing cartilage, muscle, vascular tissue, birth tissue, adipose tissue, and stromal vascular fraction, and application of the acoustic field to the processing vessel and the combination comprising the human donor tissue and the solution is effective to increase the passage of nutrients through membranes of the human donor tissue. In some instances, the human donor tissue may be selected from the group containing cortical and/or cancellous bone, tendons, partial-thickness skin, full-thickness skin, cartilage, muscle, nerves, vascular tissue, birth tissue, adipose tissue, and stromal vascular fraction, and application of the acoustic field to the processing vessel and the combination comprising the human donor tissue and the solution is effective to increase the passage of cleansing agents through membranes of the human donor tissue. In some instances, the human donor tissue may be selected from the group containing cortical and/or cancellous bone, tendons, partial-thickness skin, full-thickness skin, cartilage, muscle, nerves, vascular tissue, birth tissue, adipose tissue, and stromal vascular fraction, and application of the acoustic field to the processing vessel and the combination comprising the human donor tissue and the solution is effective to clean microbial contamination from the donor tissue. In some instances, the application of the acoustic field to the processing vessel and the combination comprising the human donor tissue and the solution may be effective to disrupt existing or forming biofilms on the donor tissue. In some instances, the application of the acoustic field to the processing vessel and the combination comprising the human donor tissue and the solution may be effective to form a homogenous putty. In some instances, the human donor tissue may be selected from the group containing cortical and/or cancellous bone, tendons, partial-thickness skin, full-thickness skin, fascia, cartilage, muscle, nerves, vascular tissue, birth tissue, adipose tissue, and stromal vascular fraction, and application of the acoustic field to the processing vessel and the combination comprising the human donor tissue and the solution is effective to liberate microorganisms from the donor tissue for microbial contamination analysis.

In some instances of this embodiment, the solution may have a temperature between 0° C and 50° C. The volume to weight ratio for the solution to human donor tissue may be between 100 mL:0.1 g and 100 mL:50 g. In this embodiment, the volume of solution may be between 60 mL and 2,400 mL. The processing vessel may be a container capable of holding a volume of up to 3,000 mL. The acoustic frequency may be between 15 Hertz and 60 Hertz and the acceleration of the acoustic resonance energy is up to 100 times the energy of G-Force. The human donor tissue may have a volume between 0.5 cm³ (cc) and 50 cm³ (cc). The human donor tissue may have a surface area between 0.25 cm² and 30 cm². The application of the acoustic field for a period of time may be between 2 minutes and 4.5 hours. The human donor tissue may be cleaned prior to demineralization, decellularization, cellular enhancement, cleansing, cleaning microbial contamination, or microbial extraction sampling. The application of the acoustic field for a period of time may be repeated at least once. The composition may be evaluated for various characteristics, through manual or machine assessment, to determine if further application of the acoustic field for a period of time is required. The solution may be removed after the application of the acoustic field and a new volume of solution may added to the processing vessel containing the human donor tissue. The solution may be selected from the group containing Hydrochloric Acid, Acetic acid, Citric acid, Formic acid, Ethylenediaminetetraacetic acid, Nitric acid, Propionic acid, Phosphoric acid, Gluconic acid, Malic acid, Tartaric acid, Fumaric acid, Phosphate Buffered Saline, Water, Sodium hydroxide, Hydrogen peroxide, Sodium dodecyl sulfate, Triton X-100, Hypotonic/hypertonic saline solution, Minimum Essential Medium, Dimethyl sulfoxide, Cryo, PEGs, Enzymatic agents, Fetal Bovine Serum, Isopropyl Alcohol, Glutaraldehyde, Acetone, Antibiotic cocktail, Sodium hypochlorite, Super oxidized water *(e.g.,* Microcyn™), Chlorhexidine Gluconate *(e.g.,* Prontosan™), and Paracetic acid solution.

In some instances, the solution may be removed after the application of the acoustic field and the human donor tissue is submerged in a second solution. In this embodiment, the second solution may be selected from the group containing water, Phosphate Buffered Saline, Sodium hydroxide, Hydrogen peroxide, Sodium dodecyl sulfate, Triton X-100, Hypotonic/hypertonic saline solution, Minimum Essential Medium, Dimethyl sulfoxide, Cryo, PEGs, Enzymatic agents, Fetal Bovine Serum, Isopropyl Alcohol, Glutaraldehyde, Acetone, Antibiotic cocktail, Sodium hypochlorite, Super oxidized water *(e.g.,* Microcyn™), Chlorhexidine Gluconate *(e.g.,* Prontosan™), and Paracetic acid solution. The solution may be removed after the application of the acoustic field and the human donor tissue may be dried. In this embodiment, the solution may be removed after the application of the acoustic field and harmonics and air are applied to the processing vessel and any remaining moisture may be vaporized from the human donor tissue. The solution may be removed after the application of the acoustic field and an aerosolized component may be added to the processing vessel containing the human donor tissue. The aerosolized component may selected from the group containing gluteraldehyde, acetic acid, and perchloric acid. In this embodiment, the method may result in a composition. This composition may be an allograft treatment composition, prepared according to the method, and this composition may be combined with stem cells recovered from the same human donor.

### EXAMPLES

### EXAMPLE 1. Bone Demineralization

**Study #1.** The purpose of this study was exploratory to assess the demineralization of cancellous bone grafts produced by a standard demineralization protocol compared to a protocol in which resonant acoustic energy (RAE) was used. Standard demineralization protocols use stir plates and are generally have lengthy processing times, resulting in the tissue being exposed to HCL for a long period of time (anywhere from 0.75-6.0 hours). The hypothesis evaluated in this study is whether use of RAE may increase the rate of demineralization. RAE was introduced using a LabRAM™ II ResonantAcoustic® Mixer (Resodyn, Butte, MT). Demineralization was assessed by compression, with a pass criteria that the graft could be compressed to at least 50% of its original volume and then return to its original form. The study did not evaluate visual or dimensional failures.

The study was performed using cleansed cancellous bone obtained from human donors. The cancellous bone was cut to the desired dimensions and then was cleansed using the methods and apparatus described in U.S. Patent Nos. 7,658,888; 7,776,291; 7,794,653; 7,919,043; 8,202,898; and 8,486,344. The tissue was then rehydrated using Dulbecco's Phosphate-Buffered Saline (DPBS). The tissue sizes assessed were:

| | |
|---|---|
| Medium Cube | 10 mm x 10 mm x 10 mm |
| Large Block | 20 mm x 15 mm x 10 mm |
| Small Block | 14 mm x 10 mm x 10 mm |
| Large Strips | 50 mm x 20 mm x 5 mm |

The tissue was weighed and grouped based on weight to maintain uniformity between each sample group and processing method.

Demineralization was performed using 1 N HCl as the processing solution. For the RAE protocol, the ratio of HCl volume to weight of tissue used was 1500 ml to 100 g. Approximately 26 grams of tissue was placed in the jars supplied with the LabRAM machine for each test group. The jars were filled to 80% full based on Resodyn recommendation, which was approximately 360 ml of HCl. The same ratio of HCl volume to weight of tissue was used for the standard demineralization protocol, with 26 g tissue and 360 mL of HCl used.

RAE Procedure: Samples were processed using 5 cycles total.
1. The jars containing the tissue and HCl were placed in the LabRAM machine and processed. The LabRAM machine was set to 60 G intensity and frequency at 60 Hz. Two cycle length times were tested: 10 min and 30 min.
2. After each cycle, the HCl in the jars was discarded, and the tissue was evaluated to assess 50% compression (visual assessment). Tissue passing rate was then recorded.
3. For each subsequent cycle, samples were placed back in fresh HCl (repeat steps 1-2).
4. After the last cycle, the tissue was neutralized by placing in a beaker with a stir bar on a stir plate with 26g/360ml of DPBS and stirred for 5-10mins. A pH reading was taken after each period. If pH was not >6.0, old DPBS is removed and fresh DPBS was added. This repeats until pH >6.0 is achieved.

Standard demineralization procedure: Samples were processed using 5 cycles total.
1. The tissue was placed in a beaker containing a magnetic stir bar and a sufficient volume of 70% isopropyl alcohol to fully submerge the tissue.
2. The beaker was placed on stir plate at room temperature (-25 °C) and stirred for 30 min, after which the isopropyl alcohol was decanted.
3. 200 mL ±250 mL of sterile water was added to the beaker, and the beaker was returned to the stir plate and stirred for 5-25 minutes at room temperature (-25 °C), after which the water was decanted.
4. The tissue was placed in a beaker containing a magnetic stir bar with 4000 mL ± 250 mL of HCl and agitated on the stir plate for 30-50 minutes at room temperature (-25 °C).
5. After each cycle, the HCl in the vessel was discarded, and the tissue was evaluated to assess 50% compression as above. Tissue passing rate was then recorded.
6. For each subsequent cycle, samples were placed back in fresh HCl (repeat step 4).
7. 2000 mL ± 250 mL of sterile water was added, and it was stirred for 5-25 minutes at room temperature (-25 °C)
8. Sterile water was decanted from the beaker, and the tissue was stirred for 10-30 minutes at room temperature (-25 °C) in PBS.

| **Table 3. Average Pass Rate** | | | | | | |
|---|---|---|---|---|---|---|
| RAE Protocol - 10 min cycles | | | | | | |
| Cycle: | 1 | 2 | 3 | 4 | 5 | n |
| Medium Cube | 84% | 93% | 95% | 95% | 96% | 4 |
| Large Blocks | 77% | 94% | 100% | 100% | 100% | 3 |
| Small Blocks | 67% | 94% | 100% | 100% | 100% | 2 |
| Large Strips | 100% | 94% | 94% | 94% | 94% | 1 |

| RAE Protocol - 30 min cycles | | | | | | |
|---|---|---|---|---|---|---|
| Cycle: | 1 | 2 | 3 | 4 | 5 | n |
| Medium Cube | 94% | 95% | 95% | 95% | 95% | 3 |
| Large Blocks | 100% | 100% | 100% | 100% | 100% | 3 |
| Small Blocks | 94% | 94% | 100% | 100% | 100% | 2 |

| Standard Protocol - 30 min cycles | | | | | | |
|---|---|---|---|---|---|---|
| Cycle: | 1 | 2 | 3 | 4 | 5 | n |
| Medium Cube | 46% | 56% | 63% | 70% | 78% | 3 |
| Large Blocks | 16% | 58% | 58% | 77% | 81% | 3 |
| Small Blocks | 6% | 17% | 22% | 28% | 39% | 2 |

Observations: The difference in the compression passing yield rates and the processing time between the LabRAM machine and the standard stir plate processing method was substantial. The RAE process can yield demineralized tissue with a compression passing rate above 80% in less than 30 minutes. There was little difference between using a 10 min and 30 min cycle time for the RAE protocol. The 10 min cycle protocol demineralized cubes 15 times faster than the standard protocol (10 min vs 150 min; 1 cycle vs 3 cycles). The 10 min cycle protocol demineralized the large and small blocks 7.5 times faster than the standard protocol (20 min vs. 150 min; 2 cycles vs 3 cycles). In addition, no over-demineralization of tissue was observed (breakdown of structural integrity of bone tissue).

**Study #2.** The study was performed as described above in Study #1 except that samples were processed using five cycles of 10 minutes. The portions of human cancellous bone tissue assessed were:

| | |
|---|---|
| Medium Cube (120) | 10 mm x 10 mm x 10 mm |
| Large Cube (76) | 14 mm x 14 mm x 14 mm |
| Large Strips (29) | 50 mm x 20 mm x 5 mm |

The tissue pieces were pooled together and then split randomly into 10 samples for assessment. After each cycle tissue was removed and assessed. Tissue that passed the compression criteria evaluation was removed from the processing vessels for subsequent cycles. Tissue that failed visual inspection (e.g., due to altered dimensions or shape) was also removed from the vessels for subsequent cycles. Tissue that failed the compression evaluation but passed visual inspection was placed into the processing vessels with fresh HCl for subsequent cycles.

| **Table 4** | | | | |
|---|---|---|---|---|
| | Medium Cube | Large Cube | Large Strip | Grand Total |
| Sum of No. of Grafts | 120 | 76 | 29 | 225 |
| Sum of Total Pass | 106 | 62 | 18 | 186 |
| Sum of Total Compression Failures | 1 | 0 | 0 | 1 |
| Sum of Total Visual Failures | 13 | 14 | 11 | 38 |
| Ave. % passing (all samples) | 99.2% | 100% | 100% | 99.6% |

| **Table 5** | | | | | |
|---|---|---|---|---|---|
| **Jar / Cycle:** | **1** | **2** | **3** | **4** | **5** |
| 1 | 69% | 85% | 85% | 85% | 85% |
| 2 | 90% | 95% | 95% | 95% | 95% |
| 3 | 70% | 78% | 78% | 83% | 83% |
| 4 | 37% | 63% | 63% | 63% | 63% |
| 5 | 48% | 52% | 57% | 57% | 57% |
| 6 | 50% | 75% | 75% | 88% | 86% |
| 7 | 62% | 81% | 81% | 81% | 81% |
| 8 | 79% | 88% | 88% | 88% | 88% |
| 9 | 79% | 95% | 96% | 95% | 95% |
| 10 | 52% | 64% | 68% | 68% | 68% |
| Average | 63% | 78% | 78% | 80% | 80% |

Out of 225 grafts, only 1 graft failed compression after 5 cycles. After 3 cycles, only 7 out of 120 medium cubes failed compression (5.8%). After 2 cycles, there were no compression failures of the large cubes or large strips. The visual failures appear to be to the natural variance in cancellous bone and the method by which the tissue pieces are cut. Compared to the typical processing time using the standard demineralization protocol described in the First Study, the RAE protocol increase yield by approximately 28% and is substantially faster.

**Study #3.** This study was performed as described above for Study #2 except that two HCl concentrations were tested: 0.5 N HCl and 1 N HCl. Four sets of medium cubes (10 mm x 10 mm x 10 mm) of human cancellous bone tissue from human donors were run for each condition. After each cycle, tissue was assessed visually and for compression criteria. Unlike the Second Study, all tissue (passed or failed) was placed back into the processing vessels in fresh HCl for subsequent cycles.

In brief, cubes of cancellous bone tissue (10 mm³) were cleaned according to standard cleaning protocols and then rehydrated. The tissue was weighed and divided into processing sample weights of 26 grams. Each processing sample was loaded into the processing vessel containing approximately 360 mL of the processing solution (1 N or 0.5 N HCl). Acoustic resonance energy was applied to the processing vessel containing the combination of tissue and acid at 60 G and 60 Hz for five ten-minute cycles. After application of each acoustic resonance energy cycle, the processing solution was discarded and the tissue was assessed for compression. The tissue was then re-loaded in the processing vessel with a fresh volume of processing solution and application of acoustic reosnance energy was applied to the processing vessel for each subsequent cycle. At the completion of five cyles, the processing solution was removed and the bone tissue was neutralized.

**Table 6** and **Table 7** summarize the percent of samples that passed the compression analysis after each ten-minute cycle. These results indicate that the HCl concentration had little impact on the number of samples passing, as all groups had a >79% passing rate after 3 cycles, with the peak passing rate at cycle 4 for both concentrations of HCl. **Table 6** and **Table 7** summarize the average passing percentage per cycle and the passing percentage by sample per cycle, respectively.

| **Table 6** | | | | | |
|---|---|---|---|---|---|
| Group (n=4) | Cycle | | | | |
| | 1 | 2 | 3 | 4 | 5 |
| 0.5 M HCl | 50% | 74% | 89% | 93% | 93% |
| 1.0 M HCl | 61% | 81% | 90% | 97% | 97% |

| **Table 7** | | | | | |
|---|---|---|---|---|---|
| RAE Protocol - 0.5 N HCl | | | | | |
| Sample / Cycle: | 1 | 2 | 3 | 4 | 5 |
| 1 | 51% | 82% | 92% | 95% | 95% |
| 2 | 34% | 65% | 79% | 85% | 85% |
| 3 | 50% | 69% | 92% | 100% | 100% |
| 4 | 63% | 81% | 94% | 94% | 94% |

| RAE Protocol - 1 N HCl | | | | | |
|---|---|---|---|---|---|
| Sample / Cycle: | 1 | 2 | 3 | 4 | 5 |
| 1 | 61% | 73% | 84% | 92% | 92% |
| 2 | 50% | 86% | 94% | 97% | 97% |
| 3 | 91% | 100% | 100% | 100% | 100% |
| 4 | 42% | 67% | 83% | 100% | 100% |

**Study #4.** Cancellous bone tissue from human donors was prepared for demineralization as described above in Study #1. Thirty medium cubes (10 mm x 10 mm x 10 mm) were processed in 750 mL of 1 N HCl in a 32 ounce jar (∼80% full) for 1 cycle of 5 min at 50 G intensity and 60 Hz frequency. The tissue samples were then assessed for compressibility and residual calcium content (performed by Pace Analytical, Centennial, CO). The compression criteria was that the graft could be compressed to at least 50% of its original volume and then return to its original form. A residual calcium content of less than or equal to 8% is desirable to be consistent with AATB criteria for demineralized cancellous bone tissue products.

The data from this study is shown in **FIG. 6****.** The tissue samples are organized in the graph by compressibility, with sample 1 being most compressible and sample 30 being least compressible. Residual calcium content (w/w) is represented on the y-axis and a subset of the samples are depicted (labeled by sample number) across the x-axis. Eighty percent (80% - 24/30) of the tissue samples met the compression criteria. In addition, all but two of the samples met the residual calcium criteria as well. In all, only two samples (28 and 30) failed both the compression and calcium criteria. This data demonstrates that a short acid (HCl) exposure time of only 5 minutes in the RAE procedure was sufficient to demineralize bone tissue grafts to meet desired criteria for demineralized bone products. This study also demonstrates that the compression criteria used is comparable to assessing residual calcium content for determining extent of bone demineralization.

**Table 8** provides the average percent increase in demineralization efficiency based on the studies described above. Increased demineralization efficiency was determined by comparing the time to demineralization from resonant acoustic processing techniques as disclosed herein with a commonly known bone demineralization technique that involves stirring the bone sample in an acid solution. For example, if the standard literature method treats bone with acid for 10 minutes to effect 10% demineralization, while the resonant acoustic energy methods described herein can effect 95% demineralization in the same period of time, the average % increase in demineralization efficiency is 85%. Commonly reported methods of demineralizing bone include those as described in Urist, M., Science 150(3698):893-899 (Nov. 12, 1965) and Pietrzak, W.S., et al., J. Craniofac. Surg. 17(1):84-90 (Jan. 2006). Urist describes demineralization of sections of bone *(e.g.,* long bones from rabbits cut in lengths of 1 to 2 cm) placed in an acid solution, and Pietrzak describes demineralization of human bone powder by stirring in acid. As evidenced in **Table 8,** a significant improvement in demineralization efficiency can be observed after a single resonant acoustic processing cycle.

| **Table 8** | | | | | |
|---|---|---|---|---|---|
| **Average % Increase in Demineralization Efficiency** | | | | | |
| **Bone Volume** | **Cycle 1** | **Cycle 2** | **Cycle 3** | **Cycle 4** | **Cycle 5** |
| **1 cc cube** | 50% | 35% | 30% | 25% | 20% |
| **3 cc block** | 60% | 75% | 78% | 72% | 60% |
| **1.4 cc block** | 60% | 35% | 42% | 23% | 20% |
| **4 cc strip** | 100% | 94% | 94% | 94% | 94% |

### EXAMPLE 2. Cell Viability Assessment

This study was performed to assess the impact of resonant acoustic energy (RAE) on tissue containing living cells. Cartilage tissue from human donors that contains native, viable chondrocytes was chosen as the representative test tissue.

Samples of cartilage were punched into 8 mm circles and shaved down to 1 mm thick disks, and then laser etched (square pattern) as described in U.S. Patent No. 9,186,253.

Prior to processing, samples were tested using Presto Blue assay to determine the initial cell count of each graft as described in U.S. Patent No. 9,186,253. A1: 10 ratio of PrestoBlue® reagent (Life Technologies, Carlsbad, CA) to cell culture medium was added to a sample so that the sample is covered by the medium. The metabolic activity of the cells changes the color of the medium. After 3 hours incubation, 100 µl aliquots were taken from each sample and added to a multi-well plate for reading in a plate reader. The samples were then rinsed in media.

Samples of 3 8 mm x 1 mm cartilage disks were then placed in triplicate in 125 mL sterile plastic specimen cups (Covidien, Minneapolis, MN) and filled with of human chondrocyte growth medium (Cell Applications, Inc., San Diego, CA). The cups were placed in a LabRAM™ II ResonantAcoustic® Mixer (Resodyn, Butte, MT) and RAE was applied at various settings for different amounts of time (as shown in **Table 9** below). The frequency was kept at 60 Hz for all conditions.

After processing, the cartilage samples from each condition were placed into 24 well culture plates, covered with fresh chondrocyte growth media, and incubated at 37°C for 24 hours. A Presto Blue assay was then run again to determine the final cell count for each sample. Initial and final cell counts were compared to determine if application of RAE had a negative effect on the final cell viability in the tissue. The results of this study are shown in **Table 9** below. Samples for which the cell count of the processed sample remained about the same as the original cell count (no impact on cell viability) are denoted with "+++". Samples for which the cell count of the processed sample reflected a decrease of 50% or less compared to the original cell count are denoted by "+". Samples that reflected a greater than 50% reduction in cell viability after processing are denoted by "-".

| **Table 9** | | | | | | | | |
|---|---|---|---|---|---|---|---|---|
| **Chondrocyte Cell Viability** | | | | | | | | |
| | **10 min.** | **15 min.** | **20 min.** | **25 min.** | **30 min.** | **35 min.** | **40 min.** | **45 min.** |
| **10G** | +++ | +++ | +++ | +++ | +++ | +++ | +++ | +++ |
| **20G** | +++ | +++ | +++ | +++ | +++ | +++ | +++ | +++ |
| **30G** | +++ | +++ | +++ | +++ | +++ | +++ | +++ | +++ |
| **40G** | +++ | +++ | +++ | +++ | +++ | +++ | +++ | +++ |
| **50G** | +++ | +++ | +++ | +++ | +++ | +++ | +++ | + |
| **60G** | +++ | +++ | +++ | + | + | + | + | + |
| **70G** | + | + | + | - | - | - | - | - |
| **80G** | + | + | - | - | - | - | - | - |
| **90G** | - | - | - | - | - | - | - | - |
| **100G** | - | - | - | - | - | - | - | - |

A range of conditions for RAE may be applied to cartilage that have no effect on the viability of the chondrocytes in the tissue. Many of the samples that had reduced cell viability after processing were noted to be very hot upon removal from the LabRAM machine. The increased temperature may be the cause of the reduction in the cell viability. If the temperature of the sample in the processing vessel was maintained so as to not increase above about 37°C (for example, by cooling the interior of the LabRAM machine), the higher intensity conditions (60 G and above) and the longer processing times for these conditions would likely be usable. The data from this study can be used to guide selection of intensity and duration for a wide range of other methods described in this application. Depending on the type of tissue to be processed, lower or higher intensity conditions may also be selected based on the hardiness or other aspects of the tissue in comparison to cartilage.

### EXAMPLE 3. Cryopreservation Method

This study was performed to assess whether resonant acoustic energy (RAE) could facilitate cryopreservation of tissue containing living cells. Cartilage tissue containing native, viable chondrocytes was chosen as the representative test tissue.

Samples of cartilage were prepared as described above in Example 2. Samples were then tested using Presto Blue assay to determine the initial cell count of each graft as described in U.S. Patent No. 9,186,253. Samples of 3 8 mm x 1 mm cartilage disks were then placed in triplicate in 125 mL sterile plastic specimen cups (Covidien, Minneapolis, MN) containing 20% DMSO + 80% human chondrocyte growth medium (Cell Applications, Inc., San Diego, CA). The jars were then placed into a LabRAM™ II ResonantAcoustic® Mixer (Resodyn, Butte, MT) and processed at 30 G intensity and 60 Hz frequency for 30 min, 35 min, 40 min, or 45 min. Only one sample set was used per condition. After processing, all samples were placed in cryo vials with 10% DMSO + 90% fetal bovine serum (FBS). Vials were then placed into a Mr. Frosty™ Freezing Container (ThermoFisher Scientific), a controlled rate freezing device, which was then placed into a -80°C freezer. Samples were stored for 3 months and then thawed in a water bath (37°C). The samples were then placed into 24 well culture plates, covered with fresh chondrocyte growth media, and incubated at 37°C for 4 days. Finally, the Presto Blue assay was run on all the samples to determine final cell count.

Control samples were prepared in the same manner but did not receive the DMSO treatment in the LabRAM machine. Instead, they were cryopreserved using a standard protocol: placed in a controlled rate freezer and brought down to -80% before storage at -80%. This protocol typically results in 0-20% viability of cryopreserved cartilage and other tissues. The control samples were stored for 1 week instead of 3 months before thawing. These samples were also given 4 days to recover before final Presto Blue assay was run.

The data from this study is summarized in **Table 10** below. The data demonstrates that using RAE to process tissue for cryopreservation significantly increases the viability of the cryopreserved tissue as compared to the control tissue. Each processing time tested resulted in at least about a two-fold increase in cell viability compared to the control. This increase may be due to the ability of RAE to drive the cryoprotectant (a large molecule) into the matrix of the tissue (in the case of this example, the cartilage matrix) thereby protecting cells that would otherwise be more succeptible to the negative impact of freezing and be destroyed or severely weakened.

| **Table 10** | | | |
|---|---|---|---|
| | **# of Cells Pre-RAE** | **# Cells Post-Thaw** | **% Viability** |
| 30G/ 30 Min | 2043.67 | 874 | 54.57% |
| 30G/ 35Min | 2127.67 | 1154 | 58.36% |
| 30G/ 40 Min | 980.33 | 803 | 82.65% |
| 30G/ 45Min | 1975 | 943.67 | 46.28% |
| Control | 6237 | 1521 | 24.39% |

### EXAMPLE 4. Production of Stromal Vascular Fraction (SVF)

Current methods of processing adipose tissue to yield SVF can be damaging to the cells produced during the process, due to high concentrations of enzyme and the physical shear on the cells. This process can also be time consuming. This study was conducted to determine if resonant acoustic energy (RAE) could be used to produce SVF and whether processing time and/or amount of digestive enzyme could be reduced as a result.

Adipose tissue was obtained from two human donors. Tissue from Donor 1 was portioned into 3 portions of 500 cm³ (cc) for testing RAE protocols. Tissue from Donor 2 was portioned into a 100 cm³ (cc) portion for testing a RAE protocol and the remaining tissue (∼1L) was processed using the standard SVF protocol.

For RAE processing, adipose tissue (500 cm³ (cc) or 100 cm³ (cc)) was loaded into 500 mL processing jars filled with approximately 500 mL DMEM and various concentrations of collagenase as indicated in the first column of **Table 11.** A Blender Ball™ was also added to each processing jar to phyiscally aid in breaking up the tissue. The processing jars were placed into a LabRAM™ II ResonantAcoustic® Mixer (Resodyn, Butte, MT) and processed for 15 min using the conditions set forth in **Table 11.** One sample was processed per condition. After processing, the processing solution was removed, and the processed tissue was poured through a 300-500 µm mesh filter into a separatory funnel. The filtered contents rested for 10 minutes to allow phase separation.

SVF was also prepared from a control sample processed using a standard protocol. Donor tissue (∼1L) was mixed with a solution containing 310,000-350,000 Units collagenase and 500 mL medium and then ground using a manual tissue grinder/mincer that mechanically breaks down tissue. The processed tissue was then poured through a 9.5 mm sieve and a 4 mm sieve and then passed through a 300-500 µm filter mesh into a separatory funnel. The filtered contents rested for 10 minutes to allow phase separation.

After phase separation, the supranatant for each sample was collected and cetrifuged for 10 minutes at 500 g. The cell pellet was collected and assessed for cell viability and the percentage of cells expressing CD90, a marker for mesenchymal stem cells (MSC). The data from this study is summarized in **Table 11.**

| **Table 11** | | | | |
|---|---|---|---|---|
| | **% Collagenase** | **Time (min)** | **Viability** | **CD90+%(MSC content)** |
| Donor 1 | | | | |
| Sample 1: 50 G, 60 Hz | 50% | 15 | 99.40% | 19.27% |
| Sample 2: Control | 100% | 45-50 | 99.70% | 10.44% |
| | | | | |

| Donor 2 | | | | |
|---|---|---|---|---|
| Sample 1: 40 G, 60 Hz | 0% | 15 | 100.00% | 11.50% |
| Sample 2: 40 G, 60 Hz | 50% | 15 | 100.00% | 16.30% |
| Sample 3: 40 G, 60 Hz | 100% | 15 | 100.00% | 2.50% |
| | | | | |
| Control historical data | | | 99.95% | 6.14% |

Use of RAE to process adipose tissue was not found to have any negative effect on cell viability. It was found that processing adipose tissue using RAE significantly decreased processing time, while using less enzyme to digest the tissue. Compared to the 45-50 min processing time needed for processing with colleganase in standard SVF manufacturing protocols, 15 min was sufficient to produce SVF when RAE was used, regardless of how much collagenase was used. Significantly, satisfactory SVF was produced by applying RAE to the tissue without using any collagenase at all. Standard protocols for producing SVF all require the use of collagenase. Reducing the amount of collagenase during the processing may result in healthier cells long term. RAE intensities of 40-50 G were selected based on the cell viability study described in Example 2. Processing of tissue using RAE also resulted in a higher content of CD90+ cells, suggesting a higher concentration of MSCs in the processed composition. The increased CD90+ cell content may be due to the decreased exposure of the tissue to collagenase, as extended exposure of tissue to collagenase can lead to significant degradation of tissue components (such as fibrous tissue, for example, the collagen matrix). Also, when less collagenase is used for shorter periods of time the fibrous tissue of the fat may still remain intact, instead of digested, which likely leads to less "waste" cells or a cleaner sample with more stem cells. For comparison purposes, the standard protocol used for the control sample of this study historically yields SVF having 99.95% viablity and 6.14% CD90+ cell content.

### EXAMPLE 5. Tissue Decellularization

This study was conducted to determine if resonant acoustic energy (RAE) could be used to decellularize tissue. Dermal tissue - full thickness and split thickness - was used as a representative test tissue. A process using RAE was compared to a standard decellularization method for producing acellular skin graft that requires incubating the tissue for 60-75 minutes in a thermal shaker with NaOH. This study assessed whether RAE could be used in a decellularization method without the use of NaOH (or any other chemicals) and within a shorter processing time.

The process was tested on full-thickness skin (1-2 mm) and split-thickness skin (0.4-1 mm) from human donors (one 250 g piece each).

Skin tissue (250 g) was loaded into a 2 L processing vessel with 1 L of 5% saline solution. The processing vessel was shaken at 145 RPM in a thermal shaker (New Brunswick Excella 24r, Eppendorf) for 48 hours at 8 °C. The tissue was then removed and delaminated with a guage, and histology punches were taken for pre-RAE assessment. Delaminated tissue (250 g) was loaded into a 1 L rectangular jar processing vessel with 600 mL of processing solution: warmed, sterile water at 37 °C. The processing vessels were placed into a LabRAM™ II ResonantAcoustic® Mixer (Resodyn, Butte, MT) and processed using the conditions set forth in **Table 12.** The rationale for the program cycle was that, by creating an oscillating program at varying times and intensities, a hostile environment could be created that would cause cells in the tissue to burst from exposure to the sterile water after previously having been soaked in the 5% saline solution as part of the delamination process. At the completion of the program cycle, the water in the processing vessel was removed and replaced with fresh warm, sterile water. The program cycle was completed for a total of three times for each sample. At the completion of these cyles, histological punches were taken from the tissue.

| **Table 12** | | |
|---|---|---|
| RAE Program Cycle | | |
| Step | Time | Intensity |
| 1 | 1 sec | 20 G |
| 2 | 10 sec | 60 G |
| 3 | 3 sec | 15 G |
| 4 | 10 sec | 60 G |
| 5 | 3 sec | 15 G |
| 6 | 10 sec | 60 G |
| 7 | 3 sec | 15 G |
| 8 | 10 sec | 60 G |
| 9 | 3 sec | 15 G |
| 10 | 10 sec | 60 G |

The histological punches (pre- and post-processing) were stained with hematoxalin and eosin (H&E) staining according to standard methods and visualized with a light microscope at 40 X magnification. **FIG. 7A** and **FIG. 7B** shows representative images of pre-processed and post-processed full thickness skin, respectively. **FIGS. 8A-8C** show representative images of pre-delamination (FIG. 8A), pre-processed (FIG. 8B), and post-processed (FIG. 8C) split-thickness skin. In each of these images, the nuclei of intact cells are visualized as dark circular shapes. Some cell destruction was noticed in the full thickness skin post-processing but it was not uniform throughout the tissue sample, possibly due to the thickness of the skin. In the split-thickness skin, there was uniform cell destruction observed through the processed tissue.

This data shows that it is possible to use resonant acoustic energy to decellularize skin tissue. In particular, decellularization may be performed with water alone and without the use of harsh conditions or additives. Depending on the thickness of the tissue to be decellularized, the specific series of intensities and duration in which RAE is applied may be varied to increase the extent of decellularization. Also, in some instances, the processing solution may include additives or have more acidic or basic properties, to facilitate decellularization of tissues. Of note, the processing time for decellularization was significantly shorter as compared to a standard protocol used to decellularize skin tissue, indicating that RAE may be used to increase the decellularization rate of tissue.

It is to be understood that the figures and descriptions of the disclosure have been simplified to illustrate elements that are relevant for a clear understanding of the disclosure. It should be appreciated that the figures are presented for illustrative purposes and not as construction drawings.

## Claims

1. A method of processing a tissue, the method comprising:
(a) loading a processing vessel with a tissue and a processing solution, thereby providing a combination comprising the tissue and the processing solution disposed in the processing vessel; and
(b) applying resonant acoustic energy having a frequency between 15 Hertz and 60 Hertz to the processing vessel, thereby vibrating the processing vessel and the combination disposed therein to form a processed tissue.

2. The method of claim 1, wherein the processed tissue is demineralized bone tissue, decellularized tissue, tissue mixed with a cryoprotectant, tissue cleaned of microbial contamination, or homogenized tissue.

3. The method of claim 1 or claim 2, wherein the tissue comprises cortical bone, cancellous bone, cortical and cancellous bone, tendon, skin, cartilage, fascia, muscle, nerves, vascular tissue, birth tissue, or adipose tissue.

4. The method of any preceding claim, wherein the processing solution comprises a demineralization solution or agent, a decellularization solution or agent, a cryopreservation solution or agent, a cleansing solution or agent, an extraction solution, a saline solution, a buffer solution, a cell culture medium, a cell culture component, or water.

5. The method of any preceding claim, wherein the processing solution comprises at least one of an acid solution, a basic solution, a buffer solution, a saline solution, an alcohol, an organic solvent, a detergent, a cross-linking agent, an oxidizing agent, a chelating agent, an antimicrobial solution or agent, a polymer, a cryoprotectant, a disinfectant, or water.

6. The method of any of claims 1-5, wherein the tissue is bone tissue, the processing solution comprises an acid solution, and the processed tissue is demineralized bone.

7. The method of any of claims 1-5, wherein the tissue is adipose, the processing solution comprises a cell culture medium, and the processed tissue is stromal vascular fraction.

8. The method of any of claims 1-5, wherein the tissue is adipose or skin, the processing solution comprises cell culture medium, a buffer solution, an acid solution, an alkaline metal salt solution, a solution comprising a digestive enzyme, and the processed tissue is a homogenized tissue.

9. A method of demineralizing a bone tissue, such as wherein the bone tissue comprises cortical bone, cancellous bone, or cortical and cancellous bone, the method comprising:
(a) loading a processing vessel with a bone tissue and a processing solution, thereby providing a combination comprising the bone tissue and the processing solution disposed in the processing vessel, wherein the processing solution is an acid processing solution, such as wherein the acid processing solution comprises a mineral acid; and
(b) applying resonant acoustic energy having a frequency between 15 Hertz and 60 Hertz to the processing vessel, thereby vibrating the processing vessel and the combination disposed therein to form a demineralized bone tissue.

10. A method of cryopreserving a tissue, such as wherein the tissue comprises bone, tendon, skin, cartilage, fascia, muscle, nerves, vascular tissue, birth tissue, or adipose tissue, the method comprising:
(a) loading a processing vessel with a tissue and a processing solution comprising a cryoprotectant, thereby providing a combination comprising the tissue and the cryopreservation processing solution disposed in the processing vessel;
(b) applying resonant acoustic energy having a frequency between 15 Hertz and 60 Hertz to the processing vessel, thereby vibrating the processing vessel and the combination disposed therein to form a processed tissue comprising the tissue mixed with the cryoprotectant; and
(c) freezing the processed tissue to form a cryopreserved tissue.

11. A method of reducing microbial contamination of a tissue, such as wherein the tissue comprises cortical bone, cancellous bone, cortical and cancellous bone, tendon, skin, cartilage, fascia, muscle, nerves, vascular tissue, birth tissue, or adipose tissue, the method comprising:
(a) loading a processing vessel with a tissue and a processing solution, thereby providing a combination comprising the tissue and the processing solution disposed in the processing vessel, such as wherein the processing solution comprises an alcohol, an acid solution, an organic solvent, an oxidizing agent, a cross-linking agent, sodium hypochlorite, water, an antibiotic, or combinations thereof; and
(b) applying resonant acoustic energy having a frequency between 15 Hertz and 60 Hertz to the processing vessel, thereby vibrating the processing vessel and the combination disposed therein to remove at least a portion of microbial load from the tissue.

12. A method of assessing the microbial contamination of a tissue, such as wherein the tissue comprises cortical bone, cancellous bone, cortical and cancellous bone, tendon, skin, cartilage, fascia, muscle, nerves, vascular tissue, birth tissue, or adipose tissue, the method comprising:
(a) loading a processing vessel with a tissue and a processing solution, thereby providing a combination comprising the tissue and the processing solution disposed in the processing vessel, such as wherein the processing solution comprises an alcohol, an acid solution, an organic solvent, an oxidizing agent, a cross-linking agent, sodium hypochlorite, water, or combinations thereof;
(b) applying resonant acoustic energy having a frequency between 15 Hertz and 60 Hertz to the processing vessel, thereby vibrating the processing vessel and the combination disposed therein to release microbes from the tissue into the processing fluid; and
(c) assessing the processing fluid to determine the microbial load of the tissue.

13. A method of homogenizing a tissue, such as wherein the tissue comprises skin tissue or adipose tissue, the method comprising:
(a) loading a processing vessel with a tissue; and
(b) applying resonant acoustic energy having a frequency between 15 Hertz and 60 Hertz to the processing vessel, thereby vibrating the processing vessel and the tissue disposed therein to form a homogenized tissue.

14. A method according to claim 13, further comprising loading a processing solution into the processing vessel with the tissue, wherein applying resonant acoustic energy to the processing vessel forms a homogenized tissue.

15. A method of producing a homogenized tissue product, the method comprising:
(a) loading a processing vessel with:
(i) a tissue, such as wherein the tissue comprises skin tissue or adipose tissue, and/or wherein the tissue comprises homogenized tissue; and
(ii) at least one of a biological component or a chemical agent, such as wherein the biological component is particulate biological matter comprising ground bone, minced cartilage, or cells;
thereby providing a combination comprising the tissue and at least one of a biological component or a chemical agent disposed in the processing vessel; and
(b) applying resonant acoustic energy having a frequency between 15 Hertz and 60 Hertz to the processing vessel, thereby vibrating the processing vessel and the tissue disposed therein to form a homogenized tissue product.

16. A method according to claim 15 wherein the method further comprises loading a processing solution into the processing vessel with the tissue and the at least one of a biological component or a chemical agent, wherein the processing solution comprises an acid solution, an alkaline metal salt solution, a saline solution, a buffer solution, a solution comprising collagenase, or water.

17. The method of any preceding claim, wherein the resonant acoustic energy exerts up to 100 times the energy of G-force on the processing vessel and combination; and/or wherein the resonant acoustic energy is applied a plurality of times for up to a total time of 2 minutes to 4.5 hours.

18. The method of any preceding claim, wherein the tissue, the processing solution, or both, are evaluated after application of the resonant acoustic energy to assess at least one characteristic.

19. The method of any preceding claim, further comprising:
removing the processing solution from the processing vessel after application of the resonant acoustic energy;
adding a second processing solution to the processing vessel, thereby forming a second combination of the processed tissue and the second processing solution; and
applying resonant acoustic energy having a frequency between 15 Hertz and 60 Hertz to the processing vessel, thereby vibrating the processing vessel and the second combination disposed therein.

20. The method of any preceding claim, wherein the tissue comprises cleaned tissue.

21. The method of any preceding claim, wherein the tissue is dried after application of the acoustic field.

22. A cryopreserved tissue product made according to the method of claim 10 comprising cartilage tissue having a processing solution penetrated therein, wherein the processing solution comprises a cryoprotectant.

23. The cryopreserved tissue product of claim 22, wherein the cryoprotectant is selected from the group consisting of dimethyl sulfoxide (DMSO), methanol, butanediol, propanediol, polyvinylpyrrolidone, glycerol, hydroxyethyl starch, alginate, glycols, ethylene glycol, polyethylene glycol, propylene glycol, and butylene glycol.

24. The cryopreserved tissue product of claim 22 or 23, wherein the processing solution further comprises one or more of: second cryoprotectant, serum, or platelet rich plasma.

## Patentansprüche

1. Verfahren zur Verarbeitung eines Gewebes, wobei das Verfahren umfasst:
(a) Beladen eines Verarbeitungsgefäßes mit einem Gewebe und einer Verarbeitungslösung, wodurch eine Kombination, umfassend das Gewebe und die Verarbeitungslösung, bereitgestellt wird, die sich in dem Verarbeitungsgefäß befindet; und
(b) Anwenden von resonierender Schallenergie mit einer Frequenz zwischen 15 Hertz und 60 Hertz auf das Verarbeitungsgefäß, wodurch das Verarbeitungsgefäß und die sich darin befindende Kombination in Schwingungen versetzt werden, um ein verarbeitetes Gewebe zu bilden.

2. Verfahren nach Anspruch 1, wobei das verarbeitete Gewebe demineralisiertes Knochengewebe, dezellularisiertes Gewebe, mit einem Kryoprotektivum gemischtes Gewebe, von mikrobieller Kontamination gereinigtes Gewebe oder homogenisiertes Gewebe ist.

3. Verfahren nach Anspruch 1 oder 2, wobei das Gewebe Kortikalis, Spongiosa, Kortikalis und Spongiosa, Sehne, Haut, Knorpel, Faszie, Muskel, Nerven, Gefäßgewebe, Geburtsgewebe oder Fettgewebe umfasst.

4. Verfahren nach einem vorhergehenden Anspruch, wobei die Verarbeitungslösung eine Demineralisierungslösung oder ein Demineralisierungsmittel, eine Dezellularisierungslösung oder ein Dezellularisierungsmittel, eine Kryokonservierungslösung oder ein Kryokonservierungsmittel, eine Reinigungslösung oder ein Reinigungsmittel, eine Extraktionslösung, eine Kochsalzlösung, eine Pufferlösung, ein Zellkulturmedium, eine Zellkulturkomponente oder Wasser umfasst.

5. Verfahren nach einem vorhergehenden Anspruch, wobei die Verarbeitungslösung mindestens eine bzw. einen bzw. eines von einer sauren Lösung, einer basischen Lösung, einer Pufferlösung, einer Kochsalzlösung, einem Alkohol, einem organischen Lösungsmittel, einem Detergens, einem Vernetzungsmittel, einem Oxidationsmittel, einem Chelatbildner, einer antimikrobiellen Lösung oder einem antimikrobiellen Mittel, einem Polymer, einem Kryoprotektivum, einem Desinfektionsmittel oder Wasser umfasst.

6. Verfahren nach einem der Ansprüche 1-5, wobei das Gewebe Knochengewebe ist, die Verarbeitungslösung eine saure Lösung umfasst und das verarbeitete Gewebe demineralisierter Knochen ist.

7. Verfahren nach einem der Ansprüche 1-5, wobei das Gewebe Fett ist, die Verarbeitungslösung ein Zellkulturmedium umfasst und das verarbeitete Gewebe eine stromal-vaskuläre Fraktion ist.

8. Verfahren nach einem der Ansprüche 1-5, wobei das Gewebe Fett oder Haut ist, die Verarbeitungslösung Zellkulturmedium, eine Pufferlösung, eine saure Lösung, eine Alkalimetallsalzlösung, eine Lösung, umfassen ein Verdauungsenzym, umfasst und das verarbeitete Gewebe ein homogenisiertes Gewebe ist.

9. Verfahren zur Demineralisierung eines Knochengewebes, wie zum Beispiel wobei das Knochengewebe Kortikalis, Spongiosa oder Kortikalis und Spongiosa umfasst, wobei das Verfahren umfasst:
(a) Beladen eines Verarbeitungsgefäßes mit einem Knochengewebe und einer Verarbeitungslösung, wodurch eine Kombination, umfassend das Knochengewebe und die Verarbeitungslösung, bereitgestellt wird, die sich in dem Verarbeitungsgefäß befindet, wobei die Verarbeitungslösung eine saure Verarbeitungslösung ist, wie zum Beispiel wobei die saure Verarbeitungslösung eine Mineralsäure umfasst; und
(b) Anwenden von resonierender Schallenergie mit einer Frequenz zwischen 15 Hertz und 60 Hertz auf das Verarbeitungsgefäß, wodurch das Verarbeitungsgefäß und die sich darin befindende Kombination in Schwingungen versetzt werden, um ein demineralisiertes Knochengewebe zu bilden.

10. Verfahren zur Kryokonservierung eines Gewebes, wie zum Beispiel wobei das Gewebe Knochen, Sehne, Haut, Knorpel, Faszie, Muskel, Nerven, Gefäßgewebe, Geburtsgewebe oder Fettgewebe umfasst, wobei das Verfahren umfasst:
(a) Beladen eines Verarbeitungsgefäßes mit einem Gewebe und einer Verarbeitungslösung, umfassend ein Kryoprotektivum, wodurch eine Kombination, umfassend das Gewebe und die Kryokonservierungsverarbeitungslösung, bereitgestellt wird, die sich in dem Verarbeitungsgefäß befindet;
(b) Anwenden von resonierender Schallenergie mit einer Frequenz zwischen 15 Hertz und 60 Hertz auf das Verarbeitungsgefäß, wodurch das Verarbeitungsgefäß und die sich darin befindende Kombination in Schwingungen versetzt werden, um ein verarbeitetes Gewebe, umfassend das Gewebe, gemischt mit dem Kryoprotektivum, zu bilden; und
(c) Einfrieren des verarbeiteten Gewebes, um ein kryokonserviertes Gewebe zu bilden.

11. Verfahren zur Verringerung einer mikrobiellen Kontamination eines Gewebes, wie zum Beispiel wobei das Gewebe Kortikalis, Spongiosa, Kortikalis und Spongiosa, Sehne, Haut, Knorpel, Faszie, Muskel, Nerven, Gefäßgewebe, Geburtsgewebe oder Fettgewebe umfasst, wobei das Verfahren umfasst:
(a) Beladen eines Verarbeitungsgefäßes mit einem Gewebe und einer Verarbeitungslösung, wodurch eine Kombination, umfassend das Gewebe und die Verarbeitungslösung, bereitgestellt wird, die sich in dem Verarbeitungsgefäß befindet, wie zum Beispiel wobei die Verarbeitungslösung einen Alkohol, eine saure Lösung, ein organisches Lösungsmittel, ein Oxidationsmittel, ein Vernetzungsmittel, Natriumhypochlorit, Wasser, ein Antibiotikum oder Kombinationen davon umfasst; und
(b) Anwenden von resonierender Schallenergie mit einer Frequenz zwischen 15 Hertz und 60 Hertz auf das Verarbeitungsgefäß, wodurch das Verarbeitungsgefäß und die sich darin befindende Kombination in Schwingungen versetzt werden, um mindestens einen Teil einer mikrobiellen Belastung von dem Gewebe zu entfernen.

12. Verfahren zur Beurteilung der mikrobiellen Kontamination eines Gewebes, wie zum Beispiel wobei das Gewebe Kortikalis, Spongiosa, Kortikalis und Spongiosa, Sehne, Haut, Knorpel, Faszie, Muskel, Nerven, Gefäßgewebe, Geburtsgewebe oder Fettgewebe umfasst, wobei das Verfahren umfasst:
(a) Beladen eines Verarbeitungsgefäßes mit einem Gewebe und einer Verarbeitungslösung, wodurch eine Kombination, umfassend das Gewebe und die Verarbeitungslösung, bereitgestellt wird, die sich in dem Verarbeitungsgefäß befindet, wie zum Beispiel wobei die Verarbeitungslösung einen Alkohol, eine saure Lösung, ein organisches Lösungsmittel, ein Oxidationsmittel, ein Vernetzungsmittel, Natriumhypochlorit, Wasser oder Kombinationen davon umfasst;
(b) Anwenden von resonierender Schallenergie mit einer Frequenz zwischen 15 Hertz und 60 Hertz auf das Verarbeitungsgefäß, wodurch das Verarbeitungsgefäß und die sich darin befindende Kombination in Schwingungen versetzt werden, um Mikroben von dem Gewebe in die Verarbeitungsflüssigkeit freizusetzen; und
(c) Beurteilen der Verarbeitungsflüssigkeit, um die mikrobielle Belastung des Gewebes zu bestimmen.

13. Verfahren zur Homogenisierung eines Gewebes, wie zum Beispiel wobei das Gewebe Hautgewebe oder Fettgewebe umfasst, wobei das Verfahren umfasst:
(a) Beladen eines Verarbeitungsgefäßes mit einem Gewebe und
(b) Anwenden von resonierender Schallenergie mit einer Frequenz zwischen 15 Hertz und 60 Hertz auf das Verarbeitungsgefäß, wodurch das Verarbeitungsgefäß und das sich darin befindende Gewebe in Schwingungen versetzt werden, um ein homogenisiertes Gewebe zu bilden.

14. Verfahren nach Anspruch 13, weiterhin umfassend ein Laden einer Verarbeitungslösung in das Verarbeitungsgefäß mit dem Gewebe, wobei das Anwenden von resonierender Schallenergie auf das Verarbeitungsgefäß ein homogenisiertes Gewebe bildet.

15. Verfahren zur Produktion eines homogenisierten Gewebeprodukts, wobei das Verfahren umfasst:
(a) Beladen eines Verarbeitungsgefäßes mit:
(i) einem Gewebe, wie zum Beispiel wobei das Gewebe Hautgewebe oder Fettgewebe umfasst und/oder wobei das Gewebe homogenisiertes Gewebe umfasst; und
(ii) mindestens einer bzw. einem von einer biologischen Komponente oder einem chemischen Mittel, wie zum Beispiel wobei die biologische Komponente eine teilchenförmige biologische Substanz ist, umfassend gemahlenen Knochen, zerkleinerten Knorpel oder Zellen;
wodurch eine Kombination, umfassend das Gewebe und mindestens eine bzw. eines von einer biologischen Komponente oder einem chemischen Mittel, bereitgestellt wird, die sich in dem Verarbeitungsgefäß befindet; und
(b) Anwenden von resonierender Schallenergie mit einer Frequenz zwischen 15 Hertz und 60 Hertz auf das Verarbeitungsgefäß, wodurch das Verarbeitungsgefäß und das sich darin befindende Gewebe in Schwingungen versetzt werden, um ein homogenisiertes Gewebeprodukt zu bilden.

16. Verfahren nach Anspruch 15, wobei das Verfahren weiterhin ein Laden einer Verarbeitungslösung in das Verarbeitungsgefäß mit dem Gewebe und der bzw. dem mindestens einen von einer biologischen Komponente oder einem chemischen Mittel, wobei die Verarbeitungslösung eine saure Lösung, eine Alkalimetallsalzlösung, eine Kochsalzlösung, eine Pufferlösung, eine Lösung, umfassend Kollagenase, oder Wasser umfasst.

17. Verfahren nach einem vorhergehenden Anspruch, wobei die resonierende Schallenergie bis zu das 100-fache der Energie einer g-Kraft auf das Verarbeitungsgefäß und die Kombination ausübt und/oder wobei die resonierende Schallenergie mehrmals für bis zu eine Gesamtzeit von 2 Minuten bis 4,5 Stunden angewendet wird.

18. Verfahren nach einem vorhergehenden Anspruch, wobei das Gewebe, die Verarbeitungslösung oder beide nach Anwendung der resonierenden Schallenergie evaluiert werden, um mindestens ein Charakteristikum zu beurteilen.

19. Verfahren nach einem vorhergehenden Anspruch, weiterhin umfassend:
Entfernen der Verarbeitungslösung aus dem Verarbeitungsgefäß nach Anwendung der resonierenden Schallenergie;
Zugeben einer zweiten Verarbeitungslösung zu dem Verarbeitungsgefäß, wodurch eine zweite Kombination des verarbeiteten Gewebes und der zweiten Verarbeitungslösung gebildet wird; und
Anwenden von resonierender Schallenergie mit einer Frequenz zwischen 15 Hertz und 60 Hertz auf das Verarbeitungsgefäß, wodurch das Verarbeitungsgefäß und die sich darin befindende zweite Kombination in Schwingungen versetzt werden.

20. Verfahren nach einem vorhergehenden Anspruch, wobei das Gewebe gereinigtes Gewebe umfasst.

21. Verfahren nach einem vorhergehenden Anspruch, wobei das Gewebe nach Anwendung des Schallfelds getrocknet wird.

22. Kryokonserviertes Gewebeprodukt, das gemäß dem Verfahren nach Anspruch 10 hergestellt wurde, umfassend Knorpelgewebe mit einer darin eingesickerten Verarbeitungslösung, wobei die Verarbeitungslösung ein Kryoprotektivum umfasst.

23. Kryokonserviertes Gewebeprodukt nach Anspruch 22, wobei das Kryoprotektivum aus der Gruppe bestehend aus Dimethylsulfoxid (DMSO), Methanol, Butandiol, Propandiol, Polyvinylpyrrolidon, Glycerin, Hydroxyethylstärke, Alginat, Glykolen, Ethylenglykol, Polyethylenglykol, Propylenglykol und Butylenglykol ausgewählt ist.

24. Kryokonserviertes Gewebeprodukt nach Anspruch 22 oder 23, wobei die Verarbeitungslösung weiterhin eines oder mehrere der folgenden umfasst: ein zweites Kryoprotektivum, Serum oder thrombozytenreiches Plasma.

## Revendications

1. Procédé de traitement d'un tissu, le procédé comprenant :
a) l'introduction d'un tissu et d'une solution de traitement dans un récipient de traitement, en produisant ainsi une combinaison comprenant le tissu et la solution de traitement disposés dans le récipient de traitement ; et
b) l'application d'énergie acoustique résonante ayant une fréquence de 15 Hertz à 60 Hertz au récipient de traitement, en faisant ainsi vibrer le récipient de traitement et la combinaison qui y est disposée pour former un tissu traité.

2. Procédé selon la revendication 1, dans lequel le tissu traité est un tissu osseux déminéralisé, un tissu décellularisé, un tissu mélangé à un cryoprotecteur, un tissu dont une contamination microbienne a été éliminée, ou un tissu homogénéisé.

3. Procédé selon la revendication 1 ou la revendication 2, dans lequel le tissu comprend de l'os cortical, de l'os spongieux, de l'os cortical et de l'os spongieux, des tendons, de la peau, du cartilage, un fascia, des muscles, des nerfs, un tissu vasculaire, un tissu fœtal, ou un tissu adipeux.

4. Procédé selon l'une quelconque des revendications précédentes, dans lequel la solution de traitement comprend une solution ou un agent de déminéralisation, une solution ou un agent de décellularisation, une solution ou un agent de cryoconservation, une solution ou un agent de nettoyage, une solution d'extraction, une solution saline, une solution tampon, un milieu de culture cellulaire, un composant de culture cellulaire, ou de l'eau.

5. Procédé selon l'une quelconque des revendications précédentes, dans lequel la solution de traitement comprend au moins l'un d'une solution acide, d'une solution basique, d'une solution tampon, d'une solution saline, d'un alcool, d'un solvant organique, d'un détergent, d'un agent de réticulation, d'un agent oxydant, d'un agent chélatant, d'une solution antimicrobienne ou d'un agent antimicrobien, d'un polymère, d'un cryoprotecteur, d'un désinfectant, ou d'eau.

6. Procédé selon l'une quelconque des revendications 1 à 5, dans lequel le tissu est un tissu osseux, la solution de traitement comprend une solution acide, et le tissu traité est de l'os déminéralisé.

7. Procédé selon l'une quelconque des revendications 1 à 5, dans lequel le tissu est un tissu adipeux, la solution de traitement comprend un milieu de culture cellulaire, et le tissu traité est une fraction vasculaire stromale.

8. Procédé selon l'une quelconque des revendications 1 à 5, dans lequel le tissu est un tissu adipeux ou cutané, la solution de traitement comprend un milieu de culture cellulaire, une solution tampon, une solution acide, une solution de sel de métal alcalin, une solution comprenant une enzyme digestive, et le tissu traité est un tissu homogénéisé.

9. Procédé de déminéralisation d'un tissu osseux, par exemple dans lequel le tissu osseux comprend de l'os cortical, de l'os spongieux, ou de l'os cortical et de l'os spongieux, le procédé comprenant :
a) l'introduction d'un tissu osseux et d'une solution de traitement dans un récipient de traitement, en produisant ainsi une combinaison comprenant le tissu osseux et la solution de traitement disposés dans le récipient de traitement, la solution de traitement étant une solution de traitement acide, la solution de traitement acide comprenant par exemple un acide minéral ; et
b) l'application d'énergie acoustique résonante ayant une fréquence de 15 Hertz à 60 Hertz au récipient de traitement, en faisant ainsi vibrer le récipient de traitement et la combinaison qui y est disposée pour former un tissu osseux déminéralisé.

10. Procédé de cryoconservation d'un tissu, par exemple dans lequel le tissu comprend de l'os, des tendons, de la peau, du cartilage, un fascia, des muscles, des nerfs, un tissu vasculaire, un tissu fœtal, ou un tissu adipeux, le procédé comprenant :
a) l'introduction d'un tissu et d'une solution de traitement dans un récipient de traitement comprenant un cryoprotecteur, en produisant ainsi une combinaison comprenant le tissu et la solution de traitement de cryoconservation disposés dans le récipient de traitement ;
b) l'application d'énergie acoustique résonante ayant une fréquence de 15 Hertz à 60 Hertz au récipient de traitement, en faisant ainsi vibrer le récipient de traitement et la combinaison qui y est disposée pour former un tissu traité comprenant le tissu mélangé avec le cryoprotecteur ; et
c) la congélation du tissu traité pour former un tissu cryoconservé.

11. Procédé de réduction de la contamination microbienne d'un tissu, par exemple dans lequel le tissu comprend de l'os cortical, de l'os spongieux, de l'os cortical et de l'os spongieux, des tendons, de la peau, du cartilage, un fascia, des muscles, des nerfs, un tissu vasculaire, un tissu fœtal, ou un tissu adipeux, le procédé comprenant :
(a) l'introduction d'un tissu et d'une solution de traitement dans un récipient de traitement, en produisant ainsi une combinaison comprenant le tissu et la solution de traitement disposés dans le récipient de traitement, la solution de traitement comprenant par exemple un alcool, une solution acide, un solvant organique, un agent oxydant, un agent de réticulation, de l'hypochlorite de sodium, de l'eau, un antibiotique, ou des combinaisons de ceux-ci ; et
(b) l'application d'énergie acoustique résonante ayant une fréquence de 15 Hertz à 60 Hertz au récipient de traitement, en faisant ainsi vibrer le récipient de traitement et la combinaison qui y est disposée pour éliminer au moins une partie de la charge microbienne du tissu.

12. Procédé d'évaluation de la contamination microbienne d'un tissu, par exemple dans lequel le tissu comprend de l'os cortical, de l'os spongieux, de l'os cortical et de l'os spongieux, des tendons, de la peau, du cartilage, un fascia, des muscles, des nerfs, un tissu vasculaire, un tissu fœtal, ou un tissu adipeux, le procédé comprenant :
a) l'introduction d'un tissu et d'une solution de traitement dans un récipient de traitement, en produisant ainsi une combinaison comprenant le tissu et la solution de traitement disposés dans le récipient de traitement, la solution de traitement comprenant par exemple un alcool, une solution acide, un solvant organique, un agent oxydant, un agent de réticulation, de l'hypochlorite de sodium, de l'eau ou des combinaisons de ceux-ci ;
b) l'application d'énergie acoustique résonante ayant une fréquence de 15 Hertz à 60 Hertz au récipient de traitement, en faisant ainsi vibrer le récipient de traitement et la combinaison qui y est disposée pour libérer du tissu des microbes qui passent dans le fluide de traitement ; et
c) l'évaluation du fluide de traitement pour déterminer la charge microbienne du tissu.

13. Procédé d'homogénéisation d'un tissu, par exemple dans lequel le tissu comprend un tissu cutané ou un tissu adipeux, le procédé comprenant :
(a) l'introduction d'un tissu dans un récipient de traitement ; et
(b) l'application d'énergie acoustique résonante ayant une fréquence de 15 Hertz à 60 Hertz au récipient de traitement, en faisant ainsi vibrer le récipient de traitement et le tissu qui y est disposé pour former un tissu homogénéisé.

14. Procédé selon la revendication 13, comprenant en outre l'introduction d'une solution de traitement dans le récipient de traitement avec le tissu, dans lequel l'application d'énergie acoustique résonante au récipient de traitement forme un tissu homogénéisé.

15. Procédé de fabrication d'un produit tissulaire homogénéisé, le procédé comprenant :
(a) l'introduction dans un récipient de traitement :
(i) d'un tissu, par exemple dans lequel le tissu comprend un tissu cutané ou un tissu adipeux, et/ou dans lequel le tissu comprend un tissu homogénéisé ; et
(ii) d'au moins l'un d'un composant biologique ou d'un agent chimique, par exemple dans lequel le composant biologique est constitué d'une matière biologique particulaire comprenant de l'os broyé, du cartilage haché ou des cellules ;
en produisant ainsi une combinaison comprenant le tissu et au moins l'un d'un composant biologique ou d'un agent chimique disposé dans le récipient de traitement ; et
b) l'application d'énergie acoustique résonante ayant une fréquence de 15 Hertz à 60 Hertz au récipient de traitement, en faisant ainsi vibrer le récipient de traitement et le tissu qui y est disposé pour former un produit tissulaire homogénéisé.

16. Procédé selon la revendication 15, le procédé comprenant en outre l'introduction d'une solution de traitement dans le récipient de traitement avec le tissu et au moins l'un d'un composant biologique ou d'un agent chimique, la solution de traitement comprenant une solution acide, une solution de sel de métal alcalin, une solution saline, une solution tampon, une solution comprenant de la collagénase, ou de l'eau.

17. Procédé selon l'une quelconque des revendications précédentes, dans lequel l'énergie acoustique résonante exerce jusqu'à 100 fois l'énergie de la force G sur le récipient de traitement et la combinaison ; et/ou dans lequel l'énergie acoustique résonante est appliquée plusieurs fois pendant un temps total de 2 minutes à 4,5 heures.

18. Procédé selon l'une quelconque des revendications précédentes, dans lequel le tissu, la solution de traitement, ou les deux, sont évalués après l'application de l'énergie acoustique résonante pour évaluer au moins une caractéristique.

19. Procédé selon l'une quelconque des revendications précédentes, comprenant en outre :
le retrait de la solution de traitement du récipient de traitement après l'application de l'énergie acoustique résonante ;
l'addition d'une deuxième solution de traitement au récipient de traitement, en formant ainsi une deuxième combinaison, constituée du tissu traité et de la deuxième solution de traitement ; et
l'application d'énergie acoustique résonante ayant une fréquence de 15 Hertz à 60 Hertz au récipient de traitement, en faisant ainsi vibrer le récipient de traitement et la seconde combinaison qui y est disposée.

20. Procédé selon l'une quelconque des revendications précédentes, dans lequel le tissu comprend un tissu nettoyé.

21. Procédé selon l'une quelconque des revendications précédentes, dans lequel le tissu est séché après l'application du champ acoustique.

22. Produit tissulaire cryoconservé fabriqué par le procédé selon la revendication 10, comprenant un tissu cartilagineux dans lequel a pénétré une solution de traitement, dans lequel la solution de traitement comprend un cryoprotecteur.

23. Produit tissulaire cryoconservé selon la revendication 22, dans lequel le cryoprotecteur est sélectionné dans le groupe constitué du diméthylsulfoxyde (DMSO), du méthanol, du butanediol, du propanediol, de la polyvinylpyrrolidone, du glycérol, de l'amidon hydroxyéthylique, d'un alginate, de glycols, de l'éthylène glycol, du polyéthylène glycol, du propylène glycol et du butylène glycol.

24. Produit tissulaire cryoconservé selon la revendication 22 ou 23, dans lequel la solution de traitement comprend en outre un ou plusieurs de : un deuxième cryoprotecteur, un sérum, ou un plasma riche en plaquettes.
